(19) Europäisches Patentamt

European Patent Office

Office européen des brevets

(11)  **EP 3 728 566 B1**

(12)  **EUROPEAN PATENT SPECIFICATION**

(45) Date of publication and mention
of the grant of the patent:
**21.08.2024  Bulletin 2024/34**

(21) Application number: **18825797.6**

(22) Date of filing: **21.12.2018**

(51) International Patent Classification (IPC):
**C12N 5/0775** (2010.01)   **C12N 5/073** (2010.01)

(52) Cooperative Patent Classification (CPC):
**C12N 5/0605**

(86) International application number:
**PCT/IB2018/060503**

(87) International publication number:
**WO 2019/123407 (27.06.2019 Gazette 2019/26)**

(54)  **MESENCHYMAL STROMAL CELLS AND METHODS FOR OBTAINING MESENCHYMAL STROMAL CELLS FROM UMBILICAL CORD**

MESENCHYMALE STROMAZELLEN UND VERFAHREN ZUR GEWINNUNG VON MESENCHYMALEN STROMAZELLEN AUS DER NABELSCHNUR

CELLULES STROMALES MÉSENCHYMATEUSES ET MÉTHODES D'OBTENTION DE CELLULES STROMALES MÉSENCHYMATEUSES À PARTIR DE CORDON OMBILICAL

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**
Designated Extension States:
**BA ME**
Designated Validation States:
**KH MA MD TN**

(30) Priority:  **22.12.2017  PCT/IB2017/058366**

(43) Date of publication of application:
**28.10.2020  Bulletin 2020/44**

(73) Proprietor: **Chiesi Farmaceutici S.p.A.**
**43122 Parma (IT)**

(72) Inventors:
• **ARDIGO', Diego**
**43122 Parma (IT)**
• **MILAZZO, Giovanni**
**43122 Parma (IT)**
• **DOMINICI, Massimo**
**43122 Parma (IT)**
• **MURGIA, Alba**
**43122 Parma (IT)**

(74) Representative: **Chiesi Farmaceutici S.p.A. et al**
**Via Palermo, 26/A**
**43122 Parma (IT)**

(56) References cited:
• **COOPER T W ET AL: "Platelet-derived collagenase inhibitor: characterization and subcellular localization.", PROCEEDINGS OF THE NATIONAL ACADEMY OF SCIENCES OF THE UNITED STATES OF AMERICA MAY 1985, vol. 82, no. 9, May 1985 (1985-05-01), pages 2779 - 2783, ISSN: 0027-8424**
• **ANNA RADOMSKI ET AL: "Identification, regulation and role of tissue inhibitor of metalloproteinases-4 (TIMP-4) in human platelets", BRITISH JOURNAL OF PHARMACOLOGY, WILEY-BLACKWELL, UK, vol. 137, no. 8, 29 January 2009 (2009-01-29), pages 1330 - 1338, XP071100231, ISSN: 0007-1188, DOI: 10.1038/SJ.BJP.0704936**
• **LIU Y E ET AL: "PREPARATION AND CHARACTERIZATION OF RECOMBINANT TISSUE INHIBITOR OFMETALLOPROTEINASE 4 (TIMP-4)", JOURNAL OF BIOLOGICAL CHEMISTRY, AMERICAN SOCIETY FOR BIOCHEMISTRY AND MOLECULAR BIOLOGY, US, vol. 272, no. 33, 1 January 1997 (1997-01-01), pages 20479 - 20483, XP002929363, ISSN: 0021-9258, DOI: 10.1074/JBC.272.33.20479**

- JOHN E. DAVIES ET AL: "Concise Review: Wharton's Jelly: The Rich, but Enigmatic, Source of Mesenchymal Stromal Cells : Wharton's Jelly an Enigmatic MSC Source", STEM CELLS TRANSLATIONAL MEDICINE, vol. 6, no. 7, 10 May 2017 (2017-05-10), pages 1620 - 1630, XP055494058, ISSN: 2157-6564, DOI: 10.1002/sctm.16-0492
- ANNA LANGE-CONSIGLIO ET AL: "In Vitro Studies of Horse Umbilical Cord Matrix-Derived Cells: From Characterization to Labeling for Magnetic Resonance Imaging", OPEN TISSUE ENGINEERING AND REGENERATIVE MEDICINE JOURNAL 2011 BENTHAM SCIENCE PUBLISHERS B.V. NLD, vol. 4, no. 1, 30 December 2011 (2011-12-30), pages 120 - 133, XP055494616, ISSN: 1875-0435, DOI: 10.2174/1875043501104010120
- ARJUNAN SUBRAMANIAN ET AL: "Comparative Characterization of Cells from the Various Compartments of the Human Umbilical Cord Shows that the Wharton's Jelly Compartment Provides the Best Source of Clinically Utilizable Mesenchymal Stem Cells", PLOS ONE, vol. 10, no. 6, 10 June 2015 (2015-06-10), pages e0127992, XP055494087, DOI: 10.1371/journal.pone.0127992
- MARIA TERESA CONCONI: "Phenotype and Differentiation Potential of Stromal Populations Obtained from Various Zones of Human Umbilical Cord: An Overview", THE OPEN TISSUE ENGINEERING AND REGENERATIVE MEDICINE JOURNAL, vol. 4, no. 1, 30 December 2011 (2011-12-30), pages 6 - 20, XP055062745, ISSN: 1875-0435, DOI: 10.2174/1875043501104010006
- "INTERNATIONAL REVIEW OF NEUROBIOLOGY.", vol. 108, 1 January 2013, ACADEMIC PRESS, LONDON., GB, ISSN: 0074-7742, article JORGE RIBEIRO ET AL: "Perspectives of Employing Mesenchymal Stem Cells from the Wharton's Jelly of the Umbilical Cord for Peripheral Nerve Repair", pages: 79 - 120, XP055473635, DOI: 10.1016/B978-0-12-410499-0.00004-6
- KITA K ET AL: "Isolation and characterization of mesenchymal stem cells from the sub-amniotic human umbilical cord lining membrane", STEM CELLS AND DEVELOPMENT, MARY ANN LIEBERT, INC, NEW ROCHELLE, NY, US, vol. 19, no. 4, 1 April 2010 (2010-04-01), pages 491 - 502, XP002696905, ISSN: 1557-8534, [retrieved on 20090727], DOI: 10.1089/SCD.2009.0192
- BOLOMSKY ARNOLD ET AL: "Immunomodulatory drugs thalidomide and lenalidomide affect osteoblast differentiation of human bone marrow stromal cells in vitro", EXPERIMENTAL HEMATOLOGY, ELSEVIER INC, US, vol. 42, no. 7, 2 April 2014 (2014-04-02), pages 516 - 525, XP029010860, ISSN: 0301-472X, DOI: 10.1016/J.EXPHEM.2014.03.005

Remarks:

The file contains technical information submitted after the application was filed and not included in this specification

## Description

### Introduction

Field of the Invention

The present invention relates to a method for obtaining stromal cells, particularly stromal progenitor cells. Stromal progenitor cells are typically capable of giving rise to phenotypically and genotypically identical daughters (self-renewal) as well as to at least one other final cell type. Some stromal cells have self-renewal capacities and such cells have received growing attention in recent years. The stromal cells are non-embryonic mesenchymal stromal cells. They are obtainable by isolation from umbilical cord by the method of the invention, which is disclosed in detail herein.

Background of the invention

Mesenchymal stromal cells (MSC) are stromal cells of particular interest to the scientific community, *inter alia* because of their multipotency and low immunogenicity (Pittenger et al., 1999, Science;, vol. 284, p. 143-147). According to the state of the art, mesenchymal stromal cells (MSCs) are traditionally typically obtained from bone marrow, but MSCs can be also isolated from a variety of other mammalian sources, such as adipose tissue, muscle, connective tissue, dental pulp, cord blood, placenta and amniotic fluid (Iudicone et al., 2014, J. Transl. Med., 12: 28). Among MSCs, adult MSC derived from the bone marrow (BM-MSC) have been most extensively studied (e.g. WO 2008/042174 A2). In the bone marrow, MSCs co-exist with a variety of cell types including more primitive and embryonic-like stem cells, mesenchymal stem cells, lineage-committed progenitors as well as mature cells such as osteoblasts and fibroblasts, and different methods have been described for the isolation of e.g. bone marrow-derived MSCs (see e.g. Majore, Cell Commun. Signal., 2009, vol. 20; 7:6). Bolomsky et al. (2014, Experimental Hematology, vol. 42, p. 516-525) describes the effects of the immunomodulary drugs thalidomide and lenalidome on osteoblast differentiation of human bone marrow stromal cells, including the effects on expression of genes such as the gene *APCDD1*. Some efforts have also been made in the past to explore alternative sources of mesenchymal stromal cells. For example, the umbilical cord (UC) was proposed as a suitable reservoir of different cell types, some of which were shown to have stem-cell like properties. The umbilical cord is a structure in placental mammals that connects the placenta to the developing fetus, thereby providing a source of fetal nourishment. It is available after birth without any invasive procedure and ethical concerns (Hass et al., 2011, Cell. Commun. Signal., vol. 9:12). The umbilical cord comprises Wharton's Jelly, a gelatinous, loose mucous connective tissue rich in stromal cells which was named after the anatomist Thomas Wharton. The Wharton's Jelly comprises cells dispersed in an amorphous ground substance composed of proteoglycans, including hyaluronic acid and different types of glycans (see e.g. WO 2004/072273 A1). It is generally considered a primitive mesenchymal tissue of primarily extra-embryonic derivation (Weiss, 1983, Histology, cell and tissue biology, New York, Elsevier Biomedical, p. 997-998), and has been proposed as a promising source of MSCs (Wang et al., 2004, Stem Cells, vol. 22: p. 1330-1337).

It is generally recognized that stromal cells of the umbilical cord (UC) are heterogeneous regarding structure, biological properties and localization within the UC. Several methods to obtain cells from the Wharton's Jelly (WJ) of the UC have been described, and cells with MSC-like properties have been described to reside in, and originate from, different parts of the UC. The human umbilical cord comprises three vessels, which are normally organized in a left spiral (counter clockwise) turn. Mitchell et al. (2003, Stem Cells, vol. 21, p. 50-60) describe a method wherein the umbilical cord vessels are removed prior to harvesting the remaining tissue. This remaining tissue, which includes both a fraction of the WJ and the amniotic epithelium, is then diced and to produce small tissue fragments that are transferred to tissue culture plates; enzymatic digestion of the umbilical cord or remaining tissue is however not foreseen. Rather, tissue fragments are subsequently used as primary explants from which cells migrate outside the tissue specimens onto culture substratum. Another report (Romanov et al., 2003, Stem Cells, vol. 21, p. 105-110) indicates that fibroblast-like MSCs may be isolated from cord vasculature by digestion of the umbilical vein with collagenase, which however yields a mixed population of vascular endothelial and subendothelial cells. US 5,919,702 A (Purchio et al.) describes an isolation method comprising slicing open a ca. 2.5 cm long segment of cord longitudinally, removing blood vessels and casing and collecting the WJ into a sterile container, where it is cut and cultured. For example, it is described that cells may be isolated by placing a 2-3 mm$^3$ section of the WJ on a glass slide on the bottom of a Petri dish, covering it with another slide and culturing it for 10-12 days in order to allow the some cells ("pre-chondrocytes") to migrate out of the culture dish. Use of the pre-chondrocytes to produce cartilage is also mentioned.

Davies et al. (Stem Cells Translational Medicine, 2017, vol. 6, p. 1620-1630) is a review article that describes anatomy of the umbilical cord and methods for cell isolation from Wharton's Jelly. Subramanian et al. (2015, PLOS ONE, vol. 10(6), p. 1-25) compares characteristics of cells derived from the amnion, subamnion, perivascular, Wharton's Jelly and mixed cord compartments of human umbilical cords. Conconi et al. (2011, Open Tissue Engineer. Regen. Med. J., vol. 4, p. 6-20) compares extraction, culture methods and characteristics of human umbilical cord cells obtained from the subendothelial layer of the umbilical vein, the perivascular zone, the Wharton's Jelly, the umbilical cord lining and from the whole umbilical cord. Ribeiro et al. (2013, International Review of Neurobiology, vol. 108, p. 79-120) describes protocols for isolating mesenchymal stem cells from Wharton's Jelly from human umbilical cord. Kita et al. (2010, Stem Cells and Development, vol. 19(4), p. 491-501) characterises cord lining membrane mesenchymal stem cells obtained from the subamnion of human umbilical cord membrane such as by looking at the expression of surface markers.

The isolation of umbilical cord-matrix derived cells has also been reported from animal tissue origin. For example, Lange-Consiglio et al., (2011, Open Tissue Engineer. Regen. Med. J., vol. 4, p. 120-133) report the isolation of "umbilical cord-matrix derived cells" from "intervascular" and "perivascular" areas of the horse umbilical cord.

The weight of human umbilical cord of approximately 40 g at term (Raio et al., 1999, Eur. J. Obstet. Gynecol. Reprod. Biol., vol. 83; p. 131-135; Conconi et al., 2011, Open Tissue Engineer. Regen. Med. J., vol. 4, p. 6-20) poses a natural upper limit for starting material available for isolation of cells with MSC-like properties. According to US 2004/0136967 A1 and Seshareddy et al. (2008, Meth. Cell Biol., vol. 86, p. 101-119), it is difficult to obtain high numbers of desired cells from the umbilical cord. Therefore, the authors recommend extracting relatively large cell numbers, and describe conditions that have been optimized for isolation of relatively high cell numbers; for example, in order to enable extraction of large cell numbers. They propose a relatively harsh enzymatic treatment of the umbilical cord. Different zones of the Wharton's Jelly are described, but exclusion of particular areas of origin of cells for extraction, such as of perivascular area, is not specifically recommended, in line with the general recommendation to use high cell numbers. The cells are cultivated post isolation. An alternative approach is described by WO 2004/072273 A and Sarugaser et al. (Stem Cells, 2005, 23:220-229); these publications describe the isolation of cells with MSC-like properties exclusively from the perivascular area of the umbilical cord. Since this area is anatomically relatively restricted, the amount of starting material is relatively low, in comparison with isolation methods that start form the whole umbilical cord. Further, in view of the close proximity to the blood-containing vessels, contamination by blood cells cannot be excluded; therefore negative selection against CD45 expression is described to avoid contamination with undesired cells in culture. Also WO 2017/058003 A1 describes processes for isolation of MSCs from the umbilical cord, dental pulp, foreskin, cornea or other sources; the process for isolation of mesenchymal stromal cells from umbilical cord is preferably conducted under sterile conditions, and the umbilical cord is preferably subjected to collagenase treatment for relatively long time intervals. According to Conconi et al., 2011, Open Tissue Engineer. Regen. Med. J., vol. 4, p. 6-20, the most suitable protocols for enhancing *in vitro* expansion and storage of MSCs are yet to be established. Thus, there is still a need to provide populations consisting of cells which are characterized by essentially uniform properties and which are capable to expand reliably ex *vivo*; and reliable methods for providing them are also needed.

## Problem to be solved

Thus, an object of the present invention includes eliminating the disadvantages associated with the state of the art. Particular objects comprise the provision of a reliable method for obtaining a mesenchymal stromal cell (MSC), and of the reproducible provision of mesenchymal stromal cells (MSCs) and populations of MSCs, with superior self-renewal properties in good yields. It is desired that the populations be relatively uniform and do not contain undesired cells, such as apoptotic cells, hematopoietic lineage cells etc. Thus, in other words, it is desired to provide mesenchymal stromal cells by a method that is advantageous in terms of reliability and/or yield, with respect to presently known and practiced methods. Various drawbacks of the state of the art define further goals for improvement addressed by the present inventors, and these goals have arrived at by the contribution described and claimed herein.

## Summary of the Invention

The present invention relates to an ex vivo method for obtaining mesenchymal stromal cells (MSCs). Described herein are also mesenchymal stromal cells (MSCs) and to other descriptions related thereto. The main aspects and embodiments of the invention are summarized as follows.

In a first aspect, the present invention relates to an ex vivo method for obtaining a mesenchymal stromal cell. The method comprises (a) isolating the stromal Wharton's Jelly or a fraction thereof from an umbilical cord wherein the stromal Wharton's jelly is the Wharton's Jelly of the umbilical cord that excludes the perivascular area, and (b) subjecting the stromal Wharton's Jelly or fraction thereof to enzymatic treatment comprising the enzyme collagenase in the presence of platelet lysate (PL), thereby releasing at least one cell from the stromal Wharton's Jelly or fraction thereof; wherein step (b) is followed by a step (c): (c) expanding said at least one cell in a growth medium comprising platelet lysate (PL). Preferably, the cell obtained after step (b) is multipotent.

In addition to steps (a) and (b) at least one additional step is comprised in the method of the invention. In particular, it is a step for increasing the number of cells in comprised, taking advantage of the cell division potential of the cells obtained in step (b). Preferably, the cell(s) obtained after step (c) are multipotent.

The present inventors have surprisingly found that cells originating from the stroma of the umbilical cord are particularly advantageous. Advantages include homogeneity of the cell populations and stem cell like properties. In particular, in the absence of umbilical cord vessels, these advantages can be achieved. Therefore, it is preferable that the step (a) isolating the stromal Wharton's Jelly or a fraction thereof from the umbilical cord is characterized by a step of physically removing the vessels and the perivascular area from the umbilical cord. The vessels and the perivascular area may be removed before step (a) or during step (a). One technically advantageous way of removing the vessels and the perivascular area, which is preferred in the present invention, is characterized by stripping out the vessels and the perivascular area from the umbilical cord. The inventors have observed that the stripping out of the vessels, e.g. by pulling the vessels out, also removes the perivascular area.

Further, the inventors have found that it is advantageous to segment the umbilical cord prior to said step of physically removing the vessels and the perivascular area. The segmenting is typically achieved by physical means, such as scissors or a scalpel. Preferably the segmenting is perpendicular to the cord. More preferably, the perpendicular segments preferably each have a length of about 1 to about 4 cm, preferably about 2 to about 3 cm, and more preferably about 2.5 cm. In a preferred embodiment, the segment(s) of vessels are removed from the segment(s) of the umbilical cord prior to step (b). In particular, it is preferred to remove the vessels, or more precisely segments of vessels, after the - preferably perpendicular - segmenting of the umbilical cord.

The inventors have also found that the amount of collagenase activity matters. In a preferred embodiment, the collagenase is present during the enzymatic treatment step (b) at a relatively low specific activity, as detailed further below.

In the method of the present invention, a rapid isolation of stromal cells from the umbilical cord is advantageous. In a preferred embodiment, steps (a) and (b) together are conducted in a total time of less than 6 hours, more preferably less than 5 hours, more preferably less than 4 hours and most preferably less than 3 hours. It is evident that a short duration of steps (a) and (b) together also means that step (b) itself is short. Generally, the shortness of step (b), i.e. the rapid isolation, in particular the shortness of the enzymatic treatment, can contribute to the mild conditions during step (b).

Surprisingly, the inventors have further found that the presence of platelet lysate during the enzymatic digestion is advantageous. First, the inventors found that the presence of human platelet lysate does not inhibit or decrease or block the enzymatic activity of the enzyme collagenase, at least not to an extent that would negatively interfere with the desired enzymatic liberation of the cells from the surrounding tissue, e.g. from the stromal matrix. It is known that blood plasma contains numerous proteases, and there was thus a prejudice against adding a catalytic protein (such as collagenase) while blood plasma or constituents thereof are present, or *vice versa*, as one would have expected the catalytic protein (such as collagenase) to be degraded over time and its activity thus to be diminished; surprisingly, the present inventors established that the collagenase digestion works efficiently even in the presence of platelet lysate. Second, the inventors found that the cells obtainable by digestion in the presence of platelet lysate in the method according to the present invention thereof have excellent proliferative properties (see e.g. Examples 2B and 2C). Thus, preferably, in step (b) the stromal Wharton's Jelly or fraction thereof is subjected to enzymatic treatment in the presence of mammalian platelet lysate (PL), more preferably human platelet lysate (hPL).

In addition to that, platelet lysate is present in step (c). Preferably, in step (c) the platelet lysate (PL) is human platelet lysate (hPL). Preferably the at least one cell is expanded in a growth medium that does not comprise any added serum, in particular no fetal bovine serum (FBS).

In a particularly preferred embodiment, the at least one cell is expanded in a particular growth medium specifically appropriate for the method of the invention, optionally with supplements. Such a growth medium may comprise a chemically defined base medium and/or platelet lysate.

The method of the invention yields cells with particular properties, which are also described herein. Thus, it is particularly preferred that the cell obtainable by the method of the first aspect of the invention is a cell as described herein, as follows.

A mesenchymal stromal cell (MSC) is described herein. The MSC can also be referred to as the "cell described herein", or simply as the "cell". The MSC described herein is characterized in that it (a) expresses the APCDD1 gene, and (b) does not express the 3G5 gene. Preferably, the cell is an isolated cell. More preferably the cell is derived from the stromal Wharton's Jelly of the umbilical cord. Preferably, the cell is a primate cell. Thus, the cell is preferably not from a horse, i.e. not a horse cell. Also, the cell is preferably not from a rodent (e.g. mouse or rat), i.e. not a rodent cell (e.g. mouse cell or rat cell). Most preferably, the cell is a human cell.

Preferably the cell is a human cell which is derived from the stromal Wharton's Jelly and which expresses the APCDD1 gene.

Preferably, the cell further expresses the PPARG gene. It is still preferably not mutually exclusive that the cell further expresses the FLVCR2 gene. In the case of a human cell it is preferred that the human cell expresses at least one, preferably at least any combination of two, and most preferably all of the human leukocyte antigens HLA-A, HLA-B and HLA-C. Further, in the case of a human cell, it is preferred that the cell does not display the human leukocyte antigens HLA-DP, and /or HLA-DQ. Optionally also HLA-DR is not displayed. Preferably, the cell does not display any of the human leukocyte antigens HLA-DP, HLA-DR and HLA-DQ. Alternatively, HLA-DR is displayed at low levels.

The cell described herein is typically smaller than some MSCs that have been isolated by various methods according to the state of the art. For example, the cell described herein may have a smaller volume and/or a smaller surface area. Various methods for determining the cell volume or surface area are available, such as flow cytometry for determining the cell volume of detached cells, and determination of the cell surface under the microscope for adherent cells. Such methods are described herein below.

Preferably, the cell does not show any telomerase activity, and/or does not express any telomerase. Preferably the cell is multipotent. More preferably the cell is capable of differentiating into various cell types. It is particularly preferred that the cell has at least all of the following differentiation potentials: (a) adipogenic; (b) chondrogenic;(c) osteogenic.

Preferably, the cell is a mesenchymal stem cell.

The cell described herein is a cell obtainable by the method according to the first aspect of the invention. Preferred embodiments of the first aspect of the invention are combinable with the cell described herein in each and every combination, unless the context dictates otherwise. Alternatively, the cell described herein can therefore be described as a cell obtainable by the method according to the first aspect of the invention.

Described herein is also a population of cells. The population comprises at least one cell as described herein. More preferably the cell population comprises a multitude of cells, of which at least 80 % (by cell number), preferably at least 90 % (by cell number), more preferably at least 95 %, such as at least 96 %, at least 97 %, at least 98 %, at least 99 % (each by cell number) are cells as described herein. All preferred descriptions of the cell described herein apply also to the cell population described herein. Preferably at least 70 %, preferably at least 80 %, more preferably at least 90 %, such as at least 95 %, at least 96 %, at least 97 %, at least 98 % or at least 99 %, most preferably 100 %, of the total number of cells are cells as described herein, and optionally one or more of its preferred forms, alone or in combination. The cell population is positive for expression of the APCDD1 gene.

Preferably, the population is an isolated population of cells.

The cell population is preferably further characterized in that the volume and/or surface area of the cells in the cell population is similar, or, alternatively, in that a high percentage of the cells has a small volume and/or surface area, as described in detail below. The volume is normally correlated to the forward scattered light (FSC) determinable by flow cytometry.

Preferably, the cell population is further characterized in that at least 70 % of the cells (by cell number) or more are viable. This represents a significant advantage over the prior art, wherein the percentage of viable cells is lower. More preferably, at least 75 % of the cells (by cell number) are viable. More preferably, at least 80 % of the cells (by cell number) are viable. Preferably, viability is determined immediately after isolation of the cells, i.e. prior to expansion.

Preferably, the cell population does not comprise any perivascular cell. A marker for perivascular cells is 3G5. Therefore, this can alternatively be described in that the cell population does not comprise any cell which expresses the marker 3G5 and/or any cell which displays 3G5 on its surface.

Preferably, the cell population does not comprise any epithelial cell. A marker for epithelial cells is Epithelial cell adhesion molecule (EpCAM). Therefore, this can alternatively be described in that the cell population does not comprise any cell which expresses the marker EpCAM and/or any cell which displays EpCAM on its surface.

Preferably, the cell population does not comprise any endothelial cell. A marker for endothelial cells is CD31. Therefore, this can alternatively be described in that the cell population does not comprise any cell which expresses the marker CD31 and/or which displays CD31 on its surface.

Preferably the cell population is obtainable by a method according to the first aspect of the invention, particularly when step (c), i.e. the expansion step, is comprised.

Alternatively, the cell population described herein can therefore be described as a cell population obtainable by the method according to the first aspect of the invention, comprising step (c).

## Detailed Disclosure of the Invention

The following detailed description discloses specific and/or preferred variants of the individual features of the invention. The present invention also contemplates as particularly preferred embodiments those embodiments, which are generated by combining two or more of the specific and/or preferred variants described for two or more of the features of the present invention.

A person of ordinary skill in the art will appreciate that the invention described herein is susceptible to variations and modifications other than those specifically described. Thus, it will be apparent to the person of ordinary skill in the art that the present disclosure includes all such variations and modifications. The disclosure also includes all of the entities, compounds, features, steps, methods or compositions referred to or indicated in this specification, individually or collectively, and any and all combinations or any two or more of said entities, compounds, features, steps, methods or compositions. Thus, unless specifically stated otherwise herein or the context requires otherwise, reference to a single entity, compound, feature, step, method or composition shall be taken to encompass one and a plurality (i.e. more than one, such as two or more, three or more or all) of those entities, compounds, features, steps, methods or compositions.

The present disclosure is not limited in scope by the specific embodiments described herein, which are provided herein for the purposes of illustration and of exemplification. Functionally or otherwise equivalent entities, compounds, features, steps, methods or compositions are within the scope of the present disclosure.

Nothing herein is to be construed as an admission that the present invention is not entitled to antedate a specific teaching.

This document is to be read with a mind willing to understand and with the best objective intention to make technical sense out of it. All terms used herein are to be read in giving the words the meaning and scope which they normally have in the relevant art, unless in particular cases this document gives one or more words a special meaning, by explicit definition or otherwise. In the event that definitions provided herein should partially or fully diverge from definition in some or all of the literature, the definitions herein shall prevail.

Unless specifically stated otherwise or the context requires otherwise, each embodiment, aspect and example disclosed herein shall be taken to be applicable to, and combinable with, any other embodiment, aspect or example disclosed herein.

Unless specifically defined otherwise, all technical and scientific terms used herein have the same meaning as commonly understood by one of ordinary skill in the art (e.g., in genetics, molecular biology, gene expression, cell biology, cell culture, stem cells, stromal cells, neonatology, regenerative medicine, anatomy, histology, immunology, immunohistochemistry, protein chemistry, and biochemistry). Textbooks and review articles published e.g. in English typically define the meaning as commonly understood by one of ordinary skill in the art.

The expression "and/or", e.g., "X and/or Y" shall be understood to mean either "X and Y" or "X or Y" and shall be taken to provide explicit disclosure of "and", of "or" and of both meanings ("and" or "or").

As used herein, unless specified otherwise, the terms "about", "ca." and "substantially" all mean approximately or nearly, and in the context of a numerical value or range set forth herein preferably designates +/- 10 %, more preferably +/- 5 %, around the numerical value or range recited or claimed. Terms like "substantially not", "substantially absent" etc., with reference to a characteristic of a cell, mean that the respective characteristic cannot be detected on a cell with analytical methods normally used in the respective area according to the state of the art, and/or that substantially all cells in a population so described do not have the respective characteristic.

Unless expressly specified otherwise, the word "comprise", or variations such as "comprises" or "comprising" is used in the context of the present document to indicate that further members may optionally be present in addition to the members of the list introduced by "comprising". It is, however, contemplated as a specific embodiment of the present invention that the term "comprising" encompasses the possibility of no further members being present, i.e. for the purpose of this embodiment "comprising" is to be understood as having the meaning of "consisting of".

Unless expressly specified otherwise, all indications of relative amounts regarding the present invention are made on a weight/weight basis. Indications of relative amounts of a component characterized by a generic term are meant to refer to the total amount of all specific variants or members covered by said generic term. If a certain component defined by a generic term is specified to be present in a certain relative amount, and if this component is further characterized to be a specific variant or member covered by the generic term, it is meant that no other variants or members covered by the generic term are additionally present such that the total relative amount of components covered by the generic term exceeds the specified relative amount; more preferably no other variants or members covered by the generic term are present at all.

The term "allogeneic" is used herein to describe anything that is derived from a different individual of the same species. Two or more individuals are said to be allogeneic to one another when the genes at one or more loci are not identical.

The term "area" or "surface area" or "cellular surface area", with respect to a cell, refers to the surface area of the cell as viewed from atop. In the context of the present disclosure, the 2-dimensional surface area of a cell is determined while the cell is in adherent culture, i.e. the cell adheres to the culture plate. It is considered advantageous for reproducibility of determination that cells are in a low-confluence state because it is believed that at low confluence the cells do not significantly inhibit each other by contact inhibition. Methods for determination of the surface area are indicated herein below.

The expressions "average surface area" and "average area" refer to the average of the surface areas of two or more individual cells, wherein in a first step first the area of each individual cell is determined as described above, and then in a subsequent second step the average area is determined as the arithmetic mean of the areas of individual cells, as determined in the first step. Typically at least 35 cells are subjected to the first and second step for determination of the average area; in other words, the average represents the average of at least 35 cells.

The term "autologous" is used herein to describe anything that is derived from the same subject. As an example, "autologous cell" refers to a cell derived from the same subject. Transplantation of autologous cells is sometimes considered advantageous because it overcomes the immunological barrier which otherwise results in rejection.

A cell is a biological entity composed of cytoplasm enclosed within a membrane. As used herein, the term "cell" preferably refers to a eukaryotic cell. The term "cell" can refer to a stem cell or to a cell lacking stem cell properties, such as a terminally differentiated cell or even a dead cell. Typically, the cell is a cell originating from a multicellular organism or from a part thereof. Preferably, the cell is isolated from said multicellular organism, i.e. not physically connected to and/or nutritionally independent from the multicellular organism from which it originates. A stromal cell is a preferred cell as described herein.

The terms "cell population" and "population of cells" interchangeably refer to an ensemble of a plurality of cells. In the narrowest embodiment, a cell population comprises two cells, but more typically a multitude of cells. The term encompasses both populations of essentially identical cells, such as cell populations originating from a single clonogenic cell, as well as heterogeneous cell populations, i.e. cells having heterogeneous origin and/or which differ from each other in at least one functional or structural characteristic. The term encompasses populations of suspension cells only, populations of adherent cells only and mixed populations of suspension and adherent cells. A cell population may comprise both stem cells and cells that lack stem cell properties. In another but not mutually exclusive example, a cell population comprises both living cells and dead cells.

Unless the context dictates otherwise, "CD", typically used together with a number (e.g. "CD34"), stands herein for "Cluster of Differentiation".

The term "clonogenic" refers to the capability of a cell to expand, thereby giving rise to a multitude of daughter cells, which are normally genotypically identical. A "clonogenic" cell can also be referred to as "Colony Forming Unit", or "CFU".

The term "collagenase" as used herein refers to an enzyme (E.C. 3.4.24.7) that is capable of breaking peptide bonds in collagen. In general, collagenase can assist in destroying extracellular structures. The term does not imply any specific limitations on the type or origin of the collagenase, unless specified otherwise. The collagenase may be recombinant or from its natural source. Many commercially available collagenases comprise other enzymatic activities, in addition to E.C. 3.4.24.7. Such commercially available collagenases are suitable in the context of the present invention (see Examples).

The expression "confluence" is used herein to describe the proportion of the ground surface of a culture vessel which is covered by cells, typically adherent cells. Confluence is typically indicated as "% confluence".

The expressions "% confluence" or "confluence percentage" refer to the percentage of the ground surface of a culture dish that is covered by cells, typically adherent cells, i.e. the summation of the total area of cells relative to the ground surface of the culture dish. For example, 50 percent confluence means that substantially half of the surface is covered and there is still room for cells to grow. 100 percent confluence means the surface is completely covered by the cells, and no more room is left for the cells to grow as a monolayer. In the context of the present invention, cells are typically passaged before reaching 100 percent confluence in order to optimally maintain their proliferation phenotype.

The expression "low confluence" means a confluence percentage of 50 % or less, typically 10 to 50 %. The expressions "sub-confluent" and "sub-confluence mean a confluence percentage of more than 50 % but not more than 80 %.

The term "culture vessel" or "cell culture vessel" generally refers to a vessel that is suitable for cultivation of mammalian cells. Typical culture vessels are flasks, plates and stacks (whereby stacks are typically composed of a multitude of plates stacked on top of each other). Particularly suitable culture vessels in the context of the present inventions are culture vessels which allow for adherence of cells at the bottom of the culture vessel; such vessels are flasks, plates and stacks with a planar ground surface.

With respect to a culture vessel, the terms "surface" or "ground surface" are interchangeably intended to refer to the inner ground surface of the culture vessel, i.e. that surface to which cells with adherent properties will normally adhere while grown in that culture vessel. Normally, said surface is at the inner bottom of the culture vessel and is confined solely by the side walls of the culture vessel.

The term "density" or "cell density" refers to the number of cells per two-dimensional area, such as in particular the number of cells per $cm^2$, typically per $cm^2$ ground surface of the cell culture vessel, unless the context dictates otherwise. Typically, the term is used to describe how many cells are seeded into to the culture vessel, e.g. at the onset of a particular passage. The total ground surface of said culture vessel and the total number of cells seeded in that culture vessel then define the cell density per $cm^2$ of said culture vessel at the onset of said passage. In practice, the cell number used as inoculum at the beginning of a passage (I) is suitably determined by cell counting before the beginning of said passage; I is thus a function of the cell density and the ground surface of the culture dish. Unless explicitly stated otherwise, "cell density" refers to the average cell density over the entire ground surface of the culture vessel; nevertheless, it is preferred in the present invention that the cells are evenly distributed throughout the entire ground surface of the culture vessel.

The term "diameter" or "cell diameter", with respect to a cell, refers to the longest diameter of a cell as viewed from atop. In the context of the present document, the diameter of a cell is determined while the cell is in adherent culture, i.e. the cell adheres to the culture plate. It is important that cells are in a sub-confluence state. Sub-confluence is considered advantageous for reproducibility of determination because it is believed that sub-confluent cells do substantially not inhibit each other by contact inhibition. For determination of the diameter, one or more cells are photographed or otherwise visually observed or recorded, typically with a microscope, from the dimension perpendicular (i.e. 90 °) to the surface of the culture plate to which the cell or cells adhere. The boundaries of the area are manually defined on a graphical image (i.e. photograph) of the cell. Preferably, the culture vessel is not agitated prior to recording the graphical image. In case of a bipolar cell, the diameter is the distance between the two poles of the cell. For multipolar, e.g. triangular, cells, the diameter is the distance between those two poles of the cell that are furthest apart from each other. In each case, "distance" means the shortest straight connection line between the respective two poles.

The term "average diameter" refers to the average of the diameters of two or more individual cells, wherein in a first step the diameter of each individual cell is determined as described above, and then in a subsequent step the average diameter is determined as the arithmetic mean of the cell diameter determined in the first step.

The terms "expand" or "expanding", as used herein, generally refer to the proliferation of a cell or of a population of cells. Typically, expansion within the context of the present invention occurs *in vitro* or ex *vivo.* Typically, expansion is an activity or process that is subsequent to the isolation of cells. Typically, during expansion the total number of cells increases. The increase of the cell number can be described by referring to "cumulative Population Doubling(s)" or by referring to "Passage Number(s) ("P"), or by indicating the total number of cells at the respective stage, etc.

The terms "express", "expressed", and "expression", "gene expression" and the like, as used herein, relate to the use of information from a gene in the synthesis of a functional gene product. Gene expression comprises at least the transcription, and optionally comprises one of more additional features, optionally selected from the open list comprising RNA editing, translation and post-translational modification. When gene expression is determined, the presence of an expression product, such as non-edited or edited RNA, or even the encoded protein, is determined. The above terms, used in connection with a particular gene or locus, intend to specify the expression of the genetic information from that gene or locus; for example, when it is said that APCDD1 is expressed, it is meant to say that the APCDD1 gene is expressed.

The term "extract", as used herein, refers to the isolation from a biological environment. Depending on the context, the term can mean "to extract" (verb) or the physical entity that has been extracted (e.g. a cell or a cell population obtained by extraction from its natural environment). For example, when cells are extracted from the umbilical cord, the cells are isolated from their original location in the umbilical cord and/or separated from other anatomical areas of the umbilical cord. Preferably, an extracted cell is a cell outside a living body and/or physically separate from blood vessels/and or physically separate from other cells of the same organ or tissue and/or physically separate from non-cell anatomical areas of the same organ or tissue.

As used herein, the term "flow cytometry" refers to a laser- or impedance-based, biophysical technology suitable for cell counting, cell sorting, analysis of cell properties, and biomarker detection (such as, in particular, detection of cell surface molecules, such as Cluster of Differentiation (CD) molecules). Flow cytometry requires cells in suspension; in order to analyze adherent cells, these need to be detached from the substrate, e.g. culture vessel, to which thy adhere, e.g. by enzymatic treatment such as trypsinization, by which they become cells in suspension. The cells in suspension, i.e. cells in a stream of fluid, are passed through an electronic detection apparatus (flow cytometry apparatus). The flow cytometry apparatus analyzes the cell, e.g. based upon the specific light scattering of each cell. A commercial flow cytometry apparatus can be used, such as FACSAria III flow cytometer (BD Biosciences). The data generated by flow-cytometers can be plotted in a single dimension, to produce a histogram, or in two-dimensional dot plots or even in three dimensions. Plots may be made using scales of choice, such as linear or logarithmic scales. The regions on these plots can be sequentially separated, based on fluorescence intensity, by creating a series of subset extractions, termed "gates".

"Fluorescence-activated cell sorting", or interchangeably "FACS", as used herein, is a specialized type of flow cytometry. FACS is a method for sorting a heterogeneous mixture of biological cells into two or more populations, based upon the specific light scattering and/or fluorescent characteristics of each cell. The type of fluorophore used as label for FACS is not particularly limited; sometimes, fluorophores are attached to an antibody that recognizes a target feature, such as a cell surface protein (such as, in particular, detection of cell surface molecules, such as Cluster of Differentiation (CD) molecules). A fluorophore may alternatively be attached to a chemical entity with affinity for the cell membrane or another cellular structure. Each fluorophore has a characteristic peak excitation and emission wavelength, which is detected by the apparatus suitable for FACS. A commercial apparatus can be used, such as FACSAria III flow cytometer (BD Biosciences).

The term "heterologous" as used herein describes something consisting of multiple different elements. For example, the transfer of one individual's cell into a different individual constitutes a heterologous transplant. A heterologous gene is a gene derived from a source other than the subject.

The terms "immortal" and "immortality", as used herein, designate cells that can divide without being subject to the Hayflick limit (Hayflick limit is the point at which cells can no longer divide due to DNA damage or shortened telomeres). Immortal cells typically express the telomere-lengthening enzyme telomerase, but this is not a strict requirement.

The term "inoculum", as used herein, refers to the total number of cells by which a given cell culture (passage) is initiated in a given culture vessel.

By "isolated" is meant material that is substantially or essentially free from components that normally accompany it in its native state. For example, an "isolated cell" , as used herein, refers to a cell, which has been purified from the cellular and extracellular environment, such as tissue, which surround it in a naturally-occurring state, e.g., a mesenchymal stromal cell which has been removed from the umbilical cord tissue that is normally adjacent to the cell. In an alternative description, an "isolated cell" or and the like, as used herein, refer to *in vitro* isolation and/or purification of a cell from its natural cellular environment, and from association with other components of the tissue in which the cell normally resides. Unless the context dictates otherwise, an "isolated cell" need not necessarily be single cell devoid of any other cells; in contrast, even a population of two or more cells, such as a multitude of cells, can be referred to as "isolated" when said population of cells is isolated in the sense that it is substantially or essentially free from components that normally accompany such population of cells in its native state. For example, a population of mesenchymal stromal cells can be referred to as "isolated" when it has been removed from the original tissue, such as the umbilical cord. In accordance with this definition of the word "isolated", "to isolate", as used herein, is the verb that describes the activity to obtain "isolated" material, such as e.g. an isolated cell.

The term "lineage-committed" as used herein refers to a progeny cell that is capable of mitotic divisions, but typically incapable of an unlimited number of mitotic divisions. Eventually, the progeny of a lineage-committed cell is determined to differentiate.

As used herein, the term "medium", is meant to refer to an aqueous mixture suitable for cultivation of cells, particularly mammalian cells. The aqueous mixture is typically a solution, although suspensions and colloidal mixtures are also comprised. The medium is typically liquid, although some media can also be temporarily frozen, e.g. for storage purposes; in any case, a medium is used for cell culture when it is liquid. A medium may either be a "basal medium", which is a defined aqueous mixture, or a "growth medium", which is an aqueous mixture comprising a basal medium and at least one supplement, and which usually has the capacity to sustain viability of cells ex *vivo,* and optionally expansion of cells, also ex *vivo.* Unless expressly specified, the context dictates whether a basal medium or a growth medium is meant. A basal medium is preferably a chemically defined medium. A growth medium preferably comprises a basal medium (typically 85 % to 99.5 % vol./vol.) plus at least one human-derived, animal-derived or plant-derived supplement, such as serum, cell lysate, or particularly in the context of the present invention, platelet lysate.

The term "mesenchymal" is used herein to refer to a cell that belongs to a mesenchymal cell type, such as a fat cell, a cell of the connective tissues, bone, cartilage, blood, lymphatic vessels and blood vessels, as well as to a stromal cell or stem cell that is capable of differentiating into a mesenchymal cell type. Without wishing to imply any limitations on the present invention, mesenchymal cells are *in vivo* sometimes loosely packed and set in a gelatinous ground substance. Typically, a mesenchymal cell is of mesodermal origin (embryonic mesoderm or extra-embryonic mesoderm). Sometimes, a mesenchymal cell is a stromal cell (mesenchymal stromal cell) or stem cell (mensenchymal stem cell), or a cell derived from any of these.

The term "mesenchymal stromal cell", abbreviated "MSC", is used herein to refer to a cell of mesenchymal origin, i.e. of origin from stroma, such as particularly mesoderm-derived connective tissue. The tissue origin of a "mesenchymal stromal cell" is not further limited as such; however, according to preferred aspects of the description herein, a "mesenchymal stromal cell" originates from umbilical cord, more precisely from the stromal Wharton's Jelly of the umbilical cord, as described in detail herein. A mesenchymal stromal cell typically expresses the surface markers CD105 and CD73, and normally also CD90. Preferably, the "mesenchymal stromal cell" as described herein is characterized by the expression of CD105 (known as endoglin), CD73 (known as ecto 5' nucleotidase) and CD90 (also known as Thy-1. Mesenchymal stromal cells are characterized by substantially not expressing hematopoietic antigens, such as particularly CD45, CD34, CD14, CD19, or CD3. Typically, MSCs express MHC Class I molecules *in vitro,* but not Class II molecules unless stimulated, e.g. by interferon, in tissue culture. Thus, a typical surface marker phenotype of MSCs is CD105+, CD73+, CD90+, CD45-, CD34- CD14-, CD19-, CD3-, HLA DR-. While unequivocally identifying MSCs, this surface marker profile is complex. A typical characteristic feature of MSCs is the capacity to differentiate *in vitro* into a variety of cell types, typically including osteoblasts, adipocytes, and chondroblasts. Typically MSCs are plastic adherent *in vitro.* The term "mesenchymal stromal cell" does not inherently require that the cell so referred to is a stem cell; nevertheless, as described herein, the MSCs as described herein, or obtained according to the present invention, may have stem cell-like properties in preferred disclosures.

The term "mesenchymal lineage precursor cell" refers to a mesenchymal stromal cell which has the capacity to self-renew while maintaining multipotency and the capacity to differentiate into a number of cell types either of mesenchymal origin, for example, osteoblasts, chondrocytes, adipocytes, stromal cells, fibroblasts and tendons, and possibly also to cells of non-mesenchymal origin, for example neural cells and epithelial cells. The term "mesenchymal lineage precursor cell" refers thus to a cell which is able to differentiate into a variety of cell types, although as the cell is multipotent (as opposed to totipotent). Without wishing to be bound to any particular theory, it is understood that a "mesenchymal lineage precursor cell" is a cell of the mesoderm lineage. However, unless specified otherwise or the context dictates otherwise, the term "mesenchymal lineage precursor cell" does not imply any limitations on the tissue origin of the respective cell. Thus, in general, cells termed "mesenchymal lineage precursor cell" are not limited to a particular tissue origin and can originate e.g. from the bone marrow, umbilical cord, adult peripheral blood, adipose tissue, trabecular bone and dental pulp. According to preferred aspects as described herein, the "mesenchymal lineage precursor cell" originates from umbilical cord, more precisely from the Wharton's Jelly of the umbilical cord, as described in detail herein. The term "mesenchymal lineage precursor cell" includes both parent cells and their undifferentiated progeny. The term "mesenchymal lineage precursor cell" also includes mesenchymal precursor cells, multipotent stromal cells, mesenchymal stem cells and their undifferentiated progeny. According to the description herein, it is strongly preferred that a "mesenchymal lineage precursor cell" is a stem cell, as defined herein, with the limitation that the stem cell is not totipotent.

The terms "multi" and "multiple" as used herein mean a multitude, i.e. any number of two or more.

As used herein, "multipotent" refers to a cell which is capable of giving rise to any of several mature cell types. The term "multipotent" encompasses progenitor cells and all progeny of such cells which is still multipotent. The term "multipotent" refers to a progenitor cell which has the potential to differentiate into more than one discrete cell type. Without wishing to be bound to a particular theory, it is understood that a multipotent stem cell sits atop a lineage hierarchy and can generate multiple types of differentiated cells. Without limitation, multipotent cells may been found in the umbilical cord, adipose tissue, cardiac cells, bone marrow, and dental pulp. Sometimes, multipotent cells are pluripotent.

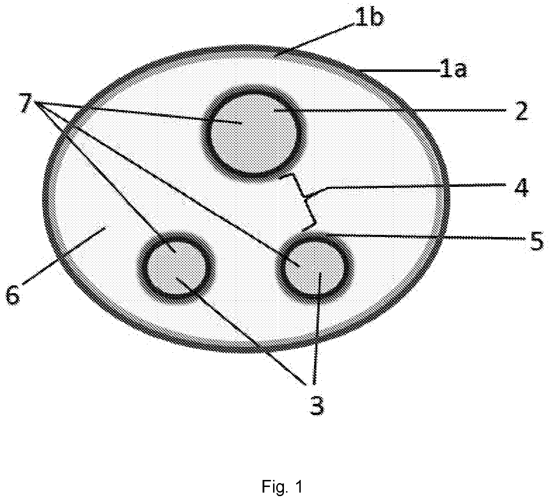

The term "perivascular" or "perivascular zone" or "perivascular area", as used herein, refers to a physical area or zone near a vessel, such as a blood vessel. The perivascular zone" or "perivascular area" is depicted as item (5) in Fig. 1. In particular, perivascular zone/area of the umbilical cord (5) refers to each zone/area around each blood vessel of the umbilical cord. The perivascular zone/area can suitably be defined as the space extending to 3 mm from the external walls of each of the umbilical cord vessels (WO 2004/072273 A1). By means of example, the perivascular zone/area has been described by Takechi et al., 1993 (Placenta vol. 14; p. 235-245).

The term "platelet lysate" refers to a preparation obtainable from blood platelets (also called thrombocytes). Platelets are obtainable from a donor, preferably a human donor, either isolated from collected units of whole blood and pooled, or collected by platelet apheresis: blood is taken from the donor and passed through a device which removes the platelets. Following their isolation, the platelets, e.g. (re)-suspended in plasma, are typically lysed, e.g. by one or more freeze/thaw cycle(s). Platelet lysate is available from a large number of commercially suppliers, e.g. Macropharma (Mouvaux, France), from clinical blood banks, e.g. Blood Bank of the Polyclinic in Modena (Italy), but can also be prepared directly from blood platelets, as described e.g. by Naaijkens et al., Cell Tissue Res., 2012, vol. 348, p. 119-130 and WO 2013/042095 A1.

The term "pluripotent" as used herein refers to a cell that has the potential to differentiate into any of the three germ layers (endoderm, mesoderm, ectoderm), but not into germ line cells. Typically, a pluripotent cell can differentiate into all cell types of an adult organism except for germ line cells. In one embodiment, a pluripotent cell is a cell that is able to differentiate into any of the mammalian body's cell types - without wishing to be bound to any theory, these may be approximately 260 cell types. A pluripotent cell can be self-renewing, and can remain dormant or quiescent within a tissue. A pluripotent cell complies preferably with all of the following: (i) is capable of proliferation in an undifferentiated state, (ii) maintains a normal karyotype during prolonged culture and (iii) maintains the capability to differentiate into any of the three germ layers (endoderm, mesoderm, ectoderm) even after prolonged culture.

The term "passage number", abbreviated "P", followed by a number (e.g. "P1"), refers to the number of times a culture of cells has been sub-cultured. Thus, the term "passage number" is a term useful for describing a status during expansion of the cells.

The term "Population Doubling", abbreviated "PD", is used herein to describe that the number of cells has doubled, e.g. in a time interval from an early time point $t_0$ to a later time point $t_{0+x}$. In that case, x designates the time interval during which the number of cells has doubled. For example $t_0$ may be the time point of primary isolation, or the time point at which a certain passage was initiated. Thus, the term "Population Doubling" is a term that refers to the expansion of cells. The term generally refers to the doubling of the number of cells *in vitro*. Population Doubling (PD) is calculated by the formula:

$$PD = \log(N/N_0)/\log 2$$

where

N$_0$ is the seeded cell number and
N is the harvested cell number
(Purpura et al., 2004, Stem Cells. Vol. 22, p. 39- 50).

The term "cumulative Population Doubling" is used herein to refer to the total number of times the cells in a cell population have doubled *in vitro,* since a particular time point, e.g. their primary isolation. Thus, also the term "cumulative Population Doubling" is a term that refers to the expansion of cells, generally *in vitro.* Suitably, the cumulative PD is determined for each passage. The cumulative PD number is calculated by the formula:

$$n = \log( N / N_0 )/\log2 + X$$

where

n = the cumulative PD number,
$N_0$ = is the seeded cell number (i.e. at the beginning of a passage),
N = the harvested cell number (i.e. at the end of said passage),
X = the cumulative PD number at the beginning of said passage.

$N_0$ is suitably determined by cell counting before the beginning of a passage; $N_0$ is a function of the cell density and the ground surface of the culture dish. N is suitably determined by cell counting after detachment, e.g. trypsinization, at the end of a passage.

The cumulative PD are additive (hence the X in the formula above); for example, when cells have undergone 5 cumulative PD during a first passage P1 and again 5 cumulative PD during a subsequent second passage P2, then the cells have undergone 10 cumulative PD during the total course of P1 plus P2. Freshly isolated cells have a cumulative PD = 0 by definition. Thus, at the onset of P0, X = 0. Since it is sometimes difficult to determine $N_0$ prior to P0 (and hence, to determine n for P0), the cumulative Population Doublings are optionally determined only from P1 onwards; however, where this is the case, this is specifically indicated in the present disclosure.

It is possible to indicate the cumulative PD as an estimate rounded off to the nearest whole number. When defined culturing conditions are used, then it is possible to arithmetically convert the passage number into cumulative PD, and *vice versa.*

The term "progenitor cell" is used herein to refer to a cell, which may be a stem cell and/or a descendant of a stem cell. Progenitors are typically the descendants of stem cells, only they are more constrained in their differentiation potential or capacity for self-renewal. Progenitor cells may be e.g. multipotent or unipotent.

The terms "secrete" or release", when used herein in connection with a cell, generally refer to any material that is externalized from the cell into the external environment. Said material has typically been produced and/or modified by the cell, although this is not a requirement. For example, some cells secrete proteins and/or regulatory molecules such as hormones, prostaglandins and neurotransmitters, and/or microvesicles and/or exososomes, into their respective external environment. For example, a prostaglandin produced by a cell may be externalized into the cell's environment. Said terms, as used herein, are neither limited to *in vivo* secretion/in *vivo* environment nor to *in vitro* secretion/in *vitro* environment, unless the context dictates otherwise.

The term "cell volume" or simply "volume", with respect to a cell, refers to the cell volume as determinable by flow cytometry, in particular FACS analysis, more particularly by determination of forward-scattered light (FSC). In the context of the present disclosure, the volume of a cell is determined while the cell, e.g. a detached cells, such as a trypsinized cell (following adherent culture) or a collagenase-released cell (following isolation from tissue), is in suspension, i.e. the cell does not adhere to a culture plate or to a tissue. Determination of SSC and optionally of the cell volume is described herein below.

The term "average volume" or "average cell volume" refers to the average of the volume of two or more individual cells, wherein in a first step the volume of each individual cell is determined by flow cytometry as described herein, and then in a subsequent second step the average volume is determined as the arithmetic mean of the cell volume of individual cells, as determined in the first step. Typically at least 1,000 cells are subjected to the first and second step for determination of the average cell volume; in other words, the average represents the average of at least 1,000 cells.

The term "stem cell" is used herein to refer to a eukaryotic cell that is capable of giving rise to phenotypically and genotypically identical daughters ("self-renawal") as well as to at least one other final cell type (e.g., terminally differentiated cells). The term "stem cell" refers to a cell that may be totipotent, pluripotent or multipotent, as well as to a progenitor and/or precursor cell derived from the differentiation thereof and/or a lineage committed progeny cell. In general, the term "stem cell" can refer to an adult stem cell or to an embryonic stem cell. The term "stem cell" may optionally refer to an induced stem cell, such as an induced pluripotent stem cell, but more preferably refers to a cell that has not been induced.

The terms "sternness" or "stem cell-like property"/"stem-cell-like properties" are used interchangeably to designate the property of a cell, in particular the capacity to self-renew and the ability to generate differentiated cells. More explicitly, cells having stem-cell-like properties can generate daughter cells identical to their mother (self-renewal), as well as produce progeny with more restricted potential, such as differentiated cells. Cells with stem-cell-like properties are characterized by a certain degree of potency, i.e. potential to produce differentiated progeny. Some cells with stem cell-like properties may be capable to generate multiple differentiated cell types (e.g. "multipotent" or "pluripotent"), while others may be capable of producing one type of differentiated cell ("unipotent"). Although cells with stem cell-like properties have the capacity to self-renew, most somatic cells, including those with stem-cell like properties, when cultured *in vitro* are capable of expanding for a finite number of cumulative population doublings prior to replicative arrest or senescence. In preferred embodiments the mesenchymal stromal cells according to the present disclosure are mesenchymal stromal cells with stem cell-like properties.

In general, "stroma" is the part of a human or animal tissue or organ that has a role in mechanical supporting the tissue, such as providing connective, structural support, and/or (preferably and) in functional supporting the tissue. Thus, within the living body, the stroma is the connective, functionally supportive framework of a biological tissue, or organ (as opposed to the parenchyma which is the functional aspect of a tissue). The term "stroma" as used herein, refers to the connective tissue cells of any organ, for example without limitation in the umbilical cord, uterine mucosa (endometrium), prostate, bone marrow, lymph node and the ovary. In the respective organ, the stroma is understood to support the function of the parenchymal cells of that organ. Thus, the stroma consists of all the parts which do not conduct the specific functions of the organ, for example, connective tissue, blood vessels, nerves, ducts, etc. Thus, the stroma of the umbilical cord is a matrix comprised of the umbilical cord except the vessels, the perivascular area and the amniotic epithelia. The term stroma can refer to stroma of an entire organ or to a specific anatomical area thereof. In particular, the term stroma can refer to stroma of the umbilical cord in its entirety or to a specific anatomical area thereof, i.e. stroma contained in a specific section of the umbilical cord.

The term "stromal" as used herein refers to anything present in the stroma or derived from the stroma. The term is not limited to the location of the "stromal" material (e.g. stromal cell) to reside inside the stroma, it also refers to material (e.g. a cell) that was present in the stroma in an organ of a living human or animal body, but that has subsequently been extracted or isolated from the organ so that is no longer present in in an organ of a living human or animal body. In other words, the term "stromal" refers to the specific *in vivo* origin of material (e.g. a cell), irrespective of whether the material (e.g. cell) has been extracted or isolated. Material (e.g. cell) that has been extracted or isolated from the stroma can also be referred to herein as "stroma-derived", "derived from stroma", or the like. In some preferred embodiments of the description herein the stromal material (e.g. stromal cell) is or has been extracted or isolated, so that it is not, or no longer, present in in an organ of a living human or animal body.

Thus, a "stromal cell" is a biological cell which is present in the stroma or derived from the stroma, i.e. extracted or isolated, so that it is not, or no longer, present in in an organ of a living human or animal body. A stromal cell is alive unless otherwise specified, or the context dictates otherwise. In many organs fibroblasts and pericytes are among the most common types of stromal cells. As an illustrative example, a stromal cell may be a cell present in the stroma (e.g. the stroma of the umbilical cord) or a cell that has been isolated (e.g. extracted) - and optionally expanded - from the stroma (e.g. the stroma of the umbilical cord). Stromal cells are typically non-malignant cells.

The term "syngeneic" is used herein to describe anything that is derived from individuals or cells having identical genotypes, such as identical twins or animals of the same inbred strain, or their cells.

The term "totipotent" as used herein refers to a cell that is able to divide and produce all of the differentiated cells in an organism. Typically, a totipotent cell is able to give rise to a complete embryo. In mammals, typically only the zygote and subsequent blastomeres are totipotent.

The term "umbilical cord", also called "naval string", refers to the conduit between a newborn baby and the placenta in placental animals. During prenatal development, the umbilical cord is physiologically and genetically part of the fetus. Although an umbilical cord is present throughout embryogenesis, where it connects the placenta with the developing embryo or fetus, in the present specification the term specifically refers to the umbilical cord of a baby that has already been born. In humans, the umbilical cord normally comprises three blood vessels, i.e. two arteries (the umbilical arteries) and one vein (the umbilical vein). The three blood vessels are typically embedded in the Wharton's Jelly. Neither the placenta or parts thereof, nor the newborn, or parts thereof, are part of the umbilical cord; that term refers exclusively to the conduit.

The term "unipotent" refers to a cell with stem-cell like properties that can only produce one type of differentiated descendant. Such unipotent cell may also be referred to as progenitor cell.

The term "viable" as used herein, when referring to a cell, means that the cell is a living cell. Living cells are typically metabolically active. Although several different coloring agents and fluorochromes can be used to stain non-viable cells, preference is given herein to staining with 7-amino actinomycin D (7-AAD). 7-AAD is a membrane impermeable dye that is generally excluded from viable cells. It binds to double stranded DNA by intercalating between base pairs in G-C-rich regions. 7-AAD can be excited at 488 nm, e.g. with an argon laser, and emits fluorescence at a maximum wavelength of 647 nm. Using 7-AAC, viability of cells can be determined by flow cytometry, in particular FACS (fluorescence-activated cell sorting).

The term "vessel" (not to be confused with a "culture vessel" - see there), as used herein, generally refers to a tube or canal (such as a vein or an artery) in which a body fluid (such as blood) is contained and conveyed or circulated. Unless the context dictates otherwise, the term "vessel" is used herein also to specifically refer to the vessels of the umbilical cord. In humans and many other mammals, the umbilical cord normally contains three blood vessels, i.e. one vein, which *in vivo* carries oxygenated, nutrient-rich blood to the fetus, and two arteries that *in vivo* carry deoxygenated, nutrient-depleted blood away; although occasional cases have been reported where only two vessels (one vein and one artery) are present in the umbilical cord.

The term "Wharton's Jelly", abbreviated "WJ", refers to a gelatinous substance of placenta animal origin that comprises cells. Typically, the Wharton's Jelly comprises stromal cells, more particularly mesenchymal stromal cells. Further, the Wharton's Jelly typically comprises mucopolysaccharides, such as hyaluronic acid and chondroitin sulfate, as well as water. Although Wharton's Jelly has also been described in vitreous humor of the eyeball, it is strongly preferred in this specification that the Wharton's Jelly is a part of the umbilical cord of a newborn placenta animal, preferably a mammal, such as a human. In that case, the Wharton's Jelly can also be referred to as substantia gelatinea funiculi umbilicalis. In other words, it is strongly preferred that the Wharton's Jelly as used herein is of umbilical cord origin, even more preferred human umbilical cord origin. As described in the following, an umbilical cord normally comprises Wharton's Jelly in the perivascular area (5), in the intervascular area (4) and in the subamniotic area (1b).

"Perivascular Wharton's Jelly" or "PVWJ" or "VPWJ" all synonymously refer to the Wharton's Jelly of a specific anatomical area of the umbilical cord, namely the anatomical area of the Wharton's Jelly that is located around the vessels of the umbilical cord (5). For the case of a human umbilical cord, which represents a preferred embodiment, the PVWJ is located in the area extending from the outer surface of the vessel 3.0 mm or less outwards (for review see Davies et al., Stem Cells Translational Medicine, 2017, vol. 6, p. 1620-1630). Therefore, when the area that is located within 3.0 mm from the outside wall of the vessels of the human umbilical cord is entirely removed, the Perivascular zone, and thus any perivasculat Wharton's Jelly comprised therein, as the case may be, is entirely removed. For illustration see also Fig. 1. The abbreviations "PVWJ" and "VPWJ" are also used herein synonymously to refer to cells originating from the perivascular zone, including the "Perivascular Wharton's Jelly", as the case may be, e.g. in the Figure legends. Perivascular Wharton's Jelly (PVWJ)-derived mesenchymal stromal cells can also be reffered to as "perivascular zone-derived mesenchymal stromal cells".

The term "subamniotic stroma" refers to the zone (1b) directly adjacent to the amniotic epithelium (1a) of the umbilical cord. For the case of a human umbilical cord, which represents a preferred embodiment, the subamniotic stroma is located in the area extending from the inner surface of the amniotic epithelium 3.0 mm or less inwards.

The term "subamniotic Wharton's Jelly" refers to the Wharton's Jelly in the subamniotic stroma (1b) directly adjacent to the amniotic epithelium (1a) of the umbilical cord. For the case of a human umbilical cord, which represents a preferred embodiment, the subamniotic Wharton's Jelly is located in the area extending from the inner surface of the amniotic epithelium 3.0 mm or less inwards.

The term "stromal Wharton's Jelly", abbreviated "SWJ", as used herein, refers to the Wharton's Jelly of the umbilical cord that excludes the perivascular area (4). Thus, the term "stromal Wharton's Jelly" does not include the perivascular area (4) and consequently does not include cells from the perivascular area (4). While there has been some debate in the art on whether or not the perivascular area comprises mesenchymal stromal cells, such debate is not critical for the present invention, at least because the present invention does not contemplate the isolation of mesenchymal stromal cells from the perivascular area, at least for the reason that the perivascular area is not a source of cells according to the present invention. Unless explicitly stated otherwise, the subamniotic stroma (1b) is comprised in the stroma referred to herein. Unless explicitly stated otherwise, the subamniotic Wharton's Jelly is comprised in the Wharton's Jelly referred to as "stromal Wharton's Jelly" herein. The subamniotic Wharton's Jelly is comprised in the subamniotic stroma. The abbreviation "SWJ" is also used herein to refer to cells originating from the "stromal Wharton's Jelly", e.g. in the Figure legends. Thus, with reference to Fig. 1, the stroma (and thus the origin of the Wharton's Jelly) in its broadest definition, comprises all those areas of the umbilical cord which are not part of any of the following: (1a) amniotic (or epithelial) layer; (2) umbilical vein; (3) umbilical arteries; (5) perivascular area; (7): umbilical cord blood. In other words, in this broadest definition, the stroma (and thus the origin of the Wharton's Jelly) comprises (6) the Wharton's Jelly in the lumen of the umbilical cord plus (1b) the sub-amniotic (or mesenchymal) layer); plus (4) the intervascular area. Preferably, unless explicitly stated otherwise, the subamniotic Wharton's Jelly (1b) is comprised in the Wharton's Jelly referred to as "stromal Wharton's Jelly" herein. Preferably, (4) the intervascular area is comprised in the Wharton's Jelly referred to as "stromal Wharton's Jelly" herein. Thus, the stroma (and thus the origin of the Wharton's Jelly) in its broadest definition, comprises all those areas of the umbilical cord which are schematically depicted as 1b, 4 and 6 in Fig. 1. In the event that the anatomical definitions provided herein with respect to the umbilical cord should partially or fully diverge from definition in some or all of the literature, the defintions herein shall prevail.

It has been proposed in the literature (Davies et al., Stem Cells Translational Medicine, 2017, vol. 6, p. 1620-1630) that, from a pragmatic perspective, it is only (1a) the cord lining and (5) the perivascular Wharton's Jelly that can be distinctly dissected from the remaining regions or zones. The present invention contemplates, in one embodiment, that distinct dissection, i.e. the separation of the stromal Wharton's Jelly from (1a) the cord lining and (5) the perivascular zone, including the perivascular Wharton's Jelly.

The present invention is based on several findings, which are interrelated and thus together lead the inventors to arrive at the various aspects of the invention, which will all be described individually in the following. Therefore, the aspects described in this specification are all combinable with each other, even if not explicitly so described, unless the context clearly dictates otherwise. All aspects of the present invention are based *inter alia* on the finding that the stromal Wharton's Jelly of the umbilical cord holds cells with unique properties in a particularly expansion-competent state, and that a specific isolation procedure reliably provides these potentially multi-potent stromal cells so that they can be maintained and expanded in culture.

No human embryos are, or have been, destroyed in the context of the present invention. In particular, neither the method nor the cell nor the cell population of the present invention requires the destruction of a human embryo.

Method of obtaining a mesenchymal stromal cell

In a first aspect, the invention relates to a method for obtaining a mesenchymal stromal cell. The method comprises (a) isolating the stromal Wharton's Jelly or a fraction thereof, and (b) subjecting the stromal Wharton's Jelly or fraction thereof to enzymatic treatment, thereby releasing at least one cell from the stromal Wharton's Jelly or fraction thereof.

In particular, with reference to Fig. 1, which represents a cross-section of the (human) umbilical cord and schematic view of sections (anatomical areas) of the (human) umbilical cord, the method can be described as: a method for obtaining a mesenchymal stromal cell, wherein the method comprises (a) isolating the stromal Wharton's Jelly (1b, 4, 6) or a fraction thereof, and (b) subjecting said stromal Wharton's Jelly or fraction thereof to enzymatic treatment, thereby releasing at least one cell from the stromal Wharton's Jelly or fraction thereof.

The cell obtained after step (b) may be pluripotent, or, more typical, multipotent. Additional steps are optionally but preferably comprised, in particular an expansion step, which is described in detail below.

In the method of the present invention, the Wharton's Jelly is isolated from an umbilical cord. This represents a paradigm shift with respect to the general trend in the art according to which bone narrow (BM)-derived mesenchymal stromal cells (MSCs) represent the most extensively studied population of adult MSCs and are considered as the gold-standard for MSC-based applications (e.g. Batsali et al., Blood, 2013 vol. 122, p. 1212). However, the aspiration of bone marrow is generally invasive and painful. A big advantage of the method according to the present invention over isolation from bone marrow (BM) is that umbilical cord is readily available at birth of a mammal, whereas surgery required for obtaining bone marrow is usually invasive and painful. Patients, donors and healthcare professionals are therefore reluctant to use bone marrow-derived MSCs, and likely would be even more so, if only reliable alternatives were available.

Thus, it is an advantage of the method according to the present invention that the method is an ex *vivo* method. Preferably, invasion into a living human or animal body is completely avoided. Thus, the method of the present invention preferably does not represent a method for treatment of the human or animal body by surgery or therapy. Thereby, pain aspects, handling aspects and ethical aspects are successfully addressed, compared to isolation from bone marrow as described in the literature.

A further key difference to several earlier studies and reports is that, according to the prior art MSCs can be efficiently isolated from the connective tissue that surrounds umbilical cord vessels (e.g. Batsali et al., Blood, 2013 vol. 122, p. 1212), whereas the present invention relies on isolation of MSCs from an area that excludes the perivascular zone, and thus the perivascular Wharton's Jelly. Thus, the inventors of the present invention have found that a specific anatomical area of the umbilical cord should be purposively selected in order to arrive at advantages as described herein. More specifically, the method according to the present invention is characterized by a specific anatomical area of origin of the cells and/or a specific sectioning and segmenting of the umbilical cord and/or a specific isolation step and/or optionally a specific expansion step, all as described in detail herein below and illustrated by the examples. It is important that preferred and/or optional embodiments and details of the specific anatomical area of origin of the cells, specific sectioning and segmenting of the umbilical cord, specific isolation step, and optional specific expansion step are combinable with each other according to the present invention in each and every combination, unless the context clearly teaches that a particular combination is not possible or not desired according to the present invention.

In the method of the present invention, a rapid isolation of stromal cells from the umbilical cord is advantageous. In a preferred embodiment, steps (a) and (b) together are conducted in a total time of less than 6 hours, more preferably less than 5 hours, more preferably less than 4 hours and most preferably less than 3 hours. Less than 3 hours includes time intervals of 0.5 to 2.5 hours and 1 to 2 hours. It is evident that a short duration of steps (a) and (b) together also means that the time interval of duration of step (a) itself is short.

Unless specified otherwise, all reagents, ingredients, culture vessels, containers and materials which are brought into direct or indirect contact with the cells are preferably sterile, and more preferably GMP grade.

A detailed description of the method and its embodiments follows.

Tissue origin

The first step of the method according to the method of the invention is step (a) characterized by isolating the stromal Wharton's Jelly or a fraction thereof from an umbilical cord. Step (a) is preferably a mechanical step, i.e. it does not normally rely on chemical or enzymatic steps for the purposes isolation. Thus, in a preferred embodiment, step (a) is no ingredient with enzymatic activity is added during step (a). As will be described further below, enzymatic treatment is, however, important for the subsequent step (b).

The word "fraction", when used in connection with the stromal Wharton's Jelly, is not particularly limited and generally means that, in some embodiments, less than 100 % of the total stromal Wharton's Jelly of an umbilical cord may be used in the method of the invention. For example, 10 to 99 %, such as 20 to 90 %, 30 to 80 %, 40 to 60 %, of the total stromal Wharton's Jelly may be used in some embodiments. Reasons for using less than 100 %, in some embodiments, may be manifold and may be due to experimental limitations in recovering 100 % of the stromal Wharton's Jelly of the umbilical cord, or purposely, e.g. when it is desired to use part of the stromal Wharton's Jelly for comparative experiments. Nevertheless, it is preferably desired to use a fraction as large as possible, i.e 80 % or more, of the stromal Wharton's Jelly, preferably, 90 % or more. The % values, unless indicated otherwise, refer to the % of the total volume of stromal Wharton's Jelly contained in one umbilical cord.

Thus, according to the present invention, the cells are isolated from the umbilical cord. The umbilical cord is of placental animal origin, but, preferably, the umbilical cord is of mammalian origin. In placental mammals, the umbilical cord is a structure that connects the placenta to the developing fetus, and immediately after birth to the newborn baby. Neither the placenta nor the newborn baby are part of the umbilical cord; the umbilical cord is confined to the connective tube between the placenta and the newborn baby.

The umbilical cord is obtained after birth. Preferably, the umbilical cord is obtained 24 hours after birth or less, such as 12 hours or less after birth, 6 hours or less after birth, 3 hours or less after birth, such as 2 hours or less after birth, and preferably 1 hour or less after birth. Thus, the umbilical cord can be stored, preferably for a maximum of 24 hours, after birth, preferably at low temperatures of 0 to 10 °C, preferably 2 to 8 °C, prior to isolation of cells. The umbilical cord is, however, not frozen prior to isolation of cells. Optionally, the umbilical cord is rinsed with sterile water or a sterile buffered aqueous solution prior to isolation of cells.

The umbilical cord is preferably maintained and handled under sterile conditions prior to and during the isolation of the cells. Optionally, the umbilical cord may additionally be surface-sterilized by brief surface treatment of the cord with, for example, an aqueous (70 % vol./vol. ethanol) solution or betadine, followed by a rinse with sterile water.

Preferably, the cells are isolated from a human umbilical cord. The human umbilical cord (UC) at term weighs approximately 40 g and has a mean diameter of ca. 1.5 cm (Raio et al., 1999, Eur. J. Obstet. Gynecol. Reprod. Biol., vol. 83; p. 131-135). Thus, preferably, the umbilical cord used according to the present invention is a human umbilical cord. A human umbilical cord subjected to the method of the present invention has preferably a length of approximately 40-65 cm, such as 44 to 51 cm.

Typically, the umbilical cord is isolated after birth of a healthy newborn from a healthy mother, preferably human mother. Preferably, said mother has refrained from smoking, consuming alcohol and consuming psychoactive pharmaceuticals during at least one month before giving birth.

When it is said that the stromal Wharton's Jelly or a fraction thereof is isolated from an umbilical cord, this preferably means that not more than one umbilical cord is used as starting material. However, it is also possible that more than one umbilical cord is used as starting material. In one embodiment, one umbilical cord is used as starting material in the method according to the present invention. In that embodiment, all starting material is autologous, i.e. no allogeneic umbilical cord material is part of the starting material, and consequently all cells obtainable by the method of the present invention originate from one single donor, per each run of the method.

It possible to use an entire umbilical cord as starting material, but it is equally possible to use a fraction of one umbilical cord as starting material. When one entire umbilical cord is used as starting material in the method according to the present invention, it can be said that the method is run at "full scale". Accordingly, the method according to a preferred aspect of the present invention, comprising steps (a), (b) and optionally (c), is referred to as "full scale" run when one entire umbilical cord is used as starting material. In this context the word "entire" does not exclude that minor parts of the cord, e.g. the two ends of the cord, are trimmed or clipped off and thus not included as starting material; it does also not exclude that the umbilical cord is sectioned and/or segmented during the method described herein; it rather means that essentially the entire amount of an umbilical cord is used as starting material. For the case of a human umbilical cord from term births, an entire umbilical cord typically weighs between 35 and 40 grams, or more precisely about 40 grams. In one embodiment, a full-scale run starts with 35 to 45 gram umbilical cord from a single donor. The amount of the starting material, i.e. the umbilical cord, has of course an influence on the total amount of cells obtained during downstream phases of the method according to the present invention. Unless expressly indicated otherwise, all amounts (e.g. cell numbers) given herein refer to a full scale run. The word "full-scale" is not in conflict with the term "fraction" of the stromal Wharton's Jelly, as long as a reasonable effort is made to recover as much of the stromal Wharton's Jelly from one umbilical cord as possible.

The outermost layer of the umbilical cord (also termed cord lining, cord tissue, or umbilical cord lining membrane, schematically depicted as 1a in Fig. 1), is not a desired source of cells in the method of the invention. As the umbilical cord itself is an extension of the placenta, the umbilical cord lining membrane is an extension of the amniotic membrane covering the placenta. The umbilical cord lining membrane comprises two layers: the amniotic (or epithelial) layer (also termed also termed umbilical cord lining membrane) and the sub-amniotic (or mesenchymal) layer. The human umbilical cord, for example, is covered by single/multiple layer(s) of squamous-cubic epithelial cells (Copland et al., Placenta, 2002; vol. 23, p. 311-321; Mizoguchi et al., J. Dermatol. Sci., 2004, vol. 35, p. 199-206); these cells are not desired to be isolated in the method of the invention. In one embodiment of the method of the invention it is desired that neither cells from the amniotic layer nor cells from the sub-amniotic layer are isolated, and thus such cells not used as starting material in the optional expansion further downstream.

The inner tissue architecture of the umbilical cord is composed of two arteries and one vein and a surrounding matrix of mucous connective tissue (stromal Wharton's Jelly). The Wharton's Jelly has been described to comprise fibroblast-like cells (Parry, 1970, J. Anatomy, vol. 107, p. 505-518) and occasional mast cells. In addition, the Wharton's Jelly comprises an amorphous ground substance which is rich in proteoglycans, mainly hyaluronic acid.

Preferably the umbilical cord used in the method according to the present invention is from a birth at term, also referred to as "full term". More precisely, the human umbilical cord used in the method according to the present invention is from a birth within 38 to 42 weeks of gestation, more preferably within 39 to 41 weeks of gestation. In one embodiment the human umbilical cord is from a natural (vaginal) birth; in an alternative embodiment, the human umbilical cord is from a non-natural birth, e.g. Cesarean section.

In one embodiment, the obtaining of the umbilical cord from a human or animal birth is not part of the present invention. In that embodiment, the method of the invention starts after the umbilical cord has been obtained. In other words, the method of the invention is then practiced using an umbilical cord as starting material that has been obtained prior to step (a).

Segmenting of the umbilical cord

Further, the inventors have found that it is advantageous to mechanically dissect the umbilical cord, as described herein. Dissection preferably comprises segmenting and sectioning, bot as described herein.

In a first step, it is advantageous to segment the umbilical cord, as described herein. The segmenting is optional but preferable. In particular, the segmenting renders the subsequent steps easier to handle and to reproduce reliably. In addition, the segmenting improves the isolation efficiency for stromal Wharton's Jelly in the subsequent step of sectioning. Thus, the segmenting preferably occurs prior to the below-described step of sectioning the umbilical cord (segments).

The segmenting according to the present invention is typically achieved by physical means, such as scissors or a scalpel. By segmenting the cord, cord segments are obtained (for illustration see e.g. Example 1). The term segments is used herein to refer to such cord cuttings. Preferably the segmenting is perpendicular to the cord. Perpendicular as used herein, with respect to the umbilical cord, means that the direction of the cut of the umbilical cord has an angle of about 90 ° with respect to the direction of the cord. For determination of the angle, the cord is preferably placed in a stretched manner, i.e. without curls or bends. About 90 ° means 75 ° to 105 °, preferably 80 ° to 100 °, more preferably 85 ° to 95 °. A skilled artisan will be capable to cut the cord perpendicular also in the absence of a device for measuring the angle, based on his experience. Nevertheless, a device can be used if desired, for determining the angle.

More preferably, the perpendicular segments preferably each have a length of about 1 to about 4 cm, preferably about 2 to about 3 cm, and more preferably about 2.5 cm. In a preferred embodiment, the segment(s) of vessels are removed from the segments(s) of the umbilical cord prior to step (b). In particular, it is preferred to remove the vessels, or more precisely segments of vessels, after the - preferably perpendicular - segmenting of the umbilical cord.

The segmenting is optional but advantageous and thus, it is preferably part of the method of the invention. The segmenting is preferably conducted under sterile conditions.

Sectioning in order to obtain stromal Wharton's Jelly

In general, mammalian umbilical cord, and in particular human umbilical cord, shows a tissue compartmentalization in which cellular characteristics and extracellular matrix elements differ from each other. At least six distinct zones or sections have been described, based on the structural and functional studies (see also Fig. 1): here listed in the order from outside to inside, whereas numbers refer to the numbers in Fig. 1: cord lining (comprising amniotic epithelium and sub-amniotic cells) (1), (intervascular) stroma (named classically as stromal Wharton's Jelly), intervascular stroma (4), perivascular stroma (5), vessels (2 and 3), subamniotic stroma (1b), clefts (not illustrated).

In the state of the art it had been considered that, given the hypothesis that perivascular cells typically migrate away from the proximity to the vasculature, the majority of MSC in Wharton's Jelly originate from the perivascular region (Davies et al, Stem Cells Translational Medicine, 2017, vol., 6, p. 1620-1630).

The inventors have, however, arrived at the surprising finding that a sectional approach is advantageous when aiming to extract MSC from UC, and that the section harboring MSCs with superior properties is the stromal Wharton's Jelly. The inventors surprisingly found that cells derived from the stromal Wharton's Jelly or a fraction thereof have superior properties. Therefore, it is an essential step of the method of the present invention that the stromal Wharton's Jelly or a fraction thereof is isolated.

Prior to isolation of the stromal Wharton's Jelly it is important to rinse the cord, or particularly the cord segments. Suitable for rinsing is an aqueous solution, ideally buffered at or near physiological pH and isotonic, optionally supplemented with antibiotics. For example, HEPES-Buffered Saline Solution (HBSS) or phosphate buffered saline (PBS), preferably HBSS supplemented with 300 μg/ml gentamycin and 0.15 μg/ml amphotericin B is suitable. Typical rinsing time may be preferably 1 to 30 minutes, preferably approximately 10 minutes. During that time, mechanical movement, e.g. shaking, is employed in order to properly expose the umbilical cord, or preferably umbilical cord segments, to the aqueous rinsing solution. Particularly after the cord has been segmented, the rinsing is important because thereby blood leftovers at the segment interfaces resulting from segmenting can be removed.

Then, for the isolation of the stromal Wharton's Jelly, preferably, the umbilical cord, or much preferred the umbilical cord segment(s), is/are subjected to sectioning, as described herein. Thereby, the desired stromal Wharton's Jelly is obtained. Other ways of obtaining stromal Wharton's Jelly are suitable as an alternative to the sectioning described herein, and are also part of the description herein. Preferably, however, the stromal Wharton's Jelly is obtained by the sectioning approach described herein.

For the purpose of isolating the desired Wharton's Jelly, the lateral opening of the umbilical cord, or of segment(s) of the umbilical cord, has proven advantageous. Preferably, the umbilical cord, or of the segment(s) of the umbilical cord is/are incised lengthwise, e.g. with a scalpel. Lengthwise means in parallel to the extension of the cord. One lengthwise incision per segment is preferred over multiple incisions. The lateral opening, or more specifically lengthwise incision, enables that the various sections of the umbilical cord can be subsequently efficiently separated, in particular the stromal Wharton's Jelly can be isolated, as described in the following.

As described in detail herein and illustrated in the examples herein, cells from the stromal Wharton's Jelly proved to be highly advantageous. Thus, the Wharton's Jelly to be sectioned and selected for downstream processing in the present invention excludes the perivascular zone, and thus excludes any perivascular Wharton's Jelly. In other words, the Wharton's Jelly to be sectioned and selected for downstream processing is the stromal Wharton's Jelly. The finding that the cells from stromal Wharton's Jelly isolated according to the present invention are superior is a surprising finding of the present inventors. It had been established previously by fine structural, immunohistochemical (e.g. Akerman et al., Gynecol. Endocrinol., 2002, vol. 16, p. 299-306 and Karahuseyinoglu et al., Stem Cells, 2007, vol. 25, p. 319-331) and *in vitro* functional studies (Sarugaser et al., 2005, Stem Cells, vol. 23, p. 220-229; Karahuseyinoglu et al., Stem Cells, 2007, vol. 25, p. 319-331) that there are differences in the number and nature of cells among subamniotic (1b), intervascular (4), and perivascular (5) areas, and that the density of cells in a given space differs among subamniotic, intervascular, and perivascular areas: cell density is considerably low in subamniotic regions, whereas perivascular areas possess the highest cell density (Takechi et al., 1993, Placenta; vol. 14, p. 235-245; Karahuseyinoglu et al., 2007, Stem Cells, vol. 25, p. 319 -331). Note that in this paragraph the term "cell density" is used unconventionally with respect to a three-dimensional space, whereas the term is used with respect to a two-dimensional area in the rest of this disclosure. For example, in earlier studies, samples adherent to the serosa and adherent to the vessels had been proposed a source for umbilical-cord derived stromal cells (e.g. US 2004/0136967 A1). Although it was previously known that cells can be isolated from Wharton's Jelly and that different cell populations exist in the Wharton's Jelly, the specific selection of the stromal Wharton's Jelly according to the present invention is a more specific selection than early published approaches (e.g. US 2004/0136967 A1), and a different selection than the one suggested by others (e.g. WO 2004/072273 A). Thus, the sectioning prior to the physical removal is markedly different from suggestions in the literature according to which the tissue is not normally as critically selected and sectioned prior to isolation of cells, but wherein desired cells are isolated from a cell population, such as by selective destruction of unwanted cells (negative selection, see e.g. US 2004/0136967 A1). To the knowledge of the inventors of the present invention, it was not specifically known that cells originating from the umbilical cord's stromal Wharton's Jelly are superior to perivascular cells regarding homogeneity, size (volume and surface area, respectively), differentiation potential and stemness. Thus, in contrast to the main thinking according to the state of the art, the present inventors have found that the stromal Wharton's Jelly-derived cells are superior in many aspects. Several of these aspects are illustrated in the experimental examples herein. In general, cell populations with homogenous morphological and functional properties can be envisaged to be suitable for developing a relatively uniform cell-based product. Without wishing to be bound to a particular theory, it is possible that the uniformity can be ascribed to the fact that the stromal Wharton's Jelly typically comprises less pericytes and endothelial cells than the perivascular area, if any at all.

The inventors have surprisingly found that cells originating from the stromal Wharton's Jelly of the umbilical cord are particularly advantageous. The subamniotic stroma (typically comprising Wharton's Jelly) (1b) may be obtained by scraping the inner surface of the amniotic epithelium (1a) with a mechanical means; however, care has to be employed to avoid rupture or fractioning of the amniotic epithelium, which could result in undesired obtaining of a part of the amniotic epithelium.

Advantages include homogeneity of the cell populations and stem cell like properties. In particular, in the absence of umbilical cord vessels and the perivascular zone, and thus absence of cells from the umbilical cord vessels and the perivascular zone, these advantages can be achieved.

Therefore, it is preferable that the step (a) isolating the stromal Wharton's Jelly or a fraction thereof from the umbilical cord comprises the sectioning of the umbilical cord or of segments thereof. Preferably, it comprises the sectioning of segments of the umbilical cord. During the step of sectioning, the desired stromal Wharton's Jelly is separated from the vessels with the perivascular zone, which may comprise the perivascular Wharton's Jelly. During the step of sectioning, the stromal Wharton's Jelly is also separated from at least the amniotic epithelium. Care has to be employed to avoid rupture or fractioning of the amniotic epithelium, which could result in undesired obtaining of a part of the amniotic epithelium.

The sectioning is preferably conducted at sterile conditions.

The sectioning is an essential part of the step (a) of the method according to the present invention, i.e. of step of the isolating the stromal Wharton's Jelly or a fraction thereof from an umbilical cord. Thus, in one embodiment, isolating the stromal Wharton's Jelly or a fraction thereof from the umbilical cord is achieved by subjecting the umbilical cord to physical separation, wherein vessels and the perivascular area of the umbilical cord and at least the amniotic layer of the cord lining are discarded, and the stromal Wharton's Jelly or a fraction thereof is retained.

For the sectioning, or more precisely sectioning by mechanical removal, it has proven to be greatly advantageous that the umbilical cord has been previously segmented as described above, and additionally incised lengthwise, as described above. Thus, in a preferred embodiment of the present invention, step (a) of the method comprises both a segmenting step, as described above, and a sectioning step, as described here.

A suitable way of isolating the stromal Wharton's Jelly or a fraction thereof from an umbilical cord comprises the removal of blood vessels and of the perivascular area from the umbilical cord. Suitably, the perivascular zone, which has been proposed in the literature to comprise perivascular Wharton's Jelly, is also separated from the cord lining, particularly at least the amniotic layer of the cord lining.

Thus, the sectioning according to the present invention is characterized by the physical removal of the vessels and the perivascular area from the umbilical cord. According to the present invention, the vessels and the perivascular area may be removed before step (a) or as part of step (a), which is preferred. One technically advantageous way of removing the vessels and the perivascular area, which is preferred in the present invention, is characterized by stripping out the vessels and the perivascular area from the umbilical cord. For example, the blood vessels of the umbilical cord (two veins and an artery) are stripped out with sterile forceps.

The inventors have observed that the stripping out of the vessels, e.g. by pulling the vessels out, also removes the perivascular area. In a preferred embodiment, the perivascular area is removed in its entirety; this means that no cells belonging to the perivascular area remain in the cord (segments) once the vessels have been removed. Any other method is equally suitable, as long as the perivascular area is completely removed. The zone proximal to the external wall of the umbilical vasculature is the perivascular zone. The perivascular zone lies typically within a zone extending to about 3 mm or less from the external wall of the vessels. The perivascular zone is removed together with the vessels when the vessels are stripped out. Any Wharton's Jelly that possibly lies within the perivascular area proximal to the external wall of the umbilical vasculature can be referred to as perivascular Wharton's Jelly. The perivascular Wharton's Jelly lies typically within a zone extending to about 3 mm or less from the external wall of the vessels. Any perivascular Wharton's Jelly is removed together with the vessels when the vessels are stripped out.

Thus, in a preferred embodiment, the vessels are stripped out of the umbilical cord, or of the segment(s) of the umbilical cord. Stripping out can be achieved by grabbing the vessel(s) by a mechanical means, such as by tweezers or any other suitable means for that purpose, and by pulling the vessel(s) out. Preferably, the vessels are pulled out one after another, but this is not a strict requirement. While the vessels are stripped out, the umbilical cord or segment thereof is usually fixed, e.g. by holding onto it with a mechanical means, such as by tweezers or any other suitable means for that purpose. Thereby, mechanical force can be applied and the vessels can be removed. The removal should be carried out with care, in the sense that opening of the vessels and/or loss of vascular or perivascular cells should be avoided. The removal should be carried out with care, in the sense that pulling out the cord matrix should be avoided, or alternatively, kept to a minimum.

Preferably, after the step or removing the vessels, the umbilical cord, or the respective segment(s), is/are optically re-examined to ensure that the vessels (and thus also the PVWJ) have been completely removed.

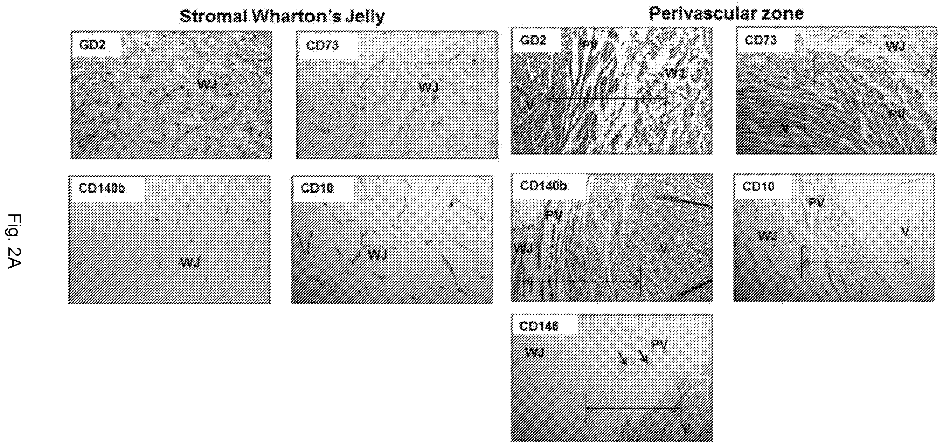

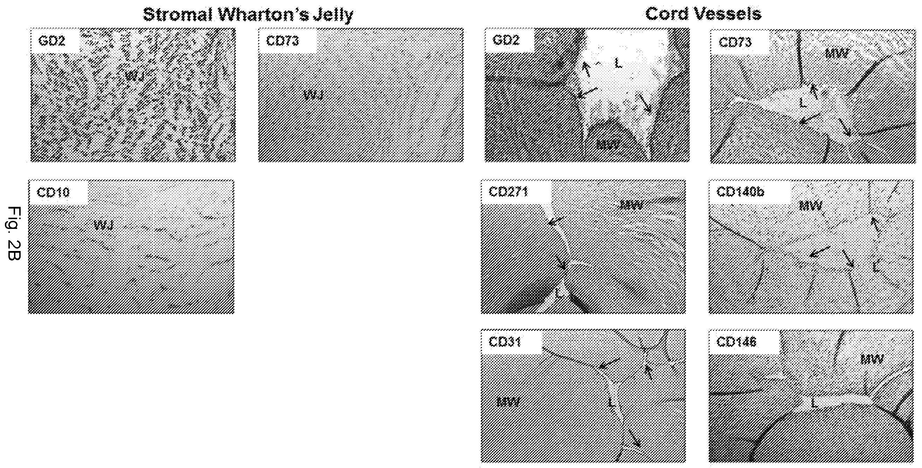

The vessels, including any tissue that adheres to them after being stripped out, are discarded. That means that vessels, including any tissue that adheres to them after being stripped out, are not subjected to the further downstream step of enzymatic treatment. According to the method of the present invention, the perivascular zone Wharton's Jelly is not used as a source for isolation of cells. Thus, no perivascular Wharton's Jelly is used as a source for isolation of cells. For illustrative purposes, in Fig. 1 and 2, the perivascular Wharton's Jelly is illustrated (5 in Fig. 1).

According to the present invention the vessels and the area around the vessels is discarded. The area around the vessels is at least about 2.0 mm in diameter, whereby measurement extends from the outer surface of the vessel outwards (see Fig. 2), such as 2.0 mm in diameter, preferably, 2.5 mm, and more preferably 3.0 mm. Thus, at least about 2.0 mm, such as 2.0 mm, preferably, 2.5 mm, and more preferably 3.0 mm around the vessels of the umbilical cord are not used as starting material for isolation of cells according to the present invention. A preferred way of conveniently achieving this is the physical removal ("strapping out") of the vessels from the umbilical cord, or more precisely, of the vessel segments from umbilical cord segments, as described herein. Thus, in a preferred embodiment, the vessels are removed physically from the umbilical cord, or vessel segments are removed physically from segments of the umbilical cord. The latter option is preferred; this requires segmenting of the umbilical cord as described herein prior to removal of the vessels from the segments.

Now that the vessels and the perivascular area have been removed, the stromal Wharton's Jelly is obtained from the vessel-free umbilical cord, or vessel-free umbilical cord sections, as follows: Preferably, isolating the stromal Wharton's Jelly or a fraction thereof is additionally characterized by a step of physically separating the subamniotic stroma (1b) from the umbilical cord lining membrane (1a). This can include separating the Wharton's Jelly from the umbilical cord lining membrane, particularly at least from the epithelial layer of the umbilical cord lining membrane. Thereby, stroma (comprising stromal Wharton's Jelly) free of the respective cord lining material is obtained. Preferably, the step of physically separating stroma (possibly including Wharton's Jelly) from the umbilical cord lining membrane, particularly at least from the epithelial layer of the umbilical cord lining membrane, is also done on the segments which are obtainable as described above. For practical reasons, the step is normally performed on a segment-by-segment basis (i.e. one segment after the other), although rapid conduction of this step is preferred.

The stromal Wharton's Jelly typically has a relatively loose jelly-like consistency; therefore it can be mechanically dissected out of the vessel-free umbilical cord, or preferably vessel-free umbilical cord segments.

The mechanical dissection is preferably characterized by scraping out. For scraping out, any tool, preferably mechanical tool suitable or designed for such purpose may be used. The removal is preferably by a mechanical means; suitable mechanical means are disclosed in the art, e.g. in US20080181967 A1, WO2008146992 A1, and WO2007080591 A2. In one embodiment, a mechanical means is used to snip out pieces of matrix tissue from the UC segments. In one embodiment, the inside surface of the cord (segments) is scraped out with a mechanical means. In one embodiment, the amniotic epithelial membrane is removed mechanically, e.g. with a mechanical means. If the scraping is done carefully, which is preferred, the epithelial layer is not damaged and thus, no cells of the epithelial layer are obtained by scraping.

It is important not to include pieces of the amniotic epithelial membrane when collecting the matrix tissue. To achieve this, cutting of the cord lining should be avoided, or kept at a minimum. In particular, physically separating stromal Wharton's Jelly from the umbilical cord lining membrane is preferably characterized by scraping out the Wharton's Jelly, preferably without mechanically dissecting the umbilical cord lining, or with dissecting the cord lining only to a minimal extent. "mechanically dissecting" includes cutting, squeezing or otherwise affecting the tissue integrity by application of mechanical force. In a preferred embodiment, "a minimal extent" consists of one single lengthwise incision, preferably parallel to the direction of the cord. Thereby, the cord, or segment of the cord, as the case may be, is laterally opened, thereby providing access to the inside of the cord.

Optionally, also no cells of the sub-amniotic layer are obtained. To achieve this, the scraping has to be performed with great care, without exercising undue mechanical force near the umbilical cord lining membrane. In this embodiment, the entire cord lining (comprising amniotic layer and sub-amniotic mesenchymal layer) is not desired.

At any rate, the amniotic (epithelial) layer is not desired. Any undesired cord lining material remaining after the mechanical removal of the desired stromal Wharton's Jelly is discarded.

Thereby, stromal Wharton's Jelly tissue is obtained.

Based on the advantageous properties of the cells isolated according to the present invention, the inventors conclude, without however wishing to be bound to any particular theory, that less mature cells with a beneficial ability to proliferate are located relatively further away from the vessels and thus closer to the amniotic surface, whereas more differentiated cells are found in closer proximity to the umbilical vessels. In conclusion, the inventors have selected the stromal Wharton's Jelly as region of origin for the isolation of cells according to the present invention.

In a preferred embodiment, the step (a) of isolating the stromal Wharton's Jelly or a fraction thereof from an umbilical cord comprises the step of sectioning as described herein and the step of mincing as described below.

In a more preferred embodiment, the step (a) of isolating the stromal Wharton's Jelly or a fraction thereof from an umbilical cord comprises the step of segmenting as described above, the step of sectioning as described herein and also the step of mincing as described below.

Decreasing the size of the tissue fragments by mechanical treatment

Preferably, the stromal Wharton's Jelly obtained by the sectioning is subsequently subjected to mechanical treatment which aims at decreasing the size of the tissue fragments. The mechanical treatment aims at dissecting physical bonds within the stromal Wharton's Jelly, so that the size of the pieces of stromal Wharton's Jelly is reduced. The mechanical treatment is advantageous with respect to the subsequent enzymatic treatment, as described below.

A relatively small and relatively uniform size of the fragments of stromal Wharton's Jelly is associated with several advantages: it is associated with an increased surface area and thus is understood to render the material accessible to the enzyme(s) used for enzymatic treatment, and it allows the platelet lysate, which is understood to contain growth factors and other elements suitable for MSCs, to be in close proximity to substantially the entire isolated Wharton's Jelly.

Preferably, the mechanical treatment is conducted by mincing the fragments finely with sharp scissors and/or scalpel, but any other means suitable for mechanical decrease of the fragments is suitable, as long as the cells are not substantially mechanically damaged or destroyed. For example, respective instruments are available from Miltenyi Biotec (Bergisch Gladbach, Germany). At any rate, the mechanical treatment is preferably conducted at sterile conditions.

The mechanically treated (e.g. minced) tissue is optionally added to an aqueous solution and dispersed therein, to prevent said tissue from drying out. is an aqueous solution, ideally buffered at or near physiological pH and isotonic, optionally supplemented with antibiotics. For example, HEPES-Buffered Saline Solution (HBSS) or phosphate buffered saline (PBS), preferably HBSS, is suitable. Optionally, the aqueous solution comprises antibiotics at suitable concentrations.

Preferably the mechanically treated (e.g. minced) tissue is subsequently received (pooled) in a suitable sterile dish, such as a Petri dish. After tissue isolation, and before enzymatic digestion, which will be described below, the total minced tissue weight that is obtainable in a full scale run is normally 14 grams or more, such as 16 grams or more or 18 grams or more.

Enzymatic treatment

According to the present invention, cells are released from the umbilical cord or segments thereof or sections thereof or minced tissue obtained therefrom by enzymatic treatment. In contrast to earlier teachings (e.g. Conconi et al., 2011, Open Tissue Engineer. Regen. Med. J., vol. 4, p. 6-20), the present inventors found that that enzymatic treatment, particular when carried out under the conditions as described as preferable herein, i.e. mild conditions, yields advantageous MSCs not characterized by cell damage.

The minced tissue embodiment is preferred. Thus, preferably, cells are released by enzymatic treatment from minced tissue obtained from the umbilical cord or segments thereof or sections thereof.

In particular, in step (b) of the method of the invention, the stromal Wharton's Jelly or fraction thereof is subjected to enzymatic treatment, thereby releasing at least one cell from the stromal Wharton's Jelly or fraction thereof. Typically, a multitude of cells is released. The stromal Wharton's Jelly or a fraction thereof is subjected to enzymatic digestion. In particular, the cells are released from the stromal Wharton's Jelly, which is a specific section of the umbilical cord, and which is obtainable during the preceding step (a) as described above. The step of the method according to the present invention which comprises the enzymatic treatment can be referred to as "enzymatic treatment", "enzyme digestion", or the like, or simply as "step (b)".

The enzymatic treatment can occur optionally in parallel and/or subsequent to the physical isolation of the stromal Wharton's Jelly from the umbilical cord; however, subsequent is much preferred. More precisely, it is preferred that step (b) of the method of the invention is subsequent to step (a) of the method of the invention. In that embodiment, step (b) is the only step in the method according to the present invention in which enzymatic activity is added.

The approach of the present invention, which features release of the cells from the umbilical cord tissue, specifically, the stromal Wharton's Jelly, is markedly different from prior art approaches wherein umbilical cord tissue is cultured as such, and cells grow out of the tissue, e.g. as a result of migration therefrom or cell division or both (US 2004/0136967 A1). According to the present invention, however, the at least one cells, or preferably the majority of the cells contained in the stromal Wharton's Jelly, is/are released by enzymatic treatment rather than by migration therefrom or cell division. This provides a significant temporal improvement for the isolation: instead of several days (e.g. 2004/0136967 A1), the cells can be isolated from umbilical cord in a few hours, as described in detail below.

The enzymatic treatment is a treatment wherein the cells are released from the umbilical cord or segments thereof or sections thereof by treatment with at least one enzyme. A suitable enzyme is a protease. Thus, the cells are preferably released from the umbilical cord or segments thereof by treatment with at least one protease. A protease (also called a peptidase or proteinase) is any enzyme that performs proteolysis; protein catabolism by hydrolysis of peptide bonds. Preferably, the protease is capable of digesting at least one extracellular matrix protein, preferably collagen. Most preferably, the enzymatic treatment of step (b) is a treatment comprising exposure to the enzyme collagenase. In other words, the at least one protease is preferably a collagenase. Other proteases are optionally additionally present. In one embodiment, essentially no other protease than collagenase is present. "Essentially no other protease" means that the collagenase preparation that is added has been purified to the maximum degree reasonably possible and/or that activity of other proteases in the collagenase preparation cannot be detected by normal laboratory methods.

A particularly suitable and thus preferred enzyme for extraction according to the present invention is collagenase (EC 3.4.24.7). The term "collagenase" refers to an enzymatic activity which is capable of breaking (more specifically hydrolyzing) the peptide bonds in the extracellular matrix protein collagen. Collagen is present in the extracellular matrix of various tissues, including the umbilical cord. Collagenase assists in destroying extracellular structures. Collagenase is available from both eukaryotic and prokaryotic origin, although protease from prokaryotic origin, particularly from bacteria such as *Clostridium* sp., is preferred herein. The collagenase can be isolated from its natural source, or produce recombinantly, e.g. in E. *coli.* In some embodiments, collagenase from its natural source, e.g. *Clostridium* sp., is preferred. For example, the collagenase may be from *Clostridium histolyticum.*

Unless the context dictates otherwise, "collagenase" refers herein to the enzymatic activity, whereas "collagenase preparation" refers to a product, commercial or not, which comprises collagenase activity. The collagenase activity is provided by collagenase enzyme - pure or not - present in the collagenase preparation.

Collagenase preparations are commercially available. In some embodiments, the collagenase preparation used is sterile or GMP grade. Preferably, the collagenase preparation is collagenase preparation NB4 or collagenase preparation NB5 or collagenase preparation NB6, all from SERVA (Heidelberg, Germany) or any other collagenase preparation equivalent to any of these. Equivalence can be determined by running the method described in Example 2A, in parallel with the NB4/NB6 collagenase preparation and with the collagenase preparation for which equivalence is to be determined, optionally in a titration experiment to determine equivalent collagen digestion activity.

Optionally, the collagenase preparation used does not comprise any enzymatic activity other than collagenase activity. In particular, it is preferred that no hyaluronidase activity is present. In other embodiments, hyaluronidase activity is present. It had previously been reported that the combination of collagenase with hyaluronidase can be advantageous (e.g. Bailey et al., Tissue Eng., 2007, vol. 13, p. 2003-2010). In the experimental examples of the present invention, however, satisfying results were obtained with no hyaluronidase added. Therefore, it is preferred in the present invention that no added hyaluronidase activity is present during the enzymatic digestion.

In some embodiments no protease other than collagenase activity is present. In other embodiments, the activity of one or more non-collagenase proteases is present. Other proteases may be specific proteases or unspecific proteases, endo- or exo-peptidases. Thus, optionally other enzymatic activities, such as other proteases in particular, may be used in addition to collagenase. Such other enzymatic activities may either be present in the collagenase preparation or added separately. For example, clostripain (EC 3.4.22.8, also termed clostridiopeptidase B, clostridium histolyticum proteinase B, alpha-clostridipain, clostridiopeptidase, Endoproteinase Arg-C), a proteinase that cleaves proteins on the carboxyl peptide bond of arginine, or an equivalent, may be present. In another not mutually exclusive example caseinase c (EC 3.4.24) may be present. In another not mutually exclusive embodiment trypsin (EC 3.4.21.4), a serine protease, may be present.

Optionally, the collagenase preparation used does not comprise any enzymatic activity other than proteolytic activity, i.e. collagenase activity and non-collagenase proteolytic activity. In one embodiment, however, the enzymatic treatment is performed in the additional presence of hyaluronidase, but hyaluronidase; this is merely optional.

Suitably, an aqueous solution comprising water and the enzyme for digestion, i.e. protease, more specifically the collagenase, and optionally but preferably a buffering agent, is prepared. The buffering agent ensures that the pH lies in the desired pH range. Any buffering agent may be used that is suitable for buffering the pH in the desired range.

The aqueous solution which comprises the enzyme, and the buffering agent, and water, and optionally further ingredients, is termed Digestion Buffer. In some embodiments, the Digestion Buffer does not comprise a metal-chelating agent such as EDTA or EGTA.

Preferably, the Digestion Buffer has a pH of 6.0 to 9.0, more preferably 7.0 to 8.0. Not only is this close to physiological pH, but suitable enzymes have also been described to have a pH optimum in the range from pH 7.0 to pH 8.0. Thus, the digestion is preferably performed in that pH range. pH is preferably to be determined at the temperature at which digestion is to occur.

Optionally, calcium ions are present in the Digestion Buffer; for example, a watersoluble calcium salt, such as calcium chloride, may be added during preparation of the Digestion Buffer. For example when the enzymatic treatment is a collagenase treatment, the presence of calcium ions is beneficial, and thus preferred.

Optionally, heparin is present in the Digestion Buffer. It is known that heparin is a suitable additive for platelet lysate-containing liquid compositions to avoid clogging (e.g. Lohmann et al., 2012, PLoS ONE, vol. 7e37839). Suitable concentrations of heparin in the Digestion Buffer according to the present invention are 0.1 to 100 U/mL, such as 1 to 10 U/mL, e.g. 2 U/mL heparin. The mL refer to the total volume of the Digestion Buffer. One unit of heparin (the "Howell unit") is an amount approximately equivalent to 0.002 mg of pure heparin, which is the quantity required to keep 1 ml of cat's blood fluid for 24 hours at 0 °C ("Online Medical Dictionary", 2000, Centre for Cancer Education).

To start the digestion, the Digestion Buffer is added to the tissue to be digested - or vice versa. It is important that the enzyme-containing aqueous solution, i.e. Digestion Buffer, and the tissue become mixed. Preferably, the ensemble of Digestion Buffer and tissue is immediately gently agitated to ensure suitable mixing. By the addition, the enzymatic treatment is started.

Preferably, the previously isolated stromal Wharton's Jelly derived tissue and the Digestion Buffer are mixed at a defined ratio. This is associated *inter alia* with the advantages of reproducibility of the method and digestion at mild conditions, if desired.

$$\text{Ratio} = X \text{ mg tissue} / 1 \text{ ml Digestion Buffer}$$

As the minced tissue, immediately prior to being mixed with the Digestion Buffer, is optionally stored in a saline-buffered solution (preferably HEPES-buffered saline solution, HBSS), to prevent the tissue from drying out, the defined ratio, in that embodiment, is defined as follows:

$$\text{Ratio} = \mathbf{X} \text{ mg (tissue} + \text{saline buffered solution)} / 1 \text{ ml Digestion Buffer}$$

(The "saline buffered solution" is not to be confused with the "Digestion Buffer". The "saline buffered solution" will typically not contain any digestive enzyme, particularly no collagenase.)

In both formulae above, $\mathbf{X}$ is suitably in the range of 10 to 1.000, preferably 50 to 500, more preferably 80 to 200, and most preferably essentially 100. Thus, in a preferred embodiment, 100 mg of minced tissue/HBSS is mixed with 1 mL of Digestion Buffer.

Unless stated otherwise, the collagenase treatment is typically performed at a temperature between 35 and 39 °C, more preferably between 36 and 38 °C, and most preferably at around 37 °C. Thus, preferably, the enzymatic digestion is performed at a temperature of 35-39 °C, such as 38-38°C, preferably 37 °C. The tissue originating from the umbilical cord, as described herein, is subjected to treatment by collagenase for a determined time interval (duration). Suitable time intervals are described herein.

Preferably, the enzymatic digestion is performed under agitation. For example, it was found that constant gentle agitation at ca. 50 to 100 rpm, e.g. on an orbital shaker, is suitable for digestion.

In a much preferred embodiment, the protease digestion is conducted at mild conditions. The present inventors have surprisingly found that, in one embodiment a digestion time of 1 hour is suitable. More generally, contrary to the suggestion of some earlier studies, relatively mild conditions are suitable for digestion in the method according to the present invention. Among the earlier studies, WO 2004/072273 A and Sarugaser et al., 2005, Stem Cells, vol. 23, p. 220-229 may be mentioned for illustrative purposes: according to WO 2004/072273 A, a collagenase of unspecified origin and concentration is recommended to be used in a concentration of e.g. 0.1 mg/ml or 1 mg/ml; the recommended collagenase digestion time is 12 to 36 hours, such as about 24 hours; according to Sarugaser et al. (*supra*), 1 mg/ml of collagenase from Sigma, C-0130) is used; the collagenase digestion time is 18 to 24 hours. Thus, the mild conditions used in the context of the present invention are distinguishable from the conditions described by earlier studies.

"mild conditions" or synonymously "gentle conditions", as used herein, means that at least any two, but most preferably all three of the conditions (1) digestion time, (2) enzymatic activity and (3) platelet lysate are as described below. The enzymatic activity is of course related to the type of enzyme used, and the amount of enzyme used, relative to the volume in which it is used (enzyme concentration) or relative to the amount of tissue to be digested.

Unless the context dictates otherwise, all embodiments of (1) digestion time, (2) enzymatic activity and (3) platelet lysate are combinable with each other. Thus, in general, factors that contribute to mild conditions: short enzyme digestion time, low enzyme concentration and, presence of hPL.

In one embodiment, short enzyme digestion time, as described below, is combined with low enzymatic activity, as described below. Preferred embodiments of short enzyme digestion time, as described below, can also be combined with preferred embodiments of low enzymatic activity, as described below.

In one embodiment, short enzyme digestion time, as described below, is combined with the presence of platelet lysate, as described below. Preferred embodiments of short enzyme digestion time, as described below, can also be combined with preferred embodiments of platelet lysate, as described below.

In one embodiment, low enzymatic activity, as described below, is combined with the presence of platelet lysate, as described below. Preferred embodiments of low enzymatic activity, as described below, can also be combined with preferred embodiments of platelet lysate, as described below.

In a particularly preferred embodiment, short enzyme digestion time, as described below, is combined with low enzymatic activity, as described below, and also with the presence of platelet lysate, as described below. Preferred embodiments of short enzyme digestion time, as described below, of preferred embodiments of low enzymatic activity, as described below, and preferred embodiments of platelet lysate, as described below, can all be combined with each other. This embodiment is realized in Example 2A.

Variations of the exact values of (1) digestion time indicated herein, (2) enzymatic activity indicated herein and (3) platelet lysate indicated herein are also possible without departing from the present invention. For example, it may be possible to reduce the digestion time indicated herein, by e.g. 50 %, together with increasing the enzymatic activity indicated herein, by e.g. 50 %. The skilled person can envisage and practice such variations based on his general experience, and based on the general enzyme kinetic observation that a lower enzymatic activity may still suffice to digest a given amount of substrate when the digestion time is increased accordingly, and *vice versa.* For example, it may be possible to increase the digestion time indicated herein, by e.g. 50 %, together with reducing the enzymatic activity indicated herein, by e.g. 50 %. Respective varied conditions can still be referred to as mild conditions. Collagenase is not normally autocatalytic; therefore variations are possible without normally causing a reduction of the amount of collagenase during the digestion over time.

By applying mild conditions, it can be avoided to a very large extent that the MSCs which are to be isolated are severely damaged. The mild conditions enable to obtain much higher fractions of viable cells than previously available methods.

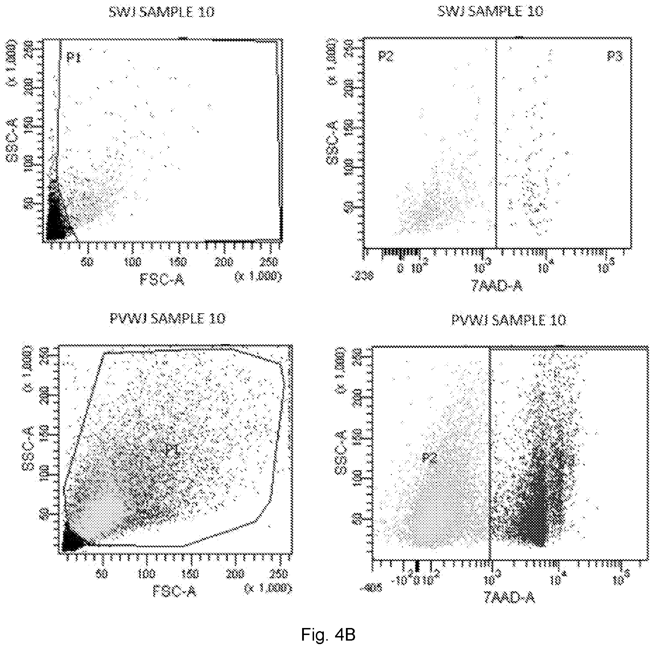

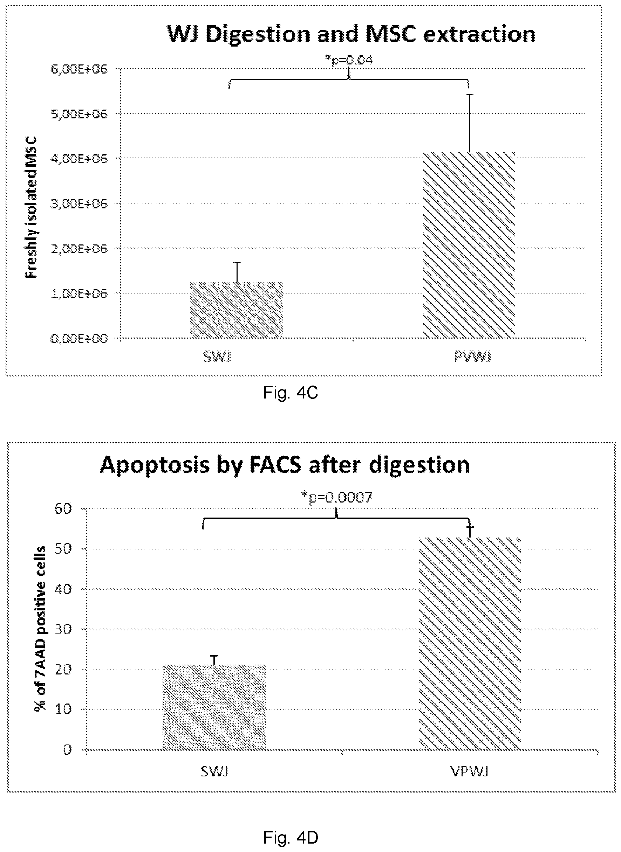

Although the method of the present invention, in typical embodiments, yields a lower number of total cells than a reference method (see Fig. 4 B and 4C), the cells isolated according to the method of the invention are in typical embodiments characterized by a low percentage of apoptotic cells (see Fig. 4 B and 4D). This is advantageous e.g. for the optional expansion, which will be described below.

(1) The present inventors have found that short enzymatic digestion times are advantageous in the method of the invention. In an example initially found by the present inventors, a short enzymatic digestion time is 1 hour. Based thereon, generally speaking, in a preferred embodiment, step (b) is conducted in a total time of less than 5 hours, more preferably less than 4 hours and most preferably less than 3 hours. Less than 3 hours includes time intervals of 0.5 to 2.5 hours and 1 to 2 hours, and any time interval with an upper limit of less than 3 hours, such as 2.5 hours, 2.6 hours, 2.7 hours, 2.8 hours, 2.9 hours and 2.95 hours. All such time intervals can be referred to as "rapid" or "short", although "rapid" and "short" are also a relative terms, e.g. 2 hours is of course more rapid/shorter than 4 hours. Rapid conduction of the enzymatic treatment avoids dehydration problems and other possible problems associated with cells being exposed to a non-natural environment for extended periods of time; a rapid conduction of the enzymatic treatment also overcomes difficulties described by Seshareddy et al. (2008, Meth. Cell Biol., vol. 86, p. 101-119).

It had been suggested that short enzyme treatment time intervals can be advantageous particularly in case of a co-digestion with hyaluronidase and collagenase (Can et al., Stem Cells, 25:2886-2895, 2007). Somewhat surprisingly, in the present invention, short treatment times are also beneficial in the absence of hyaluronidase. This is remarkable in two ways: first, in the absence of hyaluronidase no undesired effect of hyaluronidase, such as extensive undesired digestion of the extracellular environment end cell surfaces, would normally be expected to occur; second, without wishing to be bound to any particular theory, it is understood that collagenase alone acts on the extracellular matrix only, not on the desired cells themselves. Nevertheless the advantages of a mild treatment, including short duration of the treatment, on cell viability are quite remarkable. In one embodiment the enzyme digestion is an enzyme digestion under mild conditions without any added hyaluronidase. In one embodiment the enzyme digestion is an enzyme digestion under mild conditions in the absence of hyaluronidase. In a preferred embodiment, step (b) is conducted in a total time of less than 5 hours, more preferably less than 4 hours and most preferably less than 3 hours, and without any added hyaluronidase, or preferably in the absence of hyaluronidase.

Ideally, both steps (a) and (b) are handled rapidly. Thereby, cells can be obtained in little time from the umbilical cord. This represents an advantage over some earlier studies which relied either on extensive time intervals for enzymatic digestion of the umbilical cord tissue, or outgrowing of cells from umbilical cord tissue, or both. In a preferred embodiment, steps (a) and (b) together are conducted in a total time of less than 6 hours, more preferably less than 5 hours, more preferably less than 4 hours and most preferably less than 3 hours. Less than 3 hours includes time intervals of 0.5 to 2.5 hours and 1 to 2 hours, and any time interval with an upper limit of less than 3 hours, such as 2.5 hours, 2.6 hours, 2.7 hours, 2.8 hours, 2.9 hours and 2.95 hours. It is evident that a short duration of steps (a) and (b) together also means that the duration of step (b) itself is short. The present inventors have found that in one embodiment a digestion time of about 1 hour is advantageous.

(2) The inventors have also found that the amount of enzymatic activity matters. The enzymatic activity is of course related to the type of enzyme used, and the amount of enzyme used, relative to the volume in which it is used (enzyme concentration) or relative to the amount of tissue to be digested.

In particular, the inventors have shown that the amount of collagenase activity matters. For the purposes of describing the present invention, the collagenase activity is indicated in Wünsch units. 1 unit (U) according to Wünsch (Wünsch unit, or PZ unit) catalyzes the hydrolysis of 1 $\mu$mole 4-phenylazobenzyloxycarbonyl-L-prolyl-L-leucylglycyl-L-prolyl-D-arginine per minute at 25 °C, pH 7.1 (Wünsch et al., 1963, Hoppe-Seyler's Z. Physiol. Chem. 333, 149-51).

The process according to the present invention is typically characterized by a relatively low activity of collagenase during the enzymatic treatment step (b). Herein, the indicated amount of collagenase activity is indicated for the time point at which the collagenase is added. For example, in case the collagenase loses activity over time during step (b), no additional collagenase is added. However, collagenase is not normally autodigestive and thus relatively stable, and it can reasonably be assumed that, after being added, collagenase is present during the entire enzymatic treatment step (b).

The collagenase activity can suitably be indicated with reference to the total volume of the Digestion Buffer. In one embodiment, the Digestion Buffer comprises a specific collagenase activity of 0.01 Wünsch U/ml to 10 Wünsch U/ml, preferably 0.05 Wünsch U/ml to 5 Wünsch U/ml, preferably 0.1 Wünsch U/ml to 1 Wünsch U/ml, preferably 0.15 Wünsch U/ml to 0.5 Wünsch U/ml, preferably, e.g. ca. 0.1 Wünsch U/ml or ca. 0.2 Wünsch U/ml. In one embodiment the lower limit of collagenase activity is not particularly defined, as long as the collagenase activity added is sufficient to release mesenchymal stromal cells from the tissue within 5 hours or less. In this and other embodiments, the upper limit of collagenase activity can be defined as not more than 0.5 Wünsch U/ml, more preferably not more than 0.25 Wünsch U/ml, and specifically 0.18 Wünsch U/ml or 0.09 Wünsch U/ml.

In a preferred embodiment, the collagenase is present during the enzymatic treatment step (b) at a specific activity of not more than 0.5 Wünsch U/ml, more preferably not more than 0.25 Wünsch U/ml, and specifically 0.18 Wünsch U/ml with respect to the Digestion Buffer. Even more preferably, the activity of collagenase used in step (b) of the method according to the present invention is in the range of 0.18 to 0.09 Wünsch U/ml Digestion Buffer (see also Example 1). This activity differs from the activity of collagenase used according to WO 2004/072273 A and Sarugaser et al., 2005, Stem Cells, vol. 23, p. 220-229. Indeed, in one embodiment the collagenase activity during step (b) of the method of the present invention can be defined as being lower than the collagenase activity described by WO 2004/072273 A and Sarugaser et al. (*supra*).

Alternatively, the collagenase activity can suitably be indicated in relation to the weight of the tissue to be digested. (The weight of said tissue can be suitably determined by weighing the container in which it is contained prior and post filling with said tissue.) Said tissue, optionally comprised in a container, optionally comprises a saline-buffered solution (e.g. HBSS). In one embodiment, a suitable ratio lies in the range of 0.01 to 10 Wünsch U collagenase/100 mg tissue, preferably 0.05 to 5 Wünsch U collagenase/100 mg tissue, preferably 0.1 l to 1 Wünsch U collagenase/100 mg tissue, preferably 0.15 to 0.5 Wünsch U collagenase/100 mg tissue, preferably, e.g. ca. 0.1 Wünsch U collagenase/100 mg tissue or ca. 0.2 Wünsch U collagenase/100 mg tissue. In one embodiment the lower limit of collagenase activity is not particularly defined, as long as the collagenase activity added is sufficient to release mesenchymal stromal cells from the tissue within 5 hours or less. In this and other embodiments, the upper limit of collagenase activity can be defined as not more than 0.5 Wünsch U collagenase/100 mg tissue, more preferably not more than 0.25 Wünsch U collagenase/100 mg tissue, and specifically ca. 0.16 Wünsch U collagenase/100 mg tissue or ca. 0.08 Wünsch U collagenase/100 mg tissue, or any value within that preferred range. It is preferred in the present invention that the ratio of collagenase activity to total weight of the tissue is lower than the collagenase activity described by WO 2004/072273 A and Sarugaser et al., 2005, Stem Cells, vol. 23, p. 220-229.

(3) The inventors have further found that the presence of platelet lysate during the enzymatic digestion is advantageous. This is a surprising finding of the inventors. There has been some prejudice in the art that collagenase would not work well in the presence of platelet lysate. For example, it had previously been described that collagenolytic activity was inhibited by normal human plasma, due to inhibitors such as $\alpha_1$-antitrypsin (Chesney et al., J. Clin. Invest., 1974, vol. 53, p. 1647-1654). However, the inventors have found that collagenase digestion is efficient even in the presence of platelet lysate. In fact, the collagenase digestion is efficient even at the very low amounts of collagenase preferred in the present invention (see above). Thus, preferably, platelet lysate is present during the enzymatic treatment. Platelet lysate can be obtained from commercial sources or prepared freshly. Many hospitals, blood banks and research institutions have established protocols for preparing platelet lysate, and such lysates are generally suitable in the context of the invention. For example, it can be obtained by a freeze-thawing process. Such lysates can be made by freezing a platelet suspension and then thawing the material, though other freeze-thaw regimens are also suitable, provided they lead to cytolysis of the platelets. With the freeze-thaw technique, this method causes cells to swell and break, presumably because ice crystals form, followed by contraction at thawing. Thus, the cyclical swelling and contracting ultimately causes the platelets to break open. Multiple cycles may be preferentially used for more complete cytolysis, but the "more complete" cytolysis is not necessarily required. Varying degrees of platelet cytolysis can occur, e.g., at least 30%, at least 50%, at least 70%, at least 90%, or up to 100% cytolosis, by platelet count. Optionally, non-lysed material and other debris are removed from the platelet lysate prior to use.

Preferably, the platelet lysate comprises lysed mammalian platelets, and optionally mammalian plasma. In the second case, the platelet lysate is a composition comprising platelet lysate and plasma. Reasons for why plasma may be comprised include embodiments wherein platelets have not been completely separated from plasma prior to their lysis and/or wherein platelets have been (re-)suspended in plasma prior to their lysis. Preferably, the concentration of mammalian plasma in the composition is less than about 30 %, less than about 20 %, or less than about 10 % of the total volume. Preferably, the concentration of mammalian plasma in the platelet lysate is from about 0 % to about 10 %, such as from about 1 % to about 10 %. Optionally, the platelet lysate comprises concentrated platelets. Preferably, the mammalian platelet lysate is human platelet lysate. Preferably, the platelet lysate is substantially free of mammalian platelet membranes.

Platelet lysate can be generated from single or pooled donor-donated platelets isolated from whole blood or by apheresis. Preferably, the apheresis is based on separating substances having different densities, such as centrifugation, and/or, the apheresis involves absorption onto beads coated with an absorbent material and filtration.

Optionally, the platelet lysate used in the present invention is obtainable by lysing a platelet containing suspension, wherein the platelets are optionally suspended in plasma. Said platelet containing suspension preferably comprises between $1 \times 10^8$ platelets/ml and $5 \times 10^9$ platelets/ml, preferably between $3.8 \times 10^8$ platelets/ml and $1.2 \times 10^9$ platelets/ml, on average ca. $7.9 \times 10^8$ platelets/ml.

It is also envisaged that the presence of platelet lysate aids in contributing to the mildness of the enzymatic digestion. Surprisingly, the inventors found that the presence of platelet lysate has no negative effect on digestion of the cells (for illustration see Examples 2A and 3), although platelet lysate had been previously reported to negatively affect the activity of certain enzymes, including proteases. It is thus an important cornerstone of the method according to the present invention that the enzymatic treatment, more precisely the collagenase digestion, can be performed in the presence of platelet lysate.

Preferably, platelet lysate is present during the enzymatic digestion at a volume percentage of 0.01 % to 30 %, such as 0.05 % to 20 %, preferably 0.1 % to 10 %, more preferably 0.5 % to 5 %, more preferably 0.8 % to 2 %, and most preferably around 1 %. "volume percentage" means the total volume of platelet lysate present in the total volume of the Digestion Buffer.

The inventors have found that the presence of platelet lysate or derivatives thereof during the enzymatic digestion, particularly in the ratio indicated above, is advantageous.

The term "platelet lysate", when mentioned herein, comprises both platelet lysate directly obtainable by lysing platelets, as well as derivatives thereof, such as heatinactivated platelet lysate, sterile-filtered platelet lysate etc. In some cases, the use of derivatives may be required or recommendable, e.g. in view of regulatory considerations. In a more preferred embodiment, the platelet lysate is essentially free of contaminating agents. A platelet lysate essentially free of contaminating agents is defined herein as a sterile platelet preparation essentially free of infecting agents such as viruses (encapsulated and non-encapsulated), parasites, bacteria and endotoxins, i.e. a platelet lysate containing such infecting agents in a quantity that cannot be detected with culture-based, serological or molecular identification systems or with the LAL test (endotoxins). Pathogen-inactivated platelet lysate (PI-PL) is strongly preferred, see e.g. WO 2013/042095 A1 and Iudicone et al., 2014, J. Transl. Med., 12: 28.

There are several advantages associated with the presence of platelet lysate during the enzymatic treatment, which were not expected, and which will be described in the following.

First, the inventors found that the presence of human platelet lysate does not block the enzymatic activity of the enzyme collagenase, at least not to an extent that would negatively interfere with the desired enzymatic liberation of the cells from the stromal matrix.

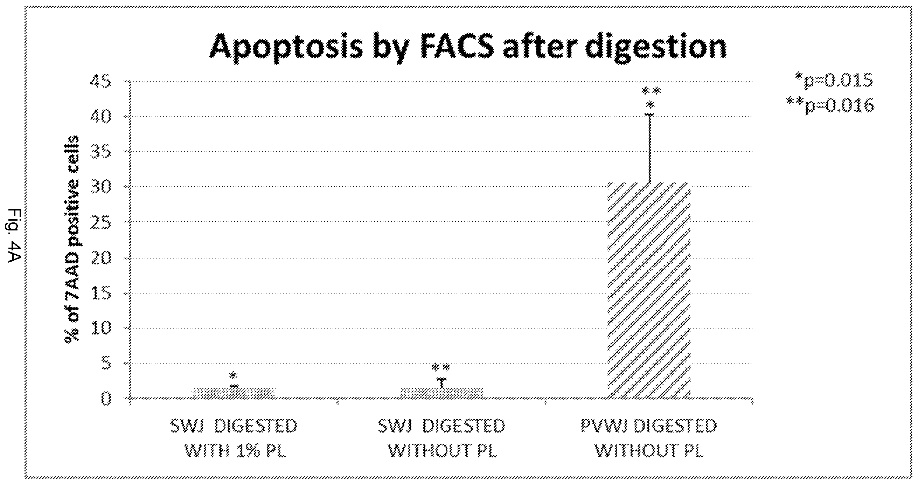

Second, the inventors found that the cells obtainable by digestion in the presence of platelet lysate or derivatives are characterized by excellent viability (see e.g. Fig. 4A).

Third, the inventors found that the cells obtainable by digestion in the presence of platelet lysate or derivatives in the method according to the present invention thereof have excellent proliferative properties. Thus, preferably, in step (b) the stromal Wharton's Jelly or fraction thereof is subjected to enzymatic treatment in the presence of mammalian platelet lysate (PL) or derivatives thereof, more preferably human platelet lysate (hPL) or derivatives thereof.

While platelets contain bioactive molecules and growth factors such as coagulation factors, adhesion molecules, and proteoglycans, basic fibroblast-derived growth factor (bFGF), epidermal growth factor (EGF), HGF, vascular endothelial growth factor (VEGF), insulin-like growth factor-1 (IGF-1), TGF-$\beta$1 and others, it is also known that they contain protease inhibitors (Astori et al., Stem Cell. Res. Ther., 2016, vol. 7, 93). In view of this teaching, the finding that platelet lysate can be suitably present during the enzymatic treatment is remarkable.

Overall, the specific conditions of extraction, which diverge from collagenase digestion protocols according to the state of the art, could be shown to be beneficial for cell viability and cell extraction and thus provide a surprisingly advantageous isolation process. In particular, it is possible to obtain a higher cell yield during the isolation process according to the present invention than by the process described by Seshareddy et al. (2008, Meth. Cell Biol., vol. 86, p. 101-119). More particularly, according to the present invention, it is possible to obtain a cell yield during isolation process that is 10-25 fold higher than the cell yield described by Seshareddy et al. (*supra*). Owing to the excellent viability of the cells isolated according to the present invention, the cells can be expanded efficiently *in vitro,* if desired.

Preferably, only one collagenase digestion step is performed in the method according to the present invention. This means that the tissue originating from the umbilical cord, as described herein, is subjected to treatment by collagenase for a determined time interval, and after termination of that time interval, cells that have become physically isolated or detached from the tissue are recovered, however, the remaining tissue is not subjected to one or more further rounds of collagenase digestion. Rather, it is preferably discarded after termination of the time interval and recovering of the cells.

At the end of the digestion interval, the digestion mixture may be diluted with a protein-containing solution, such as basal medium supplemented with platelet lysate. The moment of the addition of the protein-containing solution defines the end of the digestion time. The type of basal medium is not particularly limited, as long as the medium is suitable for cultivation of mammalian cells, preferably, however, mesenchymal stem cells and/or mesenchymal stromal cells. The protein-containing solution preferably comprises heparin.

A preferred basal medium is MSCBM CD (chemically defined basal medium for mesenchymal cells, Lonza), and a preferred supplement is 5 % (vol./vol.) human platelet lysate (hPL).

Addition of a protein-containing solution, such as human platelet lysate, is believed to inhibit the enzymatic activity. Without wishing to be bound to such theory, the addition also results in a dilution of the enzyme. Preferably, at least 1 volume of the protein-containing solution, with respect to the volume of the digestion mixture, is added. The protein-containing solution should of course be a solution that is not incompatible with cell culture purposes, i.e. illustratively it should be sterile, and optionally GMP grade, etc.

Preferably, the liquid comprising the tissue that had been subjected to digestion (post-digestion liquid) is subsequently subjected to separation. The term "separation", as used herein with reference to said post-digestion liquid, is not particularly limited and, generally means that cells may be separated from undigested tissue, incompletely digested tissue and other debris by a suitable method, including filtration and centrifugation. In one embodiment, said post-digestion liquid is filtered, to separate the released cells from undigested tissue, incompletely digested tissue and other debris, e.g. by filtration, e.g. filtration through a 120 $\mu$m mesh or filtration through gauze, as previously described (e.g. Del Bue at al., 2007, Veterin. Res. Comm, vol. 31, p. 289-292). For example, a filter pore size (mesh) of 50 to 150 $\mu$m, such as 70 to 100 $\mu$m, is suitable. Optionally, the filter material is gauze; multiple layers of gauze may be used. Thus, preferably, the liquid comprising the tissue that had been subjected to digestion is filtered through gauze. The filtrate is then termed "digested mixture". Another suitable method for separation includes low speed centrifugation; in that case the cells remain in the supernatant, whereas undigested tissue, incompletely digested tissue and other debris are pelleted; in that case the supernatant is referred to as "digested mixture". Optionally, filtration and low speed centrifugation are combinable, in any order. Alternatively, separation is by filtration only or by low speed centrifugation only, whereby filtration only is preferred.

Preferably, the digested mixture is subsequently centrifuged, preferably subsequent to filtration. The skilled person can adjust the centrifuge conditions to those suitable for pelleting mesenchymal stromal cells, without damaging their proliferative properties and viability. For example, the digested mixture can be centrifuged at 680 $\times$ g for 15 minutes using 250 ml centrifuge tubes. At the end of centrifugation, the supernatant is removed and the pellet, which contains the desired cells, is re-suspended. Ideally, in embodiments wherein the method according to the present invention comprises one or more steps of expansion, the cell pellet is re-suspended in the same growth medium as used at the (first) expansion step. Expansion according to the present invention will be described subsequently in detail.

Normally, the digested mixture comprises multiple cells, i.e. a cell population. Preferably, the cell population obtained by the enzymatic treatment as described herein is used directly as obtained, i.e. without any negative selection (selective destruction of unwanted cells). A suitable but optional use consists in expansion of the cells, which will be described below.

An illustrative example of the isolation procedure is described in Example 2A. As shown in Example 3, the use of platelet lysate does not negatively influence the collagenase activity, in releasing cells and in their viability. This is a surprising finding because platelet lysate had been previously reported to negatively affect the activity of certain enzymes, including proteases.

The enzymatic treatment is preferably conducted under sterile conditions.

Isolation of an individual cell, if desired, can be achieved e.g. by serial dilution. Thereby, an isolated individual cell is obtainable. Serial dilution is an optional step following step (b); however, obtaining a single isolated cell is not a main object of the invention; rather, it is preferred that step (b) is followed by step (c) for expansion of the cell(s), as described in the following.

Expansion

The cells obtained during the isolation, in particular step (a) and (b) of the method of the invention, can be expanded (i.e. subjected to expansion). The cells are expanded ex *vivo.* The ex *vivo* expansion allows the skilled person to select defined conditions for cultivation. These will be described in the following.

The expansion aims at multiplying the cells previously isolated, which have advantageous properties. As is common in the art, expansion occurs in a culture medium. Unless specified otherwise, the conditions suitable for expansion include conditions generally known to be suitable for culture of mammalian cells, more specifically for expansion of mammalian cells.

Expansion is a period of the cell cultivation/production process which is predominantly characterized by high cell growth and high mitotic activity. In the present invention, the expansion serves *inter alia* the purpose of increasing the number of cells, which means generating an increased number of cells, which are preferably mitotically active and more preferably in the exponential growth phase. By the expansion, larger cell populations can be obtained. The step of expanding the cells according to the present invention can be a single step of allowing the cells to proliferate under appropriate conditions: However, more preferably, the expansion according to the present invention comprises multiple passages, as will be described further below.

The expansion is generally conducted under sterile conditions, and typically also under conditions otherwise necessary or useful or recommended for cultivation of mammalian cells.

This embodiment of the invention is based on the finding that that a specific section of the Wharton's Jelly, i.e. the stromal Wharton's Jelly, comprises cells which can be isolated efficiently and reliably, and subsequently expanded efficiently and reliably. Thus, once the cells have been isolated, their population is expanded mitotically.

Thus, optionally and very generally, the method according to the present invention comprises one or more additional steps. In particular, in preferred embodiments of the present invention, a step for increasing the number of cells in comprised, taking advantage of the cell division potential of the cells obtained in step (b). In such preferred embodiments, step (b) is followed by a step (c); step (c) comprises the expanding of the at least one cell obtained in step (b). Step (c) is also referred to herein as "expansion step" or simply as "expansion".

The conditions for expansion are selected to be suitable for ex *vivo* growth and cell division of mesenchymal stromal cells. Thereby, a culture enriched for mesenchymal stromal cells is obtained. Without wishing to be bound to a particular theory, it is possible to envisage that either the culture selects for a specific subset of the isolated cells, thereby enriching them, or else the cells are changing their phenotype.

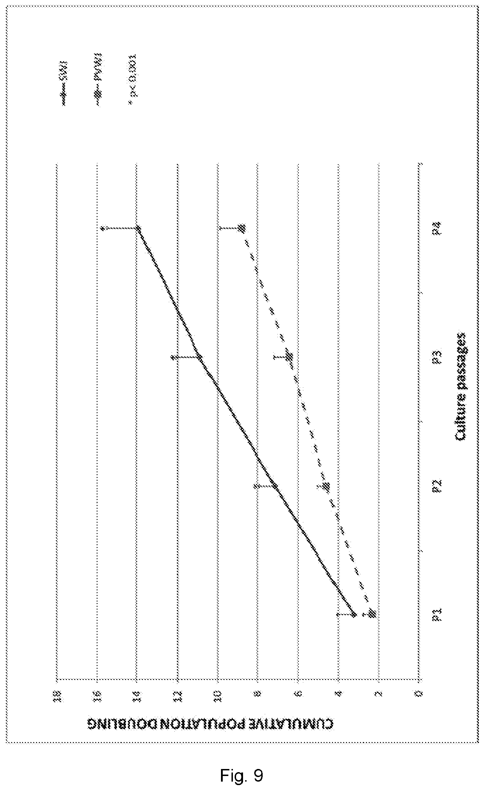

The expansion according to the present invention provides synergies with the isolation according to the invention, as follows: although the total number of cells isolated at the preferred mild conditions according to the present invention may be (and typically is) lower than the total number of cells extractable by the method suggested by US 2004 0136967 A1 and Seshareddy et al., Meth. Cell Biol., 86:101-119, 2008, the inventors have found that cells of cells isolated at the preferred mild conditions according to the present invention are particularly suitable for expansion, e.g. in view of their high percentage of viability (for illustration see Fig. 4B), and also in view of their high proliferative potential (for illustration see Fig. 9), and, thus, high cell numbers can be obtained by expanding the SWJ-derived cells isolated according to the present invention. In other words, the preferred isolation procedure according to the present invention together with the expansion according to the present invention bear the synergistic advantage that less starting material and/or fewer passages are required to arrive at a specific desired cell number because most of the cells that are subjected to the expansion are viable, and they proliferate particularly well.

Preferably the umbilical cord-derived cells obtained according to the present disclosure are the only cells subjected to the expansion step. In such embodiments, it is particularly preferred that no feeder cells are present during the expansion step.

Expansion typically occurs in a culture vessel. More precisely, cells destined to be subjected to expansion are seeded into a culture vessel. Typical such culture vessels are flasks, plates and stacks (whereby stacks are typically composed of a multitude of plates stacked on top of each other). Particularly suitable culture vessels in the context of the present inventions are culture vessels which allow for adherence of cells at the bottom of the culture vessel; such vessels are flasks, plates and stacks with a planar ground surface. Preferably, the culture vessel has at least a plastic ground surface. More preferably, the culture vessel is a plastic culture vessel. The culture vessel preferably has a flat ground surface ("flat-bottom").

The type of plastic is not particularly limited, as long as it is suitable for culturing of cells; however hydrophobic polymers such as particularly polystyrene, optionally surface modified, are preferred. Surface modification can be achieved by the plasma technique, which makes a variety of functional chemical groups available on the polystyrene surface, and which are suitable for adherence of cells in general. Such techniques are part of the state of the art, and surface-modified plastic vessels for culture of adherent cells are commercially available.

Preferably, however, the surface of the cell culture vessel is not protein coated prior to use. In particular, it is not coated with proteins such as cell matrix proteins like collagen and polylysine.

Preferably, expansion is done for multiple passages. The details of the passages are described below.

Growth medium

The growth medium for expansion according to the present invention will typically comprise a basal medium and one or more supplements. In a broad sense, the type of basal medium used is not particularly limited. Typical basal media are on water basis and are chemically defined. "chemically defined" means that the basal medium is composed of specific ingredients in specific amounts; the advantage of chemically defined basal media is that they can be prepared reproducibly, without variation due to experimental conditions, laboratory etc. The basal medium is normally sterile. Basal media for growth of mammalian cells are commercially available from different commercial suppliers.

Typically, basal media comprise ingredients comprising amino acids, salts (such as calcium chloride, potassium chloride, magnesium sulfate, sodium chloride, and monosodium phosphate), glucose, and vitamins (such as folic acid, nicotinamide, riboflavin, $B_{12}$), at least one buffering agent (such as e.g. HEPES), and typically also a coloring agent (e.g. phenol red); this is, however, not essential. Most of the commercially available basal media include phenol red as a pH indicator (coloring agent), which allows constant monitoring of the pH.

Preferably, the basal growth medium is one that does not require addition of an attachment matrix to plate cells.

Preferably, the basal medium is serum-free. Serum-free means that no human or animal serum is contained therein. More preferably, the basal medium is a chemically defined basal medium, which is serum-free

Preferably, the basal medium is chemically defined. A chemically defined basal medium is a basal medium suitable for the *in vitro* cell culture of human or animal cells in which all of the chemical components are known. In particular, chemically defined media require that all of the components must be identified and have their exact concentrations known. Therefore, a chemically defined medium must be entirely free of animal-derived components and cannot contain serum or serum-derived proteins, such as fetal bovine serum, bovine serum albumin or human serum albumin. A chemically defined basal medium does not comprise with only recombinant proteins and/or hormones To achieve this, chemically defined media do not comprise components from human or animal sources, such as proteins and/or growth factors; particularly, it does not comprise albumin from human or animal sources, nor growth factors from human or animal sources. Preferably it comprises recombinant albumin and/or recombinant growth factors, usually derived from non-human, non-animal sources such as plant cells and bacteria, and/or synthetic chemicals such as e.g. polyvinyl alcohol.

In some embodiments, a chemically defined basal medium comprises

(a) a conventional basal media (such as DMEM, F12, or RPMI 1640, comprising amino acids, vitamins, inorganic salts, buffers, antioxidants and energy sources),
and
(b) one or more serum-substituting ingredients, optionally selected from but not limited to the following open list: recombinant albumin, chemically defined lipid(s), recombinant insulin and/or metal ion(s) such as zinc, recombinant transferrin or iron, selenium and an antioxidant thiol such as 2-mercaptoethanol or 1-thioglycerol.

Basal media can e.g. be selected from the list comprising but not limited to Eagle's minimal essential medium (EMEM), Dulbecco's modified Eagle's medium (DMEM), alpha-MEM, RPMI-1640, IMDM, and others. If desired, one or more serum-substituting ingredients are added to these media. Thereby, serum-free basal media are obtained.

More preferably, the basal medium is a basal medium that is suitable for culturing of mesenchymal stromal cells and/or mesenchymal stem cells. Preferably, the basal growth medium is suitable for multiple expansion passages of MSCs, particularly of human MSCs, more particularly of stromal Wharton's Jelly derived (preferably human) MSCs.

Media suitable for culturing of mesenchymal stromal cells and/or mesenchymal stem cells are commercially available and some of these are specifically marketed for such purpose. For example, TheraPEAK™ MSCBM CD (throughout this document termed MSCBM CD, for simplicity) is a chemically defined basal medium that has been described to be suitable for mesenchymal stem cells by its manufacturer Lonza (Wakersville, MD, USA; Cat. No. 95062-688). Indeed, the present inventors have identified this basal medium as being particularly suitable. Thus, MSCBM CD is a preferred basal medium for use in the present invention.

Equally preferred are all basal media substantially equivalent to MSCBM CD. The term "substantially equivalent" is defined below. Similarity of the basal medium composition, in terms of ingredients and their relative amounts, can be tested by a combination of chromatographic and/or spectrometric methods, or the like.

In general, finding a suitable culture medium is very important for the overall performance of a cell culture. This has been addressed by identifying MSCBM CD and basal media substantially equivalent thereto as a particularly suitable basal medium for the present invention. MSCBM CD is a chemically defined basal medium for mesenchymal cell and is commercially available from Lonza (Lonza, Wakersville, MD, USA), Cat. No. 95062-688. Variations of MSCBM CD, such as particularly basal media which are composed of or comprise the same constituents as MSCBM CD, although e.g. in other amounts or ratios, or basal media which are composed of or comprise constituents partially or entirely diverse from the constituents of MSCBM CD, albeit with substantially equivalent effect on growth of MSCs, when used as described herein, are equally comprised by the use according to the present invention.

Other basal media are also suitable for expansion of MSCs in the method of the present invention. For example, the inventors have found that DMEM is a basal medium that can also be used, when comprised in a growth medium additionally comprising at least one supplement(s), as described below (data not shown).

In important embodiments of the present invention, the growth medium comprises a basal medium and at least one supplement(s), particularly platelet lysate. Thus, preferably, the at least one cell is expanded in a growth medium which is based on a basal medium and which contains at least one supplement. Platelet lysate is a preferred supplement.

"based on" means that the basal medium, e.g. MSCBM CD, is supplemented with at least one supplement. If the supplements are added in liquid form, then the basal medium is slightly diluted by addition of the at least one supplement; however, a slight dilution does not cause any general concern. In a preferred embodiment, supplements may be selected from the list comprising human or animal serum, human or animal platelet lysate, human or animal or plant cell lysate, one or more antibiotics, heparin, supplementary growth factors such as insulin, or hydrocortisone, amino acids such as glutamine or oligomers thereof.

Preferably the growth medium comprises heparin as a supplement. Suitable concentrations of heparin in the growth medium are 0.1 to 100 U/mL, such as 1 to 10 U/mL, e.g. 2 U/mL. U stands for a unit (Howell unit) of heparin, as described above. Heparin can be added to the basal medium.

In a particularly preferred embodiment, the at least one cell is expanded in a growth medium based on the basal medium MSCBM CD (Lonza, Wakersville, MD, USA; Cat. No. 95062-688), or in a basal medium substantially equivalent thereto. In the present invention, MSCBM CD, for example, has proven to be particularly advantageous for generating high cumulative population doublings/high cell numbers.

Whether or not two basal media are substantially equivalent to each other, for the purpose of the present invention, is tested by growing cells, ideally isolated from the same umbilical cord, in growth media based on said to basal media under substantially the same conditions, i.e. with the same additives (e.g. platelet lysate), in same amounts, in the same culture dishes at the same temperature etc., ideally in parallel. In other words, for testing whether said two basal media are substantially equivalent to each other, the only variable in the growth test is the basal medium itself. The cells are preferably stromal Wharton's Jelly-derived MSCs, isolated as in Example 1 and 2A, and sub-cultured ("P0") as in Example 2B. The cells from P0, after trypsinization, are then subjected to growth, in parallel, in a growth medium based on a first basal medium (e.g. MSCBM CD), and in a growth medium based on a second basal medium (to be tested for substantial equivalence). The two basal media are considered to be substantially equivalent when both of the following are observed: the average cumulative population doubling per plating (P1-P8) is substantially the same (maximum 10 % +/- diversion) and the senescence of the cells obtained after passage 8 (for reference see Example 11) is substantially the same (maximum 10 % +/diversion).

In an alternative embodiment, the growth medium is based on a basal medium which, although not substantially equivalent to MSCBM CD, is characterized as follows: the average cumulative population doubling per plating (P1-P8) is at least 70 % as high, compared to the growth in an otherwise identical growth medium based on the basal medium MSCMB CD, and the senescence of the cells obtained after passage 8 (for reference see Example 11) is 150 % or less, compared to senescence of cells grown in parallel in an otherwise identical growth medium, but based on the basal medium MSCMB CD. DMEM (commercially available from different suppliers) may be an embodiment of such a basal medium.

Preferably, the growth medium supports multi-lineage differentiation of MSCs, particularly of the MSCs as described herein. If necessary, supplements are added to a basal medium to drive differentiation of cells into a particular lineage. Tests for multi-lineage differentiation are described below.

Preferably, platelet lysate or a derivative thereof is present in in the growth medium. Thus, a growth medium useful in the present invention preferably comprises platelet lysate. More specifically, the present inventors have shown that the combination of the basal medium MSCBM CD, or basal media substantially equivalent thereto, with the supplement platelet lysate is particularly suitable, and such growth medium is much preferred. Preferably, when platelet lysate is present, no human or animal serum is present in the growth medium, and more preferably no human, animal or plant cell lysate other than platelet lysate is present. The use of platelet lysate in the growth medium according to the present invention, instead of human or animal sera, addresses concerns associated with the traditional use of animal sera in view of the risks to transmit prions, viruses or to induce immunological reactions in recipients following infusion and regulatory constraints under good manufacturing practice (GMP).

If platelet lysate is present in the growth medium, or more precisely has been added to a basal medium (such as MSCBM CD from Lonza, for example, or a basal medium substantially equivalent thereto), then the platelet lysate can also be referred to as a supplement to the basal medium.

The platelet lysate suitable as supplement in this embodiment generally corresponds to the platelet lysate described for the embodiment of adding platelet lysate during the enzymatic digestion, described, above, however, the following four additional more preferred embodiments are ideally additionally fulfilled, alone or in combination:

In a first particularly preferred embodiment, the platelet lysate is from the same species (however typically not from the same individual) as the umbilical cord. The advantage associated with the cultivation in the presence of platelet lysate from the same species is surprising, particularly in view of prejudice in the prior art against using same-species platelet lysate for culturing of stromal cells: for example, it had been reported that equine platelet lysate is not generally advantageous for the culturing of equine mesenchymal stromal cells culture (Russell et al., 2016, Equine Vet. J., vol. 48, p. 261-264) and that canine platelet lysate is inferior to fetal bovine serum for the isolation and propagation of canine mesenchymal stromal cells (Russell et al., PLOS One, 2015, vol. Vol. 10, e0136621). It is strongly preferred that the platelet lysate used comprises platelets from multiple donors, included in each preparation to compensate individual variability and to obtain a more standardized and reproducible platelet lysate product; pooling of platelets obtained by whole blood-derived buffy-coats is a standardized procedure see e.g. Iudicone et al., 2014, J. Transl. Med., 12: 28. Preferably, the platelet lysate is derived from mammalian blood. Depending on the purpose, a suitable mammal can be chosen as the blood source. The mammalians which are intended used according to the present invention can in principle be all animals, young or adult, from which the required amount of platelets can reasonably be collected. In some embodiments, mainly adult mammalian animals are used. Examples of non-human animals are slaughter animals and other farm-bred animals such as cattle, pigs, sheep or poultry. Mammalian platelet lysate is preferred, human platelet lysate (abbreviated "hPL") is strongly preferred. In some embodiments, the mammal is human. In general, the platelet lysate added is preferably from the same species as the umbilical cord/cells. In particular, for the cultivation of cells originating from human umbilical cord, the addition of human platelet lysate is preferred. The platelet lysate is typically allogeneic to the cells. In some embodiments, blood donors are healthy and satisfy the requirements set forth by relevant regulatory agencies. For example, the blood can satisfy the requirements which a national food/drug administration authority places on products intended for foodstuffs or medical use, or satisfy the corresponding regulations within the geographical area wherein the present invention is put into practice. The human platelet lysate can, wholly or partly, (preferably wholly) replace fetal bovine serum. In most preferred embodiments, the platelet lysate is from human donors. In these embodiments, it is preferable that the donors are under the age of about 50, about 45, about 40, or less.

In a second particularly preferred embodiment, the platelet lysate has increased growth-promoting activity on cells, particularly on Wharton's Jelly-derived MSCs as described herein, when used as a supplement in a cell culture media, compared to bovine serum, particularly fetal bovine serum (FBS) at the same concentration. In some embodiments, the composition has at least 20 % of increased growth promoting activity on cells compared to fetal bovine serum (FBS) at the same concentration.

In a third particularly preferred embodiment, the concentration of platelet lysate in the growth medium is from about 0.1 % (vol./vol.) to about 20 % (vol./vol.), preferably from about 0.5 % (vol./vol.) to about 10 % vol./vol.), more preferably from about 1 % (vol./vol.) to about 5 % vol./vol.), such as 1 % (vol./vol.), 2 % (vol./vol.), 3 % (vol./vol.), 4 % (vol./vol.) or 5 % ( vol./vol.). About 5 % (vol./vol.) has proven particularly useful (see examples herein) and is thus preferred, in particular in combination with a chemically defined basal medium suitable for mesenchymal stem cells, such as MSCBM CD (Lonza), or a basal medium substantially equivalent thereto.

In a fourth particularly preferred embodiment, the platelet lysate which is added to the growth medium comprises derivatives of platelets. Alternatively, the platelet lysate is substantially free of mammalian platelet membranes and/or other cell debris. As used herein, substantially free means that there is only non-detectable amount of platelet membranes, or the amount of platelet membranes cannot be further reduced by any reasonable, commercially sound method, or the amount of platelet membranes do not substantially interfere with the use of the platelet lysate. Platelet membranes can be separated from the platelet lysate using any suitable methods known in the field, such as density gradient.

Some useful embodiments are provided in Examples 0 and 2.

Preferably, platelet lysate or derivatives thereof is/are preferably present during all or part of expansion step (c). Thus, preferably, in step (c) the at least one cell is expanded in a growth medium comprising platelet lysate (PL) or derivatives thereof, preferably human platelet lysate (hPL) or derivatives thereof.

Without wishing to be bound to any theory, it is envisaged that the presence of platelet lysate provides nutrients and growth factors and contributes to an environment that enables efficient expansion of cells. Without wishing to be bound to any theory, it is also conceivable that the presence of platelet lysate aids in balancing out or inhibiting any enzymatic activity remaining after enzymatic extraction, and thus to the mildness of conditions during the subsequent expansion phase. Human platelet lysate (hPL) is preferred for use with human MSCs.

Thus, in the present invention, platelet lysate (PL) can be an alternative to animal serum for culturing mesenchymal stromal cells (for illustration and further details see also WO 2013/042095 A1). Platelets are known to be a rich source of numerous growth factors such as PDGF (platelet-derived growth factor), b-FGF (basic-fibroblast growth factor), VEGF (vascular endothelial growth factor) and TGF-$\beta$ (transforming growth factor-$\beta$).

Optionally, one or more antibiotics are present in the growth medium. If so, the one or more antibiotics may be added to the basal medium as supplements. Suitable antibiotics in the context of the present invention include e.g. amphotericin and gentamycin, among others. The concentrations of the one or more antibiotics in the growth medium are preferably in the concentration ranges normally used in the art. Alternatively, no added antibiotic is present in the growth medium.

Preferably, no additional ingredients beyond the ones listed above are added to the basal medium. In particular, it is much preferred that the at least one cell is expanded in a growth medium that does not comprise any added serum, in particular no bovine serum, such as fetal bovine serum (FBS). For the purpose of that embodiment, platelet lysate does not fall under the definition serum; in other words, even if some serum or traces of serum may be comprised in platelet lysate added to the growth medium, the growth medium can be said not to contain "added serum", which is intended to specify that serum is not added a as cell culture ingredient in the classical sense. In the present invention FBS is an undesirable additive, e.g. because it bears the risk of transmitting viral and prion diseases. In contrast, the method of the present invention works efficiently in the presence of platelet lysate, e.g. as a substitute for FBS.For growth of human MSCs, human platelet lysate is preferred as an additive over FBS.

Preferably, no attachment matrix is provided or present when the cells are subjected to a passage on a growth plate. Thus, no attachment matrix is provided or present when the cells are plated and/or no attachment matrix is added as a supplement to the basal medium.

Passages

In a preferred embodiment, the cells are expanded over one or more passages.

"passage" refers to a culture of cells in a culture vessel (of a certain type), within which culture vessels the cells are being seeded to initiate the respective passage. Normally the cells adhere to said culture vessel while they are being cultivated, and the cells normally expand within said culture vessel while being cultivated. The end of a passage is defined by detachment of the normally adherent cells (e.g. by trypsinization). Subsequently cells are then optionally seeded into a fresh culture vessel of the same or different type; the seeding into the fresh culture vessel defines the initiation of a subsequent passage. Thus, when the cells, following such expansion, are transferred from that culture vessel to another culture vessel, then the cells present in that other culture vessel represent the next passage.

"harvest" refers to the process of obtaining substantially all cells grown in a culture vessel at the end of a passage.

For each passage, one culture vessel may be used; more preferably however, multiple culture vessels are used in parallel per each passage. Cells are then cultured in parallel in the multiple culture vessels, under substantially identical conditions for substantially the same time. Preferably, the seeding density in the multiple culture vessels is substantially the same.

At the beginning of each passage, cells (non-adherent/isolated, preferably at a desired number/density) are added to a culture vessel comprising growth medium.

Although not required for cell growth, antibiotics are often used to control the growth of bacterial and fungal contaminants. This is particularly preferred at early passages, but it is not preferred at later passages, such as e.g. from P2 onwards or from P1 onwards. Thus, even if antibiotics are present in the first passage following isolation of the cells ("P0"), antibiotics are preferably discontinued for all passages after P0; in other words, a growth medium without addition of antibiotics may be used for P1 and all subsequent passages. Thus, in the present invention, antibiotics are preferably present as a supplement in the first cell culture (e.g. P0) after isolation of the cells from umbilical cord, but not in later cell cultures, i.e. sub-cultures of P0.

At the end of each passage, when the cells have reached a confluence percentage according to criterion 3 in the table below, the cells are harvested. For that purpose, the growth medium (and any non-adherent cells comprised therein, if any) is aspirated, the adherent cells are optionally washed with a suitable sterile buffer and optionally but preferably enzymatically detached, such as trypsinized, optionally counted, optionally but preferably concentrated by centrifugation, and optionally but preferably re-suspended, preferably in growth medium suitable for MScs, as described herein. The re-suspended non-adherent cells are then optionally used to be added at the beginning of the subsequent culture passage.

The passages are consecutively numbered as described herein below in detail, beginning with passage 0 (P0). P0 is the first passage after obtaining the cells, preferably by enzymatic digestion as described above. Thus, P0 can be referred to as "primary culture". The cells for inoculation of the primary originate from the Wharton's Jelly of the umbilical cord, from mechanically and/or enzymatically dissociated tissue, as described above, or - alternatively but much less preferred - from an outgrowth of migrating cells from stromal Wharton's Jelly tissue.

By growing cells in various passages, the cells can be expanded to higher total cell numbers and/or higher homogeneity and/or less contamination by non-desired cells than what would be possible in one single passage only. The cells can be expanded at defined conditions or otherwise desired conditions.

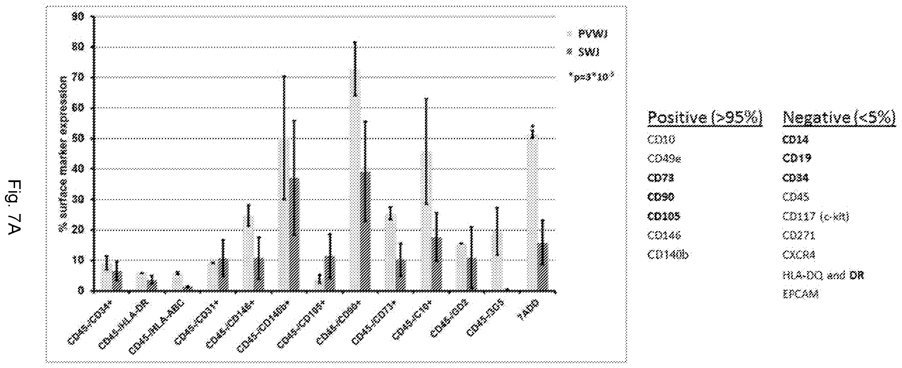

It is generally recognized that stromal cells of the umbilical cord (UC) are heterogeneous regarding structure, biological properties and localization within the UC. For example, Fig. 7A shows that the freshly isolated UC-derived cells, both according to the description herein and according to the state of the art, are heterogenous regarding display of certain surface markers.

It is typical that not all cells isolated from the stromal Wharton's Jelly (nor any other area of the umbilical cord) are clonogenic. In other words, not all isolated cells are colony forming units (CFU). However, by growing the cells isolated according to the present invention for multiple passages, the desired mesenchymal stromal cells, which are clonogenic, can be enriched. Thus, in one embodiment, the expansion according to the present invention is characterized in that the percentage of MSCs increases over time. For example, the percentage of MSCs at the end of P0 may be typically higher than the percentage of MSCs at the beginning of P0.

In the present invention, multiple culture passages confer the advantage that MSCs can be enriched, with respect to other (non-desired or contaminating) cells, such as red blood cells, because (a) non-adherent (and thus non-MSC) cells are removed together with the growth medium at the end of a culture passage, (b) cells with proliferative properties inferior to MSCs will substantially not proliferate as well as MSCs, and thus, over the course of several cumulative passages, the cell population becomes enriched in MSCs, and eventually (normally from P1 onwards) substantially pure for MScs.

It is generally possible to arithmetically convert the passage number into cumulative population doublings, as long as defined or otherwise well-established conditions are used.

The cells are preferably expanded in multiple passages, preferably more than one to less than fourteen passages. For example, the cells are cultured for three to six passages.

In one precise embodiment, freshly isolated cells are, after enzymatic digestion, initially subjected to a primary culturing passage (for exemplary illustration see Example 2B) - the primary culturing passage can be referred to P0; optionally expansion is repeated in a new passage under conditions for P0 (the repeated culturing passage is then referred to as P0+). Subsequent passages, i.e. following after P0 directly, or after P0+, are numerically numbered as P1, P2, and so on. In a preferred embodiment, cells are expanded at least until P2 and not more than until P6. In a most preferred embodiment, the cells are expanded until P4.

Regarding cumulative Population Doublings, it is typically difficult to determine the cumulative Population Doublings during P0. The principal reason is that, in the experience of the present inventors, not all cells seeded at the beginning of P0 will expand. In order to determine the cumulative Population Doublings during P0, it would be required to determine the number or fraction of clonogenic cells at the beginning of P0, i.e. those cells which are capable for expand, thereby giving origin to daughter cells. While such determination is as such not generally unfeasible, it is not required for the method of the present invention; instead, in such embodiments, cumulative Population Doublings are determined for all passages except P0. While the cells originally isolated from the umbilical cord typically comprise MSCs, which are clonogenic, as well as other cells (e.g. red blood cells), which are not clongenic, the other cells will substantially not be present beyond P0, as described below *inter alia* because such non-clonogenic cells do not generate offspring during P0.

In one embodiment, cells are expanded for 5 to 30 cumulative Population Doublings (not counting cumulative Population Doublings during P0), such as 8 to 25 cumulative Population Doublings (not counting cumulative Population Doublings during P0). In one preferred embodiment, cells are expanded for only 5 to 14 cumulative Population Doublings (not counting cumulative Population Doublings during P0). This embodiment is suitable for creation of a Primary Cell Bank.

In an alternative preferred embodiment, cells are expanded for 14 to 25 cumulative Population Doublings (not counting cumulative Population Doublings during P0). This embodiment is suitable for creation of a Secondary Cell Bank.

In the experience of the inventors, the cumulative Population Doublings during P1 and P2 (total of P1 plus P2, but not including P0), as a very general rule, are normally in the range of 7 to 15, preferably 9 to 13. In the experience of the inventors, the cumulative Population Doublings during P3 and P4 (total of P3 plus P4, but not including P0, P1 and P2), as a very general rule, are normally in the range of 4 to 12, preferably 6-10. Consequently, the cumulative Population Doublings during P1, P2, P3 and P4 (total of P1 plus P2 plus P3 plus P4, but not including P0), as a very general rule, are normally in the range of 9 to 27, preferably in the range of 15 to 23. These numbers do not include the cumulative Population Doublings during P0.

At the beginning of each passage, cells are transferred to a cell culture vessel. Preferably, the cells are transferred in a specific amount of cells per culture vessel. The amount is preferably defined as a function of bi-dimensional surface of the cell culture vessel. Thereby it is possible to start the passage with a defined cell density. Cell densities for starting culture passages in the context of the present invention are as follows:

| | For starting P0 and P0+ | For starting P1 and all passages subsequent to P1 |
|---|---|---|
| Suitable range | $1.000 - 50.000$ cells/cm$^2$ | $200 - 50.000$ cells/cm$^2$ |
| Preferred range | $8.000$ cells/cm$^2$ or more | $2.000 - 3.000$ cells/cm$^2$ |
| Defined value | Not applicable | $2.500$ cells/cm$^2$ |

At the beginning of each of P0 and P0+ (if applicable), the total of available cells are seeded. Therefore, no exactly defined cell density for initiating P0 and P0+ (if applicable) has been defined. At any rate, the cell density for initiating P0 and P0+ (if applicable), should be $8.000$ cells/m$^2$ or higher.

As is common for mesenchymal stromal cells, the cell as described herein is typically an adherent cell when grown in a culture vessel, particularly a culture vessel with a plastic ground surface. Preferably, the cell is adherent irrespective of the choice of growth medium. At any rate, it is much preferred that the cell is adherent when cultivated in a preferred growth medium according to the present invention.

At the end of each passage, the cells are harvested. For that purpose, they are optionally but preferably enzymatically detached, such as trypsinized. As is commonly known, trypsin is a commercially available enzyme suitable for proteolytically digesting cell adherence molecules, and thus separating adherent cells from the culture vessel and from each other. "trypsinized" thus means treated with trypsin - or any other enzyme with a trypsin like digestion pattern - under conditions suitable for such digestion. Standard methods for trypsinizing of mammalian cells, particularly mesenchymal cells, are also suitable for trypsinizing the cells obtained as described herein. Trypsin preparations for such purposes are commercially available and may be used in accordance with the manufacturer's instructions. Alternatively, the enzymatic detachment is performed with a suitable enzyme other than trypsin.

Following enzymatic detachment, e.g. by trypsinization, optionally but preferably the total cell number (per flask or per plate or per stack or per total of all culture vessels used in that respective passage) is determined.

When it is said herein that cells are expanded until a certain passage, e.g. until P4, then it is meant that the expansion of cells is not continued further or interrupted after that respective passage.

After each passage, the following acceptance criteria are preferably assessed: 1 yield, 2 viability, 3 confluence, 4 morphology. Determination of acceptance criteria 3 and 4 occurs prior to enzymatic detachment of the cells. Determination of acceptance criteria 1 and 2 occurs after enzymatic detachment of the cells. The details are as follows.

| Acceptance criterion | | After P0 and P0+ | After P1 and all passages following P1 | Test |
|---|---|---|---|---|
| 1 | Yield (Full Scale Run) | See Example 2B | See Example 2C | Cell counting after enzymatic detachment |
| 2 | Viability | More than 80 % of cells viable | More than 80 % of cells viable | Staining with 7-amino actinomycin D (7-AAD) after enzymatic detachment |
| 3 | Confluence | At least 80 %, preferably 80 to 90 % confluent | At least 80 %, preferably 80 to 90 % confluent | Microscopic determination |
| 4 | Morphology | Majority of cells small, spindle-shaped and in defined colonies | Majority of cells small, spindle-shaped and in defined colonies | Microscopic determination |

Preferably, all four acceptance criteria must be fulfilled.

The microscopic determination of confluence is directed at determining the percentage of the surface area of the cell culture vessel that is covered by cells adhered thereto.

If the confluence criterion is not fulfilled, the cells are expanded further, and regularly observed in order to find out at what time the confluence criterion is fulfilled.

For determination of the yield, the cells are enzymatically detached, e.g. subjected to trypsinization, and a defined fraction of the cell-containing suspension is subjected to automated cell counting, such as by the NucleoCounter®. The total number of cells is then determined arithmetically based on the fraction.

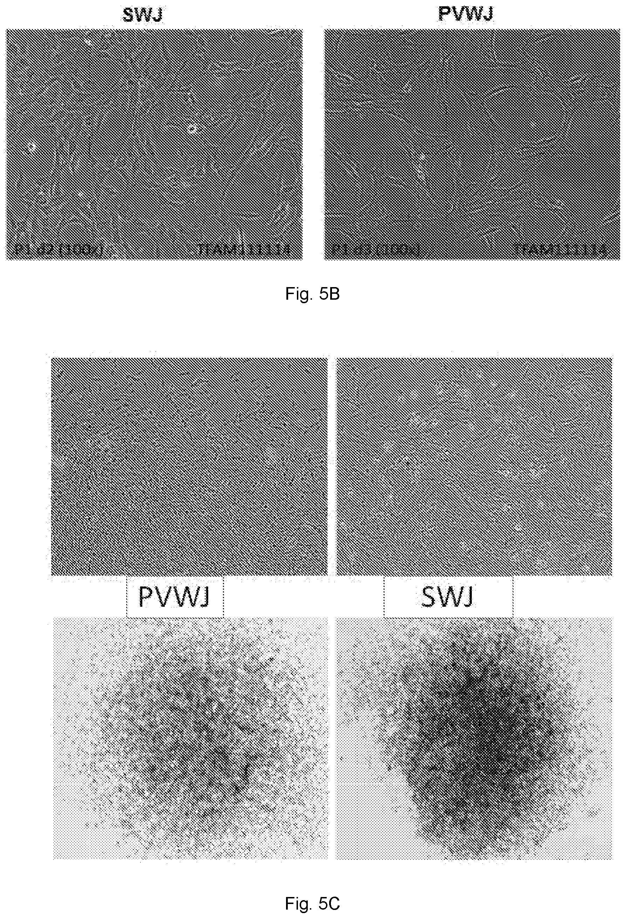

The microscopic determination of morphology (i.e. cell morphology) is directed at determining the shape of cells adhered to the ground surface area of the cell culture vessel. It is typical for MSCs that the majority of the cells is spindle-shaped. For illustrative purposes, a photograph showing a view onto the ground surface of a cell culture dish comprising such cells is shown in Fig. 5 C, panel termed "SWJ").

The determination of the viability ensures that the cells that are subjected to preparation of a cell bank and/or to a subsequent passage are fit for further expansion (following freezing and thawing, as the case may be). The cells as described herein are superior in viability compared to MSCs isolated according to methods according to the state of the art. Thereby, and by relying on expansion according to the present invention, large cell populations can be obtained even when the total number of cells initially isolated from the stromal Wharton's Jelly of the umbilical cord is relatively low. Thus, the cells obtainable according to the description herein are superior in their proliferative potential.

Freezing

The cells can be frozen, e.g. snap-frozen in liquid nitrogen, according to standard procedures. It is well possible to de-frost such frozen cells and to e.g. subject them to further culture passages after de-frosting. The freezing can occur either as entire batch or in aliquots.

When it is said herein that cells are expanded until a certain passage, e.g. until P4, then it is meant that the expansion of cells is not continued further, or interrupted, after that respective passage. An exemplary but typical embodiment is that cells are frozen after the respective passage. Repeated freezing after repeated passages is possible. For example, cells may be frozen after P2 and/or after P4. Preferably, the cells are frozen after P2 and after P4.

The frozen cells and populations of frozen cells are also subject of the description herein. Populations of frozen cells can be referred to as Cell Banks. Suitable cell banks that are obtainable in the context of the description herein will be described below.

In one embodiment, cells are frozen after 5 to 30 cumulative Population Doublings (not counting cumulative Population Doublings during P0), such as 8 to 25 cumulative Population Doublings (not counting cumulative Population Doublings during P0). In one preferred embodiment, cells are frozen after only 5 to 14 cumulative Population Doublings (not counting cumulative Population Doublings during P0). This embodiment is suitable for creation of a Primary Cell Bank. In an alternative preferred embodiment, cells are frozen after 14 to 25 cumulative Population Doublings (not counting cumulative Population Doublings during P0). This embodiment is suitable for creation of a Secondary Cell Bank.

Standard methods for freezing of mammalian cells, particularly mesenchymal cells, are suitable for freezing the cells obtained according to the description herein. Preferably, the cells are frozen at temperatures of -70 °C or less (such as -80 °C), optionally preceded by snap-freezing at temperatures of -190 °C or less. In one embodiment, the cells are frozen in liquid nitrogen.

The frozen cells can optionally but preferably be stored in frozen form. Standard methods for storing of mammalian cells, particularly mesenchymal cells, are suitable for storing the cells obtained according to the description herein. It is preferable to freeze the cells in vials. In one embodiment, the frozen cells are stored in liquid nitrogen.

In a preferred embodiment, the cells are aliquoted prior to freezing. Suitable containers for containing aliquots of the cells obtainable according to the description herein are all containers suitable for receiving respective amounts (aliquots) of cells, and for storage at low temperatures, particularly vials. A vial (also known as a flacon) is a small glass or plastic vessel or bottle, suitable to store liquids. The nature of the vial suitable in the description herein is not particularly limited as long as it allows for containing the cells in medium as indicated, and as long as it is suitable for being frozen at conditions at which the cells remain at a state which allows their subsequent thawing and sub-culturing. Thus, for that purpose the vials must be freezer-competent (cryo-vials). Vials may be made of any suitable material, although glass vials and vials made of plastics such as polypropylene are most typical. The vial can be closed, for which purpose it typically has a lid, and a cryo-vial suitable for use according to the description herein is preferably suitable for freezing at temperatures of - 196 °C or less. Lid options include screw vials (closed with a screw cap or dropper/pipette), lip vials (closed with a cork or plastic stopper) and crimp vials (closed with a rubber stopper and a metal cap), or, for the case of plastic vials, also 'hinge caps' (snap caps), which snap shut when pressed. The bottom of a vial is preferably flat, however this is not a strict requirement. Preferably, all vials used in one given isolation/expansion process are all identical to each other and filled with identical volumes of cell-containing liquid (cells in medium, typically in suspension, but this is not a requirement).

Preferred vials have a size that is suitable to contain between 2 and 10 ml of cells in medium (typically in suspension, but this is not a requirement), such as between 3 and 8 ml of cells in medium, and most preferably ca. 4 ml of cells in medium. Preferred vials have a size that is suitable to contain between $10^6$ and $10^9$ cells in medium, such as between $10 \times 10^6$ and $100 \times 10^6$ cells in medium, and most preferably ca. $20 \times 10^6$ cells, in medium.

The frozen and/or stored cells can optionally but preferably be thawed. After thawing, the cells can optionally be subjected to further culturing (sub-culturing), including expansion. Standard methods for thawing and optionally sub-culturing, particularly mesenchymal cells, are suitable for thawing the cells obtained according to the description herein. For further culturing, or sub-culturing, the methods described herein, including growth media etc. are suitable.

In a preferred embodiment, the cells are frozen following passage 2 (P2). The cells obtained and frozen after P2 are referred to herein as "Primary Cell Bank" or "PCB", or alternatively "Master Cell Bank". In one preferred embodiment, the Primary Cell Bank comprises or consists of cells that have been frozen after 5 to 14 cumulative Population Doublings (not counting cumulative Population Doublings during P0).

In another preferred embodiment, the cells are frozen following passage 4 (P4). The cells obtained and frozen after P4 are referred to herein as "Secondary Cell Bank" or "SCB", or alternatively "Working Cell Bank". In a preferred embodiment, the Secondary Cell Bank comprises or consists of cells that have been frozen after 14 to 25 cumulative Population Doublings (not counting cumulative Population Doublings during P0).

Preferably, the PCB embodiment and the SCB embodiment are linked, so that cells are first frozen as a PCB, and then subsequently, all or part of the PCB is thawed and sub-cultured, and then the SCB is obtained after two further passages (P4 in total). Preferably, not all cells obtained and frozen after P2, e.g. not the entire PCB, is/are thawed and sub-cultured together. Rather, most preferably, one container (typically one vial) obtained and frozen as PCB is thawed and sub-cultured, while the other containers (vials) remain frozen as PCB.

It is important to note that references to individual passages are given herein for guidance purposes. For example, it is also possible to prepare the PCB after a passage other than P2. The preference for preparing the PCB after P2 and the SCB after P4 is based on the experience with growth in a medium based on the basal medium MSCBM CD, or a basal medium substantially equivalent thereto, at the culture conditions specifically described in the experimental examples (particularly Example 2). When other culture conditions are used, which is equally possible in the context of the present invention, then the PCB and the SCB are ideally prepared after cumulative Population Doublings that corresponds to the cumulative Population Doublings observed after P2 and P4, respectively, in the experimental examples herein, in particular Example 2C. Isolation of an individual cell, if desired, can be achieved e.g. by serial dilution, pre- or post-expansion. Unless such isolation is specifically performed, the method of the invention yields a population of mesenchymal stromal cells. As described in the following, the yield can be relatively high, compared to previously known methods, *inter alia* in view of the superior viability and expansion properties of the cells as described herein.

Yield

The method according to the present invention, comprising step (c), yields a population of mesenchymal stromal cells. Such a population of mesenchymal stromal cells is described herein. Preferably, the method of the present invention provides a cell population devoid of blood cells. Further descriptions of the cell population yielded in the method of the invention are described herein.

In a preferred embodiment, cells yielded during the expansion according to the present invention are aliquoted and optionally frozen. A suitable point in time for aliquoting and freezing the cells is after completion of any passage, and subsequent enzymatic detachment, e.g. trypsinization, centrifugation and resuspension in growth medium. The cells (cell population), which are so-obtained and stored by freezing, preferably in aliquots, can also be referred to as cell bank.

In one preferred embodiment the cells are frozen following P2. The cells (cell population) which are so-obtained and stored by freezing, preferably in aliquots, can also be referred to as Primary Cell Bank. In one preferred embodiment, the Primary Cell Bank comprises or consists of cells that have been frozen after 5 to 14 cumulative Population Doublings (not counting cumulative Population Doublings during P0).

In one additional but not mutually exclusive preferred embodiment the cells are frozen following P4. The cells (cell population) which are so-obtained and stored by freezing, preferably in aliquots, can also be referred to as Secondary Cell Bank. In a preferred embodiment, the Secondary Cell Bank comprises or consists of cells that have been frozen after 14 to 25 cumulative Population Doublings (not counting cumulative Population Doublings during P0).

The so-obtained frozen aliquots from the respective cell bank are available for thawing individually at a desired point in time. In other words, it is not necessary - and typically not desired - to thaw all aliquots of the cell bank at the same point of time. In contrast, the aliquoting and individual freezing of the aliquots provides the advantage that individual aliquots can be removed from freezing, thawed and subjected to further culturing (sub-culturing, further expansion) at individual, and individually desired points in time.

A full-scale run starting with human umbilical cord and comprising step (c) and running until passage P2 typically yields a Primary Cell Bank (PCB) of at least $1 \times 10^9$ cells, more preferably at least $2 \times 10^9$ cells, more preferably at least $3 \times 10^9$ cells, more preferably at least $4 \times 10^9$ cells, more preferably at least $5 \times 10^9$ cells, more preferably at least $6 \times 10^9$ cells, more preferably at least $7 \times 10^9$ cells, more preferably at least $8 \times 10^9$ cells, more preferably at least $9 \times 10^9$ cells, and in some embodiments at least $10 \times 10^9$ cells. In one preferred embodiment, the Primary Cell Bank comprises or consists of cells that have been frozen after 5 to 14 cumulative Population Doublings (not counting cumulative Population Doublings during P0).

Owing to the excellent proliferative properties of the cells as described herein, the cells can be cultivated, however, well beyond P2. This may occur either by passing some or all of the cells obtained during P2, after enzymatic detachment (e.g. trypsinization) to a subsequent passage (P3), or by creating a Primary Cell Bank following P2 and then thawing one or more vials of cells from the Primary Cell bank at a desired point in time, to thereby initiate a subsequent passage (P3). In any case, P3 may be followed by one or more additional passages, and, optionally, a Secondary Cell bank may be generated, e.g. following P4. Due to the excellent proliferative properties, the total number of cells after a later passage, e.g. P4, will normally be significantly higher than the total number of cells after P2, for which typical yields are indicated above. For example, after P4, the total yield of the cells may be at least 10 fold as high as after P2, but more typically at last 100 fold to at least 1000 fold as high as after P2. In some embodiments, after P4, the total yield may be at least $10 \times 10^{12}$ cells, with respect to one full scale run. Optionally, a Secondary Cell bank is prepared following P4. In a preferred embodiment, such Secondary Cell Bank comprises or consists of cells that have been frozen after 14 to 25 cumulative Population Doublings (not counting cumulative Population Doublings during P0).

The yield may optionally be further increased by further sub-culturing, e.g. passage P5 and beyond.

Differentiation potential

The potency of the cells as described herein may be assessed experimentally. In general, this is done by subjecting a cell to conditions under which differentiation into one or more specific cell types is favoured.

Preferably, the cell(s) obtained after step (c) is at least multipotent, and optionally pluripotent. In a typical embodiment, the cell(s) obtained after step (c) are multipotent. Multipotency can be tested by a differentiation assays, as commonly known in the art. Under special conditions a multipotent cell will differentiate into tissues (adipogenic, chondrogenic and osteogenic). Examples 12, 13 and 14 show that the cells as described herein have such capabilities. Under special condition the cell will differentiate into tissues of different tissues (adipogenic, chondrogenic and osteogenic). Preferably, a multipotent cell obtainable by the description herein has at least all of the following differentiation potentials: (a) adipogenic; (b) chondrogenic; (c) osteogenic. Examples 12, 13 and 14 show that the cell as described herein has such capabilities. In these examples, particularly Example 12, specific conditions are described which allow for differentiation despite the rapid expansion of the stromal Wharton's Jelly-derived cells as described herein.

Optionally, the cell has further differentiation potentials are selected from the differentiation potentials typical for mesenchymal stromal cells. However, as is typical for mesenchymal stromal cells, the cell as described herein is typically not totipotent.

The cell as described herein is, however, typically not totipotent. Nevertheless, the cell may optionally be subjected to genetic manipulation and/or nuclear reprogramming, whereby the differentiation potential may be influenced.

Relationship to other descriptions

By the method of the invention, a cell can be obtained, as described in detail herein. By the method of the invention, a cell population can be obtained, as described in detail herein. For example, by the method of the invention, a cell bank can be obtained. In one embodiment, a cell bank is obtainable by the method according to the first aspect of the invention. In that embodiment, step (c) of the method of the invention is optionally followed by a step (d) of aliquoting the cells and a step (e) of storing the cells. The cells can be stored at any suitable condition, although freezing at temperatures of 180 °C or less is most typical. Cells may be frozen, preferably snap-frozen in liquid nitrogen. A cryoprotectant may be added prior to freezing.

Because the aspects of the present invention are related to each other, embodiments described with respect to the descriptions herein may also be read into the first aspect, and vice versa. For example, by showing substantial absence of endothelial marker proteins, by expression profiling or surface marker determination, as described herein, it can be experimentally confirmed that an aspect of the method according to the first aspect of the invention has been properly implemented, namely the removal of the epithelium.

It is particularly preferred that the cell population obtainable by the method of the first aspect of the invention is a cell population as described herein further below. It is also particularly preferred that the method for obtaining a cell according to the first aspect of the invention yields a cell as described herein; thus all features that are described herein as characterizing the cell as described herein are also suitable to characterize the method for obtaining the cell according to the first aspect of the present invention. It is also particularly preferred that the cell obtainable by the method of the first aspect of the invention is a cell as described herein, as follows.

Mesenchymal stromal cell

Further description herein relates to a mesenchymal cell. More precisely, the mesenchymal cell is a mesenchymal stromal cell (MSC). The features of the cell will be described in the following.

Some features of the cell as described herein, which will be described in the following, are related to physical characteristics of the cell as described herein. Merely for illustration, and notwithstanding the details disclosed elsewhere herein, physical characteristics include those characteristics which can be directly determined with physical methods, such as gene expression, size, shape etc. of the cell. Some features and descriptions of the cell as described herein, which will be described in the following, are related to the method by which cell as described herein is obtainable or obtained. Merely for illustration, and notwithstanding the details disclosed elsewhere herein, characteristics of the cell related to the method by which cell as described herein is obtainable or obtained can include (a) the anatomical origin of the cell, in particular the umbilical cord and the stromal Wharton's Jelly as a preferred section thereof, (b) the conditions applied for isolating the cell and optionally but preferably also (c) the conditions applied for expanding the cell.

Isolated Cell

Preferably, the cell as described herein is an isolated cell, particularly an isolated mesenchymal stromal cell. In particular, the mesenchymal stromal cell as described herein is preferably an isolated mesenchymal stromal cell originating from the intervascular area (for illustration see Fig. 1) of the umbilical cord, or derived from such a cell. An isolated stromal cell can be obtained from the stroma by a method comprising isolation as described herein. A cell can be derived from such a cell by expansion. Thus, in general, when it is said that a given cell is "derived from" another cell, then it is meant that the given cell originated from said other cell, by expansion (cell division) and/or differentiation.

Also described, the cell is an isolated cell which is located outside a human or animal body. An isolated cell is generally a cell outside its physiological *in vivo* environment. Preferably, the cell is an ex *vivo* cell. In particular, the ex *vivo* cell is preferably located outside the umbilical cord. In particular, the ex *vivo* cell is preferably not in contact with blood vessels, be it direct contact or indirect contact. Also described, the ex *vivo* cell is in a culture vessel. Also described, the ex *vivo* cell is in a storage-suitable container, such as in a vial. Also described, the ex *vivo* cell is at a temperature of ca. 35 °C to 39 °C, preferably in a culture vessel. Also described, the ex *vivo* cell is in a cryopreservation-suitable container, such as in a cryovial, preferably at a temperature of -180 °C or less. Also described, the ex *vivo* cell is in a culture vessel. Also described, the ex *vivo* cell is a metabolically active cell. Also described, the ex *vivo* cell is frozen.

Also described, the cell is characterized by at least one feature - structural or functional - that is not normally found in a cell of the human umbilical cord. For example, such a feature may be a feature specific for the method by which the cell as described herein is obtainable. Such structural or functional feature may be selected from the features explicitly described in this disclosure, and/or from features not explicitly described in this disclosure, but inherent to the cell as described herein, e.g. inherent to the cell obtainable by the method of the invention. Also described, the cell is an adherent cell, e.g. adherent to a culture vessel, such as a culture vessel with a plastic ground surface.

An isolated stromal cell as described hereincan be expanded *in vitro* by culture. Thereby a derived cell can be obtained. The derived cell is also a cell as described herein, as long as it complies with the characteristics defining the cell as described herein. Thus, due to the stem cell-like properties of the cell as described herein, multiple generations of cells can be covered. Multiple generations are obtainable by expansion as described herein. Indeed, a specific description of the cell as described hereincomprises expansion of the cell ex *vivo.* Thus, although the cell is isolated, it is capable to give offspring to same or similar cells, and thus to generate a cell population. Such a cell population is described herein. Suitable culture conditions are not particularly limited but include those described in the first aspect of the invention.

A suitable but non-limiting method for isolation of a cell, including enzymatic treatment, is described herein in the context of the first aspect of the present invention. Isolation of individual cells, if desired, can be achieved e.g. by serial dilution. Thus, preferably, the cell as described herein is a cell in an ex *vivo* environment, such as *in vitro.* Various methods for cultivating cells *in vitro* can be practiced by the skilled person, including those described with respect to the first aspect of the present invention.

In general, a mesenchymal stromal cell can originate from various tissues, such as bone marrow, adipose tissue and umbilical cord. Preferably, the cell as described herein originates from or is derived from the umbilical cord, more preferably from the stromal Wharton's Jelly of the umbilical cord. The cell as described herein is not, however, derived from umbilical cord blood. The cell is also neither an epithelial nor an endothelial cell.

Also described, the cell as described herein is a freshly isolated cell. A freshly isolated cell is a cell that has been directly obtained by isolation from the tissue it originates from, such as by the method described above. More preferably, however, the cell as described herein is a cell that is derived from a freshly isolated cell, most typically by expansion *in vitro.* There is no limitation as such on the number of passages or cumulative Population Doublings *in vitro,* following fresh isolation of a cell; however, typically the cell as described herein is derived from a freshly isolated cell by expansion *in vitro* for 12 passages or less, or an equivalent number of cumulative population doublings. Generally, any cell complying with the claimed limitations is a cell as described herein, irrespective of the number of passages or cumulative population doublings by which it was obtained, following fresh isolation from the tissue of origin.

As is common for mesenchymal stromal cells, the cell as described herein is typically an adherent cell when grown in a culture vessel, particularly a culture vessel with a plastic ground surface. Thus, also described, the mesenchymal stromal cell as described herein is a surface-adherent cell, particularly a plastic-adherent cell *in vitro.* If desired, a plastic-adherent cell can be detached from the culture vessel, e.g. by trypsinization. Thereby, a non-adherent cell is obtainable. The description comprises also such a non-adherent cell.

Also described, the mesenchymal stromal cell as described herein is spindle-shaped. This applies normally when the mesenchymal stromal cell is adhered to a culture vessel, such as a culture vessel with a plastic ground surface. In particular, in a population of mesenchymal stromal cells, the majority, e.g. 50 % or more by cell number, are normally spindle-shaped, particularly when adhering to a plastic surface *in vitro.*

When the cell as described herein is not adherent, e.g. when it has been enzymatically detached such as e.g. trypsinized, such as e.g. when it is present in a Primary or Secondary Cell Bank, optionally frozen, the cell is not normally spindle-shaped. Thus, the spindle-shape is specifically associated with surface adherence under culturing conditions.

Also described, the cell as described herein is obtainable by the method described in the first aspect of the invention. In that description, the cell as described herein is suitably selected from a freshly isolated cell, i.e. a cell that has not been subjected to ex *vivo* expansion, or an expanded cell, i.e. a cell obtainable by ex *vivo* expansion of a previously freshly isolated cell.

Gene Expression and Surface Antigen Display

The cell as described herein may be characterized by a gene expression pattern. As used herein, "gene expression pattern" means that at least one gene is expressed, or not expressed, or expressed at a certain level, as the case may be. Thus, the fact that this particular gene is expressed, or not expressed, or expressed at a certain level, characterizes the cell as described herein, e.g. for the purpose of distinction from a reference cell. The same applies for the expression of more than one gene, such as e.g. two genes, three genes, four genes, five genes, six genes, seven genes, eight genes, nine genes, ten genes, etc. When the cell as described herein is characterized by the expression of one or more genes, it is not desired to make a specific statement on the expression of any other gene not mentioned in that context. As an illustrative example, wherever it is said herein that the cell as described herein expresses APCDD1, this should not be taken as a statement that any specific other gene is also expressed, or not expressed, or expressed at a certain level, unless the context dictates otherwise.

In general, the expression of a gene can be tested by various methods, both on nucleic acid level and on protein level. Gene expression can be relatively commonly tested in molecular biology, biochemistry and cell biology, and any method known or suitable therefor may be used in the context of description herein. Some methods are described below. Methods relating to gene expression can also be referred to as transcriptomic methods. Hence, the gene expression profile of a cell can also be referred to as transcriptome. Also described, the cell as described herein is characterized by its gene expression profile.

Different terms can be used to describe that the expression of a gene is being analyzed: for example, the expression can be said to be tested, analyzed, assessed, assayed, measured, determined etc. These terms include both the qualitative determination, i.e. the question whether the respective gene is expressed or not, and the quantitative determination, i.e. the question at what the level the respective gene is expressed.

Typically, for determination of gene expression, a sample of one cell or a multitude of cells (which is preferred) is taken from a cell population, and the gene expression is subsequently analyzed in that sample, whereby the cells in that sample may be destroyed or not, as the case may be. Since the cell population as described herein is normally homogeneous, meaning that substantially all individual cells comprised therein are much alike or similar, the finding on gene expression in the cells comprised in the sample taken from the cell population (representative sample) will usually also apply to the remaining cells in that population, unless the context dictates otherwise and/or the cell population does not appear to be substantially homogeneous.

Determination of the expression of any respective gene may either be an undirected determination, i.e. where gene expression is assayed in general (e.g. by a broad approach such as microarray) without focusing on one gene, or a directed determination, where it is specifically tested whether the respective gene is expressed. In the second case particularly, the respective gene may also be said to be a "gene of interest".

Several transcriptomic technologies can be used to generate the data for gene expression analysis. For example, DNA microarrays measure the relative activity of previously identified target genes. Sequence based techniques, like RNA-Seq, provide information on the sequences of genes in addition to their expression level.

The cell as described herein is a cell from a placenta animal, preferably mammal, preferably primate, more preferably human. Thus, also described, the mesenchymal stromal cells (MSCs) as described herein is a mammalian cell. Therefore, unless specified otherwise, all indications to a specific gene mean the respective mammalian gene.

More preferably, the mesenchymal stromal cell as described herein is a cell from a primate, more particularly from a human. Thus, more preferably, the mesenchymal stromal cells (MSCs) as described herein is a human cell. Therefore, in that description, all indications to a specific gene mean the respective human gene. In some cases this is directly apparent from the context, such as in the case of the human leukocyte antigens (HLA), in other cases this is not specifically said in the context; however it is evident: for example, with reference to a human cell, expression of APCDD1 means expression of the human APCDD1, etc. As the cell as described herein is, in one preferred description, a human cell, it is preferred that the cell as described herein in that description expresses human APCDD1.

Preferably, the cell as described herein is not a transgenic cell. Therefore, all indications concerning expression of a specific gene mean that the respective gene is expressed from the genome, where it is encoded, more specifically, where it is encoded through Mendelian inheritance. However, alternatively, the cell as described herein is a transgenic cell.

Preferably, gene expression is tested at least in duplicate samples, preferably at least in triplicate samples. In some cases, the cells being subjected to the testing are destroyed, exposed to non-sterile conditions or otherwise damaged as part of the analysis and cannot be used for culturing; nevertheless, the information obtained from these destroyed, exposed or otherwise damaged cells is still valuable, particularly when the cells originate from a homogeneous cell population; the information so obtained, particularly the gene expression information, may reasonably be assumed to apply not only to the destroyed, exposed or otherwise damaged cell analyzed, but also to further cells in the population from which the respective analyzed cell originates. It can be assumed to apply both to the cell population as such, or to any individual cell comprised in such cell population. Homogeneous in this context means that substantially all cells are much alike or similar.

A sample may consist of a single cell, or it may alternatively contain multiple cells. There is no strict upper limit in the total number of cells comprised in the sample; however the number is typically much smaller than the total number of cells comprised in a cell population; for example it is 1/100 or less, 1/100 or less, 1/1.000 or less, 1/10.000 or less, 1/100.000 or less, 1/1.000.000 or less, with respect to the amount of cells comprised in a cell population.

Very generally speaking, the expression of a gene may be tested directly, such as typically by molecular biology methods including the determination of presence and, if necessary or desired, levels of a gene product, such as particular messenger RNA (mRNA) or a precursor or degradation product thereof; or, alternatively, the expression of a gene may be tested indirectly, such as typically by biochemical methods including the determination of presence and, if necessary or desired, levels of a protein encoded by a particular gene, such as a particular cell surface protein or a precursor or degradation product thereof. Suitable surface proteins for the characterization of the cell as described herein include particularly clusters of differentiation (CD).

Any known method for testing the expression of a gene may also be used in the description herein. Suitable methods include those selected from the list comprising but not limited to gene expression profiling, polymerase chain reaction, such PCR, such as preferably quantitative PCR (qPCR), including RT-qPCR, sequencing of transcription products, any type of transcriptome analysis, and others. On nucleic acid level, preferred methods include microarrays and qPCR.

Also described, the gene expression is determined by gene expression profiling. In the field of molecular biology, gene expression profiling refers to the measurement of the expression of several, often thousands, of genes, typically all at once, to create a global picture of cellular gene expression. For example, gene expression profiling is the measurement of the expression of several, typically but not necessarily thousands, of genes at once, to create a global picture of a cell. Gene expression profiling can, for example, distinguish between cells as described herein and other cells. It is even possible to determine expression of an entire genome simultaneously, that is, every gene present in a particular cell. Many experiments of this sort measure an entire genome simultaneously, that is, every gene present in a particular cell. Several transcriptomics technologies can be used to generate the necessary data to analyze. Microarrays are preferred sometimes in the description herein. Microarrays determine the relative expression of genes, including one or more genes of interest, such as, e.g. APCDD1. Without wishing to be bound to any particular theory, it is understood that, presently, gene expression profiling provides the most global possible picture of gene expression in a single experiment.

In general, a microarray is a suitable method for gene expression profiling. Indeed, a microarray is preferably used for that purpose in the description herein. As is commonly known, a microarray shows whether or not certain specific genes are differently expressed in a cell, compared to e.g. a cell prepared as described in the prior art. Microarrays are commercially available and may be used in the context in the context of the description herein.

Also described, gene expression is determined in a high-throughput experiment. Alternatively, gene expression is determined in an experiment that is not a high-throughput experiment, such as e.g. a specific experiment directed at one specific gene. Also described, gene expression is determined both in a high-throughput experiment and in an experiment which is not a high-throughput experiment; in such case, the high-throughput is typically conducted first; and the experiment which is not a high-throughput experiment is conducted subsequently is conducted at a later stage, e.g. to verify the finding of the high-throughput experiment. For example, a microarray (high-throughput) may be followed, e.g. for the purpose of validation or confirmation of expression of specific genes, by qPCR (not high throughput).

Sequence based techniques, including high-throughput sequencing, like Next Generation Sequencing (NGS), can be used to determine gene expression in the description herein. For example, RNA-Seq (RNA sequencing), also called whole transcriptome shotgun sequencing, can provide information on the sequences of genes in addition to their expression level. RNA-Seq uses next-generation sequencing (NGS) to reveal the presence and quantity of RNA in a biological sample at a given moment in time. Also described, gene expression is determined in a sequence-based technique, like RNA-Seq or PCR and any variation thereof.

The expression of some or all genes, e.g. as revealed by gene expression profiling, such as in microarray, can then be verified by quantitative RT PCR (qRT PCR).

As is commonly known, a quantitative polymerase chain reaction (qPCR, also known as real-time polymerase chain reaction (Real-Time PCR), is a laboratory method based on the polymerase chain reaction (PCR), which is suitable for determining the amount of a nucleic acid. In particular, for determining the amount of a transcript (e.g. mRNA), the method of choice is quantitative reverse transcription polymerase chain reaction (qRT-PCR). Compared to other RNA quantification methods, such as northern blot, qRT-PCR is normally considered to be the most powerful, sensitive, and quantitative assay for the detection of RNA levels, and it is therefore preferred in the description herein. qRTPCR is also much more sensitive, for determination of gene expression, than indirect determination of the gene expression product on protein level, such as immunodetection, e.g by immunodecoration with fluorescent primary or secondary antibodies and flow cytometry. qRT PCR typically requires specific primers for the respective nucleic acid, the sequence of which is, however, normally available through sequence databases, e.g. for human genes, or can be designed by the skilled person based on such information. Commercial kits and machines are available for qRT-PCR, and these can be used in the description herein. The nucleic acid which serves as template (also termed "target") for determination of gene expression in qRT PCR is a transcription product, typically mRNA.

qPCR is normally run for a total of 35 to 45 consecutive cycles, more preferably 38 to 42 cycles, and most preferably 40 cycles. When no fluorescence is observed even after completion of the total number of cycles, then it is concluded that the target nucleic acid is absent in the sample analyzed.

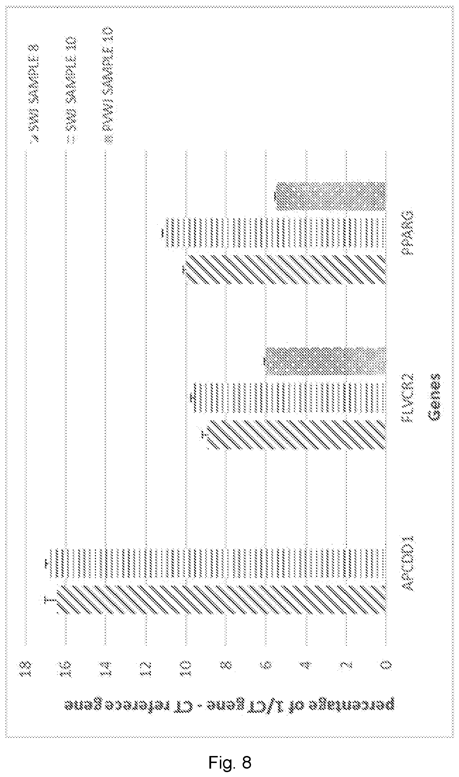

Suitably, gene expression is determined by qPCR (preferably qRT-PCR), whereby the expression levels of a gene of interest are determined, and preferably compared to the expression levels of a housekeeping gene. To that end, the preferred housekeeping gene is beta-actin (see e.g. Fig. 8).

As is standard in the art, gene expression patterns as determined by qRT-PCR are indicated by Ct and dCt, respectively: In general, in qPCR, the Ct (cycle threshold) is defined as the number of cycles required for the fluorescent signal to cross the threshold (i.e. to exceed background level). In general, the Ct value is inversely proportional to the amount of the respective (target) nucleic acid in the sample (i.e. the lower the Ct value, the greater the amount of the respective (target) nucleic acid in the sample). Ct is specific to the expression of one gene. Delta Ct (dCt) indicates the difference of expression between 2 genes, normally between a gene of interest and a housekeeping gene, such as beta-actin (see e.g. Fig. 8).

Preferably the cell as described herein expresses at least one gene specific for mesenchymal stromal cells.

The cell as described herein is characterized inter alia in that it expresses APCDD1. In particular, the cell expresses APCDD1. The inventors found that genes specific for mesenchymal stromal cells as described herein are comprised in the list comprising but not limited to APCDD1 and PPARG, and optionally FLVCR2. More preferably, said at least one gene specific for stromal cells is expressed at high levels, compared to the housekeeping gene beta-actin, relative to the expression ratio or said at least one gene vs. beta-actin in reference cells. An example of this phenotype is shown in Example 8.

While earlier literature has been uncertain on whether or not nonperivascular MSCs are distinct from perivascular MSCs (e.g. Davies et al., Stem Cells Translational Medicine, 2017, vol. 6, p. 1620-1630), the description herein provides evidence (e.g. Example 8) that stromal Wharton's Jelly derived cells obtained according to the present invention are distinct from perivascular MSCs.

Also described, gene expression is determined indirectly, i.e. not by determination of the primary transcription product, the mRNA or its precursor or its degradation product, but by determination of a specific product further downstream. Typically, the specific product further downstream is a protein encoded by the mRNA; however, it may also be any other specific product, such as e.g. a metabolite which is specific to the presence of a particular, e.g. enzymatically active, protein.

Preferably, the presence of a protein is determined. More preferably, the presence of a protein on the cell surface is determined. In other words, it is determined whether a protein is displayed on the cell surface.

Cells displaying a particular protein on the cell surface can be analyzed e.g. by immunologically active molecules, such as specific antibodies and other immunoreactive molecules. "Cell surface" is used herein in accordance with its normal meaning in the art, and thus specifically includes the outside of the cell which is accessible to binding by proteins and other molecules. A protein is displayed on the surface of cell if it is at least partially located at the surface of said cell and is accessible to binding by antigen-binding molecules such as antigen-specific antibodies added to the cell. Also described, a protein displayed on the surface of cell is an integral membrane protein having an extracellular portion that can be recognized by an antibody. The term "extracellular portion" or "exodomain" in the context of the description herein means a part of a molecule, particularly a protein, that faces the extracellular space of a cell and preferably is accessible from the outside of said cell, e.g., by binding molecules such as antibodies located outside the cell. Preferably, the term refers to one or more extracellular loops or domains or a fragment thereof. The term "portion" is used herein and refer to a continuous or discontinuous element of a structure such as an amino acid sequence. A portion or part of a protein sequence preferably comprises at least 5, in particular at least 8, at least 12, at least 15, at least 20, at least 30, at least 50, or at least 100 consecutive and/or non-consecutive amino acids of the amino acid sequence making up the protein.

A protein detectable by an antibody or other immunoreactive molecule may also be referred to as an antigen. Sometimes, the cell as described herein may be characterized by displaying - or not displaying - one or more specific antigens. In the context of the description herein, such antigen is preferably displayed on the surface of the cell. Such an antigen can also be referred to as "surface antigen". Examples thereof are provided in Example 7.

In line with the general principles of cell biology, when an antigen is displayed on the surface of the cell, then the gene encoding the antigen is expressed by the cell. Therefore, detection of an antigen that is displayed on the surface of the cell is an indirect means for showing that the gene encoding the antigen is expressed. For example, when it is said herein that the cell expresses a certain gene, which encodes an antigen expressed on the surface of the cell, then the expression of said gene may either be tested directly at the level of the gene expression product (such as mRNA), or indirectly at the level of the protein displayed on the surface of the cell. Although the word "express", in the strict sense" means that a gene is expressed, the word "express" can also mean to describe that a protein, particularly a cell surface protein, which is encoded by said gene, is displayed on the cell.

Also described, an antigen is displayed on a cell if the level of expression is above the detection limit and/ or if the level of expression is high enough to allow binding by antigen-specific antibodies added to the cell. Also described, an antigen is said to be not expressed on a cell if the level of expression is below the detection limit and/or if the level of expression is too low to allow binding by antigen-specific antibodies added to the cell. Preferably, an antigen expressed in a cell is expressed or exposed, i.e. is present, on the surface of said cell and, thus, available for binding by antigen-specific molecules such as antibodies or other immune reactive molecule added to the cell. In some cases, a secondary molecule that aids in the detection, such as e.g. an optionally labelled secondary antibody, is also added.

An antibody or other immune reactive molecule may recognize an epitope on the cell. The term "epitope" refers to an antigenic determinant in a molecule such as an antigen, i.e., to a part in or fragment of the molecule that is recognized, i.e. bound, by the immune system, for example, that is recognized by an antibody or other immunoreactive molecule. Detection of an epitope specific for any particular antigen normally allows to conclude that that particular antigen is expressed on the cell being analyzed.

Also described, the cell as described herein can be characterized by immunophenotyping. "immunophenotyping" generally means that the cell can be characterized by antigen-specific molecules such as antibodies or other immune reactive molecules, which are added to the cell to determine if an antigen is expressed on a cell. Immunophenotyping includes cell sorting using various methods including flow cytometry.

A preferred method for immunophenotyping is flow cytometry, in particular FACS. an analyte, in particular a cell surface protein, is recognized, normally with an antibody or other immunorective molecule. The antibody or other immunorective molecule is either fluorophore-labelled itself, or recognized by a fluorophore-labelled secondary antibody or other immunorective molecule, which is added for that purpose.

Preferred analytes suitable for immunophenotyping in the context of the description herein are molecules belonging to the cluster of differentiation (also known as cluster of designation or classification determinant and herein abbreviated as CD). The CD is a term used for the identification and investigation of cell surface molecules by immunophenotyping of cells. Some examples of clusters of differentiation suitable for characterizing versions of the cell as described herein are described herein. Unless the context dictates otherwise, when reference is made to expression of any Cluster of Differentiation (CD) molecule, it is intended that expression of the respective CD molecule is preferably determined by immunophenotyping; however, determination on the nucleic acid level is also possible. Herein, reference to a specific CD molecules, with reference to human cells, is intended to mean the respective specific CD molecule in accordance with the definitions assigned by the Human Leukocyte Differentiation Antigen Workshops I-IX. Example 7 provides an exemplary and useful description for immunophenotyping as described herein.

On the other hand, unless the context dictates otherwise, when reference is made in the present disclosure to expression of any gene which is not a Cluster of Differentiation (CD) molecule or nucleic acid encoding the same, it is intended that expression of the respective gene is preferably determined on the nucleic acid level, preferably by a method mentioned or described above.

First, the MSC as described herein is characterized in that it (a) expresses APCDD1. Expression of APCDD1 is preferably detected on gene expression level, most preferably by qRT PCR. An example thereof is shown in Fig. 8.

Without wishing to be bound to any particular theory, it is believed that APCDD1 is important for the cell's self-renewal capacity: the gene product was previously shown to maintain high beta-catenin levels, and beta-catenin up keeps up the stemness of cells, particular MSCs (Viale-Bouroncle et al., Biochem. Biophys. Res. Commun., 2015 vol. 457, p. 314-317; Yu, Cell Transplant., 2017, vol. 26, p. 365-377). As shown in Example 8, the cells as described herein are distinguished from PVWJ-derived cells *inter alia* by the expression of APCDD1. Thus, the APCDD1-expressing cell as described herein is clearly different from MSCs described by the prior art, see particularly WO 2004/072273 A and Sarugaser et al., 2005, Stem Cells, vol. 23, p. 220-229.

The MSC as described herein does, however, not express 3G5. 3G5 is a marker for perivascular cells. Thus, the cell as described herein is not a perivascular cell. Therefore, preferably, the cell as described herein does not express any marker for a perivascular cell which is not at the same time also a marker for a mesenchymal cell and/or a stem cell.

Preferably, the cell expresses at least one gene specific for stromal cells, more particularly for mesenchymal stromal cells. Preferably, the cell displays at least one surface marker specific for stromal cells, more particularly for mesenchymal stromal cells, on its surface.

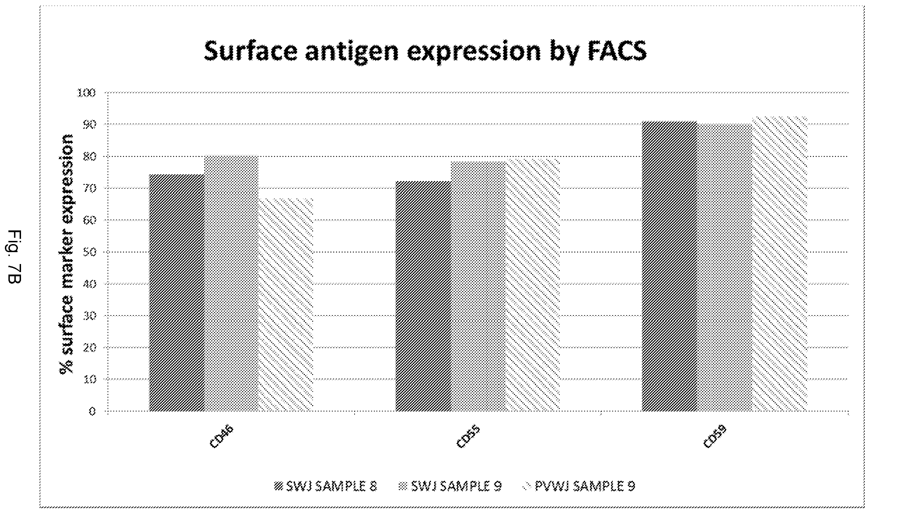

Also described, the at least one surface marker specific for stromal cells, more particularly for mesenchymal stromal cells, is selected from the list comprising but not limited to membrane cofactor protein (CD46), decay accelerating factor or CD55, and MAC inhibitory protein (CD59). All three markers have previously been described to be related to the complement cascade. As shown in Fig. 7B, cells exemplified in the present description display these markers on their surface. Without wishing to be bound to any particular theory, it is believed that one or more of these surface markers play a role in protecting the cells from complement-mediated cell lysis *in vivo.* Preferably, the cell expresses at least one of CD46, CD55, and CD59, more preferably at least any two of CD46, CD55, and CD59, and most preferably all of CD46, CD55, and CD59. To the knowledge of the inventors, CD46, CD55, and CD59 have not previously described in specific UC-derived MSCs, although it can of course not be excluded that some cells do actually express one or more of these surface molecules.

Preferably, the cell as described herein expresses CD73. CD73 is also known as ecto 5' nucleotidase and is a marker for mesenchymal stromal cells (MSCs), see Dominici et al., 2006 (Cytotherapy, vol. 8, p. 315-317).

Preferably, the cell as described herein expresses CD105. CD105 (also known as endoglin) is a further marker for mesenchymal stromal cells (MSCs), see Dominici et al., 2006 (Cytotherapy, vol. 8, p. 315-317).

Preferably, the cell as described herein expresses CD90. CD90 (also known as Thy-1) is a further marker for mesenchymal stromal cells (MSCs), see Dominici et al., 2006 (Cytotherapy, vol. 8, p. 315-317).

In a much preferred description, the cell as described herein expresses both CD105 and CD73. In addition to the general methods described above, these clusters of differentiation can be detected e.g. by the SH2 and SH3 antibodies (Horwitz et al., Curr. Opin. Hematol., 2006, vol. 13, p. 419-425). Normally, a mesenchymal stromal cell as described herein expresses both CD105 and CD73, and preferably also CD90. Thus, the expression of CD105 and/or CD73 and/or CD73 may be suitable to distinguish the cell as described herein from some other cells. More preferably, the cell expresses, in addition to any one or more of CD105 and/or CD73 and/or CD73, also at least one of CD46, CD55, and CD59, more preferably at least any two of CD46, CD55, and CD59, and most preferably all of CD46, CD55, and CD59.

In a preferred description, the cell further expresses PPARG. An example thereof is shown in Fig. 8. Without wishing to be bound to any particular theory, it is believed that the product of the PPARG gene is capable to keep cells, in particular MSCs, at a multipotent phenotype, in particular to prevent differentiation into bone tissue. For details, see e.g. Xu et al. (Curr. Stem Cell Res. Ther., 2016, vol. 11, p. 247-254).

In another and not mutually exclusive preferred description, the cell further expresses the FLVCR2 gene. An example thereof is shown in Fig. 8. The abbreviation FLVCR2 stands for Feline leukemia virus subgroup C cellular receptor family, member 2. The encoded FLVCR2 protein is a transmembrane protein from the member of the major facilitator superfamily which functions as a calcium transporter. In living beings, the encoded protein has previously been described to potentially play a role in development of brain vascular endothelial cells.

In a preferred description, the cell does not express CD117. CD117 is a cell surface marker specific for certain types of hematopoietic (blood) progenitors, such as in the bone marrow. The cell as described herein is not a blood cell, and is preferably also not a bone marrow cell. CD117 has also been associated with some types of cancer; however, the cell as described herein is not normally a cancer cell. In other words, oncogenic cells are not referred to herein as cells as described herein.

The cell as described herein preferably expresses MHC I. In the case of a human mesenchymal stromal cell it is preferred that the human cell expresses at least one, preferably at least any combination of two, and most preferably all of the human leukocyte antigens HLA-A, HLA-B and HLA-C.

Sometimes, the cell as described herein does not expresses MHC II, or expresses MHC II at low levels. "low levels" is a relative expression which indicates that the expression levels are lower than in antigen-presenting cells from the same individual, e.g. dendritic cells, mononuclear phagocytes and B cells from the same individual. Also described, the cell does substantially not express MHC II. In particular, in the case of a human mesenchymal stromal cell, it is preferred that the cell does not express any one or more of the human leukocyte antigens HLA-DP, and /or HLA-DQ. Optionally also HLA-DR is not expressed; alternatively, HLA-DR is expressed at low levels. The absence of expression of these human leukocyte antigens (HLA), or expression at low levels, causes the cell as described herein to be immunoprivileged, allowing e.g. for co-culture with cells from HLA mismatched individuals, and even cells from other species, including immune system cells. Expression of these human leukocyte antigens can be tested e.g. by surface staining with specific antibodies, or by qRT PCR.

Also described, the cell as described herein is negative for the surface markers CD34 and/or CD45. This means that the cell does not display CD34 and/or CD45.

Also described, the cell as described herein produces prostaglandin E2 (PGE2). As is commonly known, Prostaglandin E2 (PGE2), also known as dinoprostone, is a naturally occurring prostaglandin which has been described as an activator of the Wnt signaling pathway (Goessling et al., Cell, 2009, vol. 136, p. 1136-1147). Several commercial products, in particular ELISA kits, are available for determination of PGE2 production. The detection can be performed using either cell supernatant or cell lysate, or a combination thereof, as a sample.

Also described, the cell as described herein expresses one or more stem cell markers. Such stem cell markers may include typically markers selected from but not limited to Oct-4 and Nanog.

Cell Size

Besides the other features and descriptions described herein, the cell as described herein is preferably also characterized by a relatively small size. The size can be described, for example, by the cell volume, the cell diameter or the cell surface area, or by a parameter that correlates to the cell volume, to the cell diameter or to the cell surface area. The size may also be described by a combination of any of the foregoing. "relatively" means in comparison to other cells, particularly in comparison to mesenchymal stromal cells from other sources and/or other isolated by other methods. "other sources" means other anatomical areas, such as, e.g., other organs, e.g. bone marrow, but also other sections of the umbilical cord, such as the perivascular zone, and from any perivascular Wharton's Jelly.

Without wishing to be bound by any particular theory, it is believed that, sometimes, smaller cells (e.g. cells with a smaller diameter and/or cells with a smaller surface area) are earlier in the cell cycle. Methods for determination of the cell cycle state of MSCs have been published (e.g. Achille et al., J. Cell. Biochem., 2011, vol. 112, p. 1817-1821) and may be applied to the cells as described herein, e.g. for analytical purposes.

The forward-scattered light (FSC) value determined for an individual cell by flow cytometry correlates to the cell volume. Larger cells, i.e. particularly those with a larger cell volume, are characterized by higher FSC values, and *vice versa.* As is generally known, the FSC value is a dimensionless number. For determination of the FSC, one or more cells are analyzed by flow cytometry. In general, FSC values, determined by flow cytometry, depend on the instrument used, in particular the laser characteristics and the power of the machine-to-machine photomultipliers. Consequently, there is no absolute FSC value, the value typically depends the machine and laser used. Therefore, to ensure reproducibility, the following details, in combination, are preferential in the context of the description herein.

When the FSC value is determined by flow cytometry, the excitation wavelength is preferably in the range of 470 to 500 nm, most preferably 488 nm, and the forward-scattered light (FSC) is determined. It is not necessary that the cell itself be fluorescent for determination of the FSC. For such determination by flow cytometry, a commercial flow cytometry apparatus is typically used. In a preferred description, the FACSAria III flow cytometer (BD Biosciences) is used, with excitation wavelength 488 nm. The specific numerical FSC values specified in the following preferably all refer to said conditions, i.e. said cytometer and said wavelength. Preferably, the cell as described herein is characterized by a FSC value of 60 or less, such as 59 or less, such as 58 or less, such as 57 or less, such as 56 or less, such as 55 or less, such as 54 or less, such as 53 or less, such as 52 or less, such as 51 or less, or even 50 or less. Sometimes, the cell as described herein is characterized by a FSC value of 32 to 58, such as 33 to 57, such as 34 to 56, such as 35 to 55, such as 36 to 54, such as 37 to 53, such as 38 to 52, such as 39 to 51, such as 40 to 50, such as 41 to 49, such as 42 to 48, such as 43 to 47, such as 44 to 46. In a preferred description, the FSC value is about 45. These specific numerical FSC values refer to determination by the FACSAria III flow cytometer under the conditions described above.

Preferably, the cell as described herein is characterized by a FSC value which is smaller than the FSC value of a PVWJ-derived cell. Preferably, the FSC value of the cell as described herein is significantly smaller than the FSC value of a PVWJ-derived cell, see e.g. Example 4.

Preferably, the cell as described herein has a cell volume smaller than the volume of a PVWJ-derived cell. More preferably, the cell as described herein has a cell volume significantly smaller than the volume of a PVWJ-derived cell. The cell volume can be indirectly determined by determination of the FSC value by flow cytometry (for details on determination of the FSC value see above). Then, for comparative purposes, a bead of known size is optionally subjected to flow cytometry under identical conditions; thereby one can determine the volume of a cell based on the experimentally determined FSC value for the cell and the experimentally determined FSC value for the bead, and the known volume of the bead. The bead used preferably has a refractive index similar or essentially identical to the refractive index of the MSC. Commercial software is available for calculation of the cell volume based on the experimentally determined FSC. Preferred tools for determining the cell volume are FACS ARIA III and FACS DIVA (both Becton Dickinson, Franklin Lakes, NJ, USA). The data analysis is done with FACS DIVA version 6.0 software that is normally associated with the FACS ARIA III. FACS DIVA standard configuration is used. For comparing multiple samples, or cells, based on FSC values, it is necessary to always work on the same instrument and always with the same settings for the FSC photomultipliers.

The FSC and thus the cell volume can be determined both for freshly isolated cells as well as for cultured and enzymatically detached - e.g. trypsinized - cells. However, the FSC and the cell volume are preferably determined for non-adherent cells. For flow cytometry, any adherent cell must be detached from the culture vessel surface to which it adheres, prior to determination of the cell volume, typically by enzymatic treatment, more particularly by trypsinization. More specifically, the FSC is typically determined by subjecting non-adherent cells to flow cytometry, and determination of the Forward Scattered light (FSC). Commercial software is available for determination of the cell volume based on the experimentally determined FSC. A preferred commercial software for cell volume determination according to the description herein is the BD FACSDiva software (Becton Dickinson, Franklin Lakes, NJ, USA).

Although the inventors have shown that cells as described herein are relatively homogenous, there is typically some variation, as with all biological products, also with respect to cell volume. Therefore, the cell volume can also be described as the average cell volume. In a population of multiple cells, the average cell volume is preferably a cell volume that corresponds to the cell volume of cells obtained as described in Example 1 and Example 2A, for experimental determination of FSC. Example 4 describes by means of example how the FSC of a multitude of cells is determined by flow cytometry and forward scattering. The "average volume" represents the arithmetic mean of the volume of individual cells, as experimentally determined. Typically "average volume" represents the arithmetic mean of the volumes of at least 1,000 individual events, such as at least 5,000 individual events, such as at least 10,000 individual events, sometimes 100 to 1,000,00000 individual events. Each event determined by flow cytometry normally corresponds to one cell. Since cell volume determination by flow cytometry is a high throughput method, the average of such numbers of individual cells can normally be determined without unreasonable time and effort.

Also described, the cell volume is not itself determined, but the FSC value is determined. Normally, when a given cell has a FSC value lower than the FSC value of a reference cell, then the given cell will also have a smaller cell volume than the reference cell; therefore, determination of the FSC allows an indirect conclusion on the volume of a cell.

Preferably, the cell as described herein has a surface area of not more than 3,500 $\mu m^2$. Preferably the cell has a surface area of not more than 3,000 $\mu m^2$. Preferably the cell has a surface area of not more than 2,500 $\mu m^2$. Preferably the cell has a surface area of not more than 2,100 $\mu m^2$. Sometimes, the cell has a surface area of not more than 2,000 $\mu m^2$. "surface area" refers to the 2-dimensional surface area of a cell.

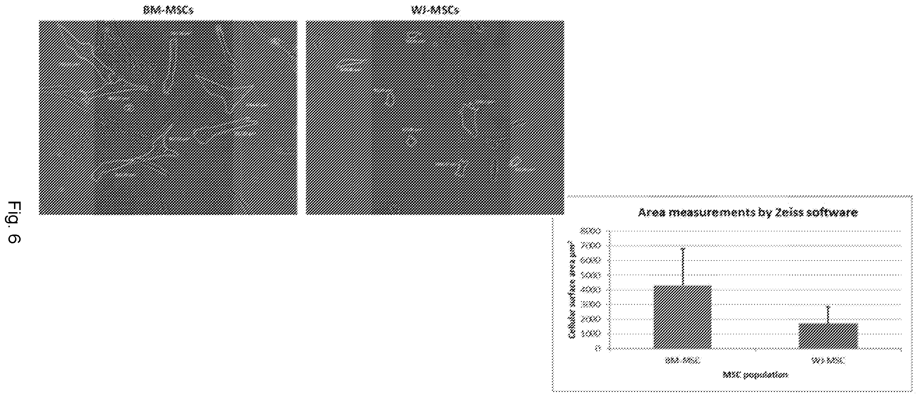

In the context of the present disclosure, the 2-dimensional surface area of a cell is determined while the cell is in adherent culture, i.e. the cell adheres to the culture plate. It is important that cells are in a low-confluence state. Sub-confluence is considered advantageous for reproducibility of determination because it is believed that sub-confluent cells do substantially not inhibit each other by contact inhibition. For determination of the surface area, one or more cells are photographed or otherwise visually observed or recorded, typically with a microscope, from the dimension perpendicular (i.e. 90 °) to the surface of the culture plate to which the cell or cells adhere. The boundaries of the area are typically manually defined on a graphical image (i.e. photograph) of the cell. Agitation of the plates prior to taking said graphical image is preferably avoided, or kept at a minimum. Methods and software for determining the surface area are not particularly limited, and respective software is available from several commercial suppliers, e.g. Carl Zeiss (Jena, Germany). For the determination in Fig. 6, Axiovision LE 4.8 from Carl Zeiss (Jena, Germany) was used. By means of example, methods for determining the surface area of cells grown in a culture dish are described by Agley et al., 2012, J. Histochem. Cytochem., vol. 60, p. 428-438, among others.

Although the inventors have shown that cells as described herein are relatively homogenous, there is typically some variation, as with all biological products, also with respect to cell surface area. Therefore, the cell surface can also be described as the average cell surface area. In such case, preferably the cell has an average surface area of not more than 3000 $\mu m^2$. Preferably the cell has an average surface area of not more than 2500 $\mu m^2$. Preferably the cell has an average surface area of not more than 2100 $\mu m^2$. Sometimes, the cell has a surface area of not more than 2000 $\mu m^2$. In a population of multiple cells, the average surface area is preferably within the constraints given here. The "average area" represents the arithmetic mean of the areas of individual cells, as experimentally determined. Typically "average area" represents the arithmetic mean of the areas of at least 35 individual cells, such as at least 50 individual cells, such as at least 100 individual cells, in some descriptions 35 to 200 individual cells.

As shown in Fig. 6, the cell as described herein preferably has a surface area which is significantly smaller than bone marrow-derived MSCs. Sometimes, the cell as described herein also has a surface area which is smaller than the surface area of perivascular Wharton's Jelly (PVWJ)-derived mesenchymal stromal cells, such as those described in WO 2004/072273 A and Sarugaser et al., 2005, Stem Cells, vol. 23, p. 220-229. Perivascular Wharton's Jelly (PVWJ)-derived mesenchymal stromal cells can also be reffered to as "perivascular zone-derived mesenchymal stromal cells".

Preferably, average volume and/or the surface area of the cells as described herein is smaller than the average volume and/or surface area of bone-marrow-derived MSCs (Fig. 6) and smaller than umbilical cord-derived MSCs from the perivascular area (Fig. 5B). In the literature, smaller cell volume of MSCs has been considered advantageous for a variety of reasons (see e.g. Ge et al., Stem Cell Rev., 2014, vol. 10, p. 295-303). Through the description herein, cells with such advantageous properties now become reliably available.

Preferably, the internal complexity of the cell as described herein is lower than the internal complexity of a PVWJ-derived MSC. Internal complexity is typically determined by side scatter analysis (SSC). In general, cells with a more complex internal structure appear more shiny/brighter in the side scatter analysis, owing to the internal structures, see e.g. Example 4A. Without wishing to be bound to any particular theory, a low internal complexity may be associated with stemness. For comparing multiple samples based on SSC values, it is necessary to always work on the same instrument and always with the same settings for the FSC and SSC photomultipliers because SSC values, like and SSC values, determined by flow cytometry, depend on the instrument used, in particular the laser characteristics and the power of the machine-to-machine photomultipliers. In the description herein the flow cytometry machine, laser and settings and software for used for determination of SSC values are preferably the same as those described above for determination of FSC values.

Also described, SWJ-derived cells as described herein are characterized by a lower internal complexity (shown by SSC) than PVWJ-derived cells.

Preferably, said SWJ-derived cells are characterized by both a smaller cell volume (shown by FSC) and a lower internal complexity (shown by SSC), than PVWJ-derived cells. PVWJ-derived cells are obtainable e.g. as described in the Comparative Examples of this disclosure.

Cell Biological Properties: Differentiation Potential, Senescence and Immortality

In general, differentiation potential, senescence and immortality can be referred to together as "cell biological properties". Needless to say, the cell as described herein has additional cell biological properties, which can be analyzed experimentally by the person skilled in the art. The term "cell biological properties", as used herein, is therefore an open term which also includes additional properties of a cell which can be determined by a biological assay, even if those additional properties are not explicitly specified herein.

The cell as described herein is a mesenchymal cell, particularly a mesenchymal stromal cell (MSC). Said MSC is normally clonogenic, unless the context dictates otherwise. Thus, even a single cell as described herein is a colony forming unit (CFU) which can give rise to a multitude of daughter cells.

The cell as described herein may also be referred to as "stromal progenitor cell". In general, a stromal progenitor cell is typically capable of giving rise to phenotypically and genotypically identical daughters (self-renewal) as well as to at least one other final cell type. Also described the cell as described herein is not lineage-committed.

Thus, preferably, the cell is not terminally differentiated. Also described, the cell is not further differentiated, beyond being a mesenchymal cell. Preferably, the cell is capable of differentiating into various cell types. Sometimes, differentiation into one or more of these various cell types is inducible, e.g. by external stimuli and/or specific culture conditions.

Preferably, the cell is at least multipotent. Multipotency can be tested by showing that a cell is capable to differentiate into multiple, at least two, different cell types. There are no particular limitations on the assay suitable for making such showing. However, typically the showing is done *in vitro.* Thus, preferably, the MSC as described herein is a cell having multipotent differentiation capacity *in vitro.* Examples for different types of differentiation are given in Examples 12, 13 and 14. For example, the osteogenic differentiation potential may be determined as described in Example 12, although established methods may alternatively be used. The assay for osteogenic differentiation potential as described in Example 12 was specifically developed in the context of description herein. Compared to state-of-the art assays for osteogenic potential, many of which have been developed with bone marrow-derived MSCs, the assay of Example 12 allows for relatively slow expansion of the cells; this, in turn, allows the (umbilical cord-derived) cells to adhere to the culture vessel, which is normally a prerequisite for an osteogenic assay.

For example, the adipogenic differentiation potential may be determined as described in Example 13, although other established methods can also be used. For example, the chondrogenic differentiation potential may be determined as described in Example 14, although other established methods can also be used. "differentiation potential" means that a given cell is capable to differentiate into a cell of a certain lineage.

Preferably, the cell is capable to differentiate into a cell of the osteogenic lineage. Preferably, the cell is capable to differentiate into a cell of the chondrogenic lineage. Preferably, the cell is capable to differentiate into a cell of the adipogenic lineage. Preferably, the cell is capable to differentiate into a cell of the osteogenic lineage and into a cell of the chondrogenic lineage. Preferably, the cell is capable to differentiate into a cell of the osteogenic lineage and into a cell of the adipogenic lineage. Preferably, the cell is capable to differentiate into a cell of the osteogenic lineage and into a cell of the adipogenic lineage. Most preferably, the cell is capable to differentiate into a cell of the osteogenic lineage and into a cell of the chondrogenic lineage and into a cell of the adipogenic lineage. When a cell is capable to differentiate into a cell of a certain lineage, this is intended to mean that the cell will differentiate accordingly, provided it is subjected to appropriate conditions. For example, particularly suitable conditions for differentiation into a cell of the osteogenic lineage are provided in Example 12. Alternatively, a commercial kit suitable for differentiation of a mesenchymal stromal cell into a cell of the osteogenic lineage may be used, as long as the kit allows for adherence of the cell to the culture vessel, to test whether any given cell (mesenchymal stromal cell) is capable to differentiate into a cell of the osteogenic lineage; one kit described for such assay is the hMSC Mesenchymal Stem Cell Osteogenic Differentiation BulletKit™ Kit from Lonza (Wakersville, MD, USA). For example, appropriate conditions for differentiation into a cell of the chondrogenic lineage are provided in Example 13. Alternatively, a commercial kit suitable for differentiation of a mesenchymal stromal cell into a cell of the chondrogenic lineage may be used to test whether any given cell (mesenchymal stromal cell) is capable to differentiate into a cell of the chondrogenic lineage; one suitable kit is the hMSC Mesenchymal Stem Cell Chondrogenic Differentiation Kit BulletKit™ from Lonza (Wakersville, MD, USA). For example, appropriate conditions for differentiation into a cell of the chondrogenic lineage are provided in Example 14. Alternatively, a commercial kit suitable for differentiation of a mesenchymal stromal cell into a cell of the chondrogenic lineage may be used to test whether any given cell (mesenchymal stromal cell) is capable to differentiate into a cell of the chondrogenic lineage; one suitable kit is the hMSC Mesenchymal Stem Cell Chondrogenic Differentiation Kit BulletKit™ from Lonza. For example, appropriate conditions for differentiation into a cell of the adipogenic lineage are provided in Example 13. Alternatively, a commercial kit suitable for differentiation of a mesenchymal stromal cell into a cell of the adipogenic lineage may be used to test whether any given cell (mesenchymal stromal cell) is capable to differentiate into a cell of the adipogenic lineage; one suitable kit is the hMSC Mesenchymal Stem Cell Adipogenic Differentiation BulletKit™ Kit from Lonza.

Optionally, the cell is even pluripotent, however it is not totipotent. The differentiation potential may be determined by subjecting a cell to specific conditions. Transdifferentiation has been described in the art, and thus, it can be tested whether any given cell, such as a MSC, is capable to differentiate into a specific ectodermal cell or endodermal cell.

Optionally the cell has further differentiation potentials. Such further differentiation potentials are selected from the differentiation potentials typical for mesenchymal stromal cells. However, as is typical for mesenchymal stromal cells, the cell as described herein is typically not totipotent.

Preferably, the cell as described herein is a mesenchymal stem cell. Thus, therein that description, the mesenchymal stromal cell is a mesenchymal stem cell. In other words, it is preferred that the cell has stem cell-like properties. Stem-cell like properties and markers therefor are described e.g. by Melton, in Chapter 2 of Lanza and Atala (eds.), Essentials of Stem Cell, Biology, 3rd ed., Elsevier, 2014.

Preferably, the cell as described herein does not show abnormally high levels of telomerase activity. "telomerase activity" refers to the enzymatic activity of the telomerase enzyme. As is generally known, telomerase is an enzyme which lengthens telomeres in DNA strands, thereby allowing senescent cells that would otherwise become postmitotic and undergo apoptosis to exceed the Hayflick limit, and in some cases to become immortal. Alternatively, the cell as described herein shows telomerase activity, however, at low levels. Telomerase activity is typically determined by determination of the enzymatic activity; more specifically, it is typically determined by determination of the enzymatic activity of telomerase reverse transcriptase (abbreviated to TERT, or hTERT in humans), which is a catalytic subunit of the enzyme telomerase. An example thereof is given in Example 10. Telomerase activity can be determined by the TeloTAGGG Telomerase PCR ELISA PLUS kit (Roche Diagnostics), a photometric enzyme immunoassay for quantitative determination of telomerase activity. For illustration see e.g. Example 10. Telomerase activity is said to be low, if the kit is used according to the manufacturer's instructions and the analyzed cells show 10 % or less, such as preferably 5 % or less, of telomerase activity with respect to the positive control provided in said TeloTAGGG Telomerase PCR ELISA PLUS kit (considered as 100%). The percentage, i.e. 10 % or less, such as preferably 5 % or less , refers to the mean of the cells, i.e. meaning that the analyzed cells show a mean of 10 % or less, such as preferably 5 % or less, of telomerase activity with respect to said control. Several alternative reagents and kits for detecting telomerase activity are commercially available and these may alternatively be used in the context as described herein.

Alternatively, the cell as described herein does not express any telomerase. In other words, therein, the cell as described herein does not show any telomerase activity. More precisely, such a cell is characterized by absence of detectable telomerase expression. Gene expression may be detected directly, or indirectly by detecting the protein transcribed from the expression product. The absence of any detectable telomerase, either on the level of enzyme activity, protein level or gene expression level, may be suitable to specifically further characterize the cells as described herein, also with reference to other MSCs that have been described in the literature (e.g. US 2004/0136967 A1). "does not express any telomerase" means that telomerase expression cannot be detected in the cell by state of the art methods available at the effective date of this document, such as particularly qRT-PCR. Typically this means that TERT activity (hTERT activity for the case of human cells) cannot be detected. For detection of expression of a gene encoding telomerase, e.g. PCR primers specific for the hTERT gene may be used (Nekani et al., 2010, Stem Cell Res., vol. 3, p. 244-254).

When cells do not show telomerase expression and/or telomerase activity, then they cannot be maintained in culture forever; in such instances, the cell as described herein is not immortal; nevertheless, the cell as described herein is suitable for cultivation under conditions as described in the experimental examples for at least 12 passages, or an equivalent number of population doublings.

Preferably, the cell as described herein is not immortal. For example, as is shown in Example 10, the telomerase activity in cells as described herein is relatively low. The cells of Example 10 are thus considered not to be immortal.

The cell as described herein, when present in a suitable growth medium at suitable conditions, is preferably mitotically active. More preferably the cell is in the exponential growth phase, this applies when the cell in a suitable growth medium at suitable conditions. Needless to say, a frozen cell is not mitotically active; however, when frozen cells as described herein are thawed and placed in a suitable growth medium at suitable conditions, they can become mitotically active and preferably grow in the exponential growth phase. For example, when frozen cells of the Primary Cell Bank (described in detail below) are thawed and placed in a suitable growth medium at suitable conditions, they will become mitotically active and give rise to a multitude of daughter cells, which in turn may optionally be used to create a Secondary Cell Bank.

The cell as described herein is not an epithelial cell, such as an amniotic epithelial cell.

The cell as described herein is not an endothelial cell.

The cell as described herein, despite its excellent proliferative properties, is not a cancer cell.

According to the description herein, the cell described herein is not an embryonic cell. In particular, the cell described herein is not an embryonic stem cell. Damaging or destruction of embryos (including human embryos) is not foreseen in this invention and was not done when the research underlying the present invention was conducted.

Preferably, the mesenchymal stromal cell as described herein is substantially free of blood or derivatives thereof, particularly umbilical cord blood. Although some MSCs are a component of the stromal scaffold of the bone marrow, which provides physical and functional support during hematopoiesis, the cell as described herein is not a hematopoietic cell. In particular, the cell as described herein is not a blood cell. More specifically, the cell is neither a hematopoietic stem cells (HSC), nor a multipotent progenitor cell of the hematopoietic system (MPP), nor a common myeloid progenitor (CMP).

Also described, the cell as described herein is not senescent. Also described, the cell as described herein is characterized by low senescence. As is generally known, the enzyme β-galactosidase is particularly active in senescent cells, where it catalyzes the hydrolysis of β-galactosides into monosaccharides. Without wishing to be bound to any particular theory, it is commonly understood that the β-galactosidase activity is ascribed to the endogenous lysosomal beta-galactosidase specifically in senescent cells. Without wishing to be bound to any particular theory, its activity is not believed to be itself required for senescence. However, β-galactosidase activity is easy to detect in a reliable manner, both *in situ* and *in vitro.* In the present description the β-galactosidase activity is determined by methods usual in the art (see e.g. Lee et al., Aging Cell, vol. 5, p.187-195. A synonym for "senescent cell" is "aging cell".

Also described, the cell as described herein is a cell that has grown until passage P11 or less, more preferably until passage P10 or less, more preferably until passage P9 or less, more preferably until passage P8 or less, more preferably until passage P7 or less, more preferably until passage P6 or less, more preferably until passage P5 or less, and most preferably until passage P4 or less. As shown in Example 9, cells as described herein have excellent proliferative properties for many passages, and as shown in Example 11 the cells as described herein not normally senescent or only marginally senescent for many cumulative population doublings.

Also described, the cell as described herein remains multipotent over at least 16 cumulative population doublings, such as at least 18 cumulative population doublings, at least 20 cumulative population doublings, following isolation. Also described, the cell as described herein remains multipotent over at least 12 passages, following isolation.

Also described, the cell as described herein remains undifferentiated over at least 16 cumulative population doublings, following isolation. Also described, the cell as described herein remains undifferentiated over at least 12 passages, following isolation.

Cell obtainable by the method according to the invention

The cell as described herein can also be characterized by the method by which it is obtainable. This is possible because the cell as described herein has properties that are different from cells obtained according to previously described methods (see examples and comparative examples herein) and thus, the method for obtaining the cell according to the present invention renders a cell with unique properties. In particular, the cell as described herein can be characterized as a cell obtainable by the method according to the first aspect of the invention.

In another and not mutually exclusive description the cell as described herein is obtainable by a method according to the first aspect of the invention. Herein the method according to the first aspect of the invention contributes to the definition of the cell, by imposing specific features on the cell, such as e.g. the origin from the stromal Wharton's Jelly of the umbilical cord, and/or the viability and/or the volume and surface area associated with the isolation and expansion procedure, to name just a few features for illustrative purposes.

Preferred embodiments of the first aspect of the invention are combinable with preferences of the cell as described herein in each and every combination, unless the context dictates otherwise. Thus, the method according to the first aspect of the invention also defines, in some embodiments, the product directly obtained by the process. Sometimes, the cell as described herein can therefore be described as a cell obtainable by the method according to the first aspect of the invention. Product-by-process features resulting from the method according to the first aspect of the present invention are generally combinable with product features as described herein.

The description also pertains to a cell obtainable by the method of the invention. In that regard, the method defines and characterizes the cell obtained thereby. Preferably, the mesenchymal stromal cell is obtainable by isolation, and optionally but preferably, expansion as described herein.

Cell derived from the mesenchymal stromal cell

Further, owing to their differentiation potential, the cell as described herein may be used to produce mature cells or cell lines. A mature cell is a cell that has no stem cell-like properties. Differentiation of mesenchymal stromal cells to mature cells can be triggered by specific conditions, such as the addition of specific exogenous growth factors to the culture medium, for illustration see Examples 12, 13 and 14.

Also described, the description pertains to a cell derived from a mesenchymal cell as described herein. Also described, the derived cell is unipotent or terminally differentiated. Also described, the derived cell is not immortal. Preferred types of derived cells include cells of the osteogenic lineage, adipogenic lineage and chondrogenic lineage.

Cell Population

The description herein relates to a population of cells (cell population). The cell population comprises at least one cell as described herein.

Also described, the cell population as described herein is a cell population directly obtainable by isolation of cells from the stromal Wharton's Jelly of the umbilical cord, as described herein. Much preferably, however, the cell population as described herein is a cell population obtainable by ex *vivo* expansion of cells that had previously been isolated from the stromal Wharton's Jelly of the umbilical cord, as described herein.

Some features of the cell population as described herein, which will be described in the following, are related to physical characteristics of the cell population, in particular the cells comprised in the population. Merely for illustration, and notwithstanding the details disclosed elsewhere herein, physical characteristics include those characteristics which can be directly determined with physical methods, such as gene expression, volume or surface area, shape etc. of the cells, ratio and distribution of any of these within the cell population. Some features and descriptions of the cell population, which will be described in the following, are related to the method by which cell population is obtainable or obtained. Merely for illustration, and notwithstanding the details disclosed elsewhere herein, characteristics of the cell population related to the method by which the cell population is obtainable or obtained can include (a) the anatomical origin of the cells, in particular the umbilical cord and the stromal Wharton's Jelly as a preferred section thereof, (b) the conditions applied for isolating the cells and (c) the conditions applied for expanding the cell, whereby a cell population is obtained.

The cell population as described herein comprises a multitude of cells. Thus, the cell population comprises at least two cells, however, normally significantly more than two cells will be comprised. Although there is no strict upper limit for the number of cells comprised in the cell population, a natural upper limit is, typically, represented by the total number of cells obtainable in a full-scale run, unless more than one cord is used as starting material. A full-scale run starting with human umbilical cord and comprising step (c) and running until passage P2 typically yields at least $1 \times 10^9$ cells, more preferably at least $2 \times 10^9$ cells, more preferably at least $3 \times 10^9$ cells, more preferably at least $4 \times 10^9$ cells, more preferably at least $5 \times 10^9$ cells, more preferably at least $6 \times 10^9$ cells, more preferably at least $7 \times 10^9$ cells, more preferably at least $8 \times 10^9$ cells, more preferably at least $9 \times 10^9$ cells, and sometimes at least $10 \times 10^9$ cells. More cells can be obtained at later passages.

The cell population comprises at least one cell which expresses APCDD1. This can normally be confirmed by showing that a sample of the cell population is positive for expression of APCDD1. In other words, the cell population is characterized by expression of the APCDD1 gene. Also described, the majority of the cells in the cell population express the APCDD1 gene. Preferably, at least 80 % (by cell number), preferably at least 90 % (by cell number), more preferably at least 95 %, such as at least 96 %, at least 97 %, at least 98 %, at least 99 % (each by cell number) of the cells comprised in the cell population express APCDD1.

Optionally, the cell comprises at least one further cell, i.e. one or more additional cell which is/are distinct from the cell as described herein, in particular the cell as described herein. In particular, herein, the at least one further cell differs in at least one feature, compulsory or optional (preferred), from the cell as described herein. Preferably, however, the at least one further cell is syngenic, more preferably autologous, to the cell as described herein.

Also described, all cells of the cell population are MSCs. Cells according to this are typically obtainable by culturing beyond the initial passage ("P0"). While, sometimes, the cells originally isolated from the umbilical cord comprise MSCs as well as other cells (e.g. red blood cells), the other cells will substantially not be present beyond P0 because (a) non-adherent cells are removed together with the growth medium at the end of P0, (b) the growth medium used is preferably particularly suitable for expansion of MSCs, and (c) cells with proliferative properties inferior to MSCs will substantially not proliferate as well as MSCs, and thus, over the course of several cumulative Population Doublings, the cell population becomes enriched in MSCs.

Also described, the cell population comprises MSCs as well as non-MSCs. Cells according to this are typically obtainable immediately after isolation, e.g. before or during the initial passage ("P0"), and/or by combining at least one cell as described herein with at least one other cell.

Preferably, all cells in the cell population originate from the same species. More preferably, all cells in the cell population are human cells. Preferably, all cells in the cell population are syngenic with respect to each other; more preferably, all cells comprised in the cell population are autologous with respect to each other. Even more preferably, all cells in the cell population originate from the same individual. Such a cell population is obtainable by using one single umbilical cord, or a fraction thereof, as starting material.

Preferably, the cell population does not comprise MSCs originating from tissues other than the umbilical cord. More preferably, the cell population does not comprise detectable amounts of MSCs originating from umbilical cord sections other than the stromal Wharton's Jelly. A cell population complies with said feature when an endothelial cell-specific marker (e.g. 3G5) and an epithelial cell-specific marker (e.g. EpCAM) cannot be detected in the cell population.

Preferably, the cell population comprises a multitude of cells, of which at least 80 % (by cell number), preferably at least 90 % (by cell number), more preferably at least 95 %, such as at least 96 %, at least 97 %, at least 98 %, at least 99 % (each by cell number) are cells as described herein. All preferences of the cell described herein apply also to the cell population described herein. Preferably at least 70 %, preferably at least 80 %, more preferably at least 90 %, such as at least 95 %, at least 96 %, at least 97 %, at least 99 % or at least 99 %, most preferably 100 %, of the total number of cells are cells as defined herein, and optionally one or more its preferences, alone or in combination. Also described, the cell population essentially consists of cells as described herein. Also described, the cell population consists exclusively of cells as described herein.

Preferably, the population is an isolated population of cells. Also described, the cell population is located outside a human or animal body. An isolated cell population is generally a cell population outside its physiological *in vivo* environment. Preferably, the cell population is an ex *vivo* cell population. In particular, the ex *vivo* cell population is preferably located outside the umbilical cord. In particular, the ex *vivo* cell population is preferably free of blood vessels. Also described, the ex *vivo* cell population is in a culture vessel. Also described, the ex *vivo* cell population is in a storage-suitable container, such as in a vial. Also described, the ex *vivo* cell population is at a temperature of ca. 35 °C to 39 °C, preferably ca. 36 °C to 38 °C, such as ca. 37 °C. In one not mutually exclusive description, the ex *vivo* cell population is in a culture vessel, or in a storage-suitable container. Preferably, the storage-suitable container is suitable for shipping of the cell within the above temperature ranges. Also described, the ex *vivo* cell population is in a cryopreservation-suitable container, such as in a cryovial, preferably at a temperature of -180 °C or less. Also described, the ex *vivo* cell population is in a culture vessel. Also described, the ex *vivo* cell population is a metabolically active, preferably expanding, cell population.

Some of the features of the cell population can be described by physical properties. For determination of physical properties, in general, it is possible to take a sample from the cell population, and to determine the physical properties for all or part of the cells comprised in the sample of the cell population. Since populations of cells according as described herein are typically homogeneous and/or characterized in that individual cells comprised therein are similar to each other or much alike, the finding on particular properties, such as e.g. gene expression, volume or surface area, shape etc. in the cells comprised in the sample will usually also apply to the remaining cells in that population, unless the context dictates otherwise and/or it is otherwise apparent that a specific cell population comprises cells which are clearly different from each other, genotypically and/or phenotypically. Homogeneous in this context means that substantially all cells are much alike or similar.

The cell population is preferably further characterized in that all cells are characterized by essentially uniform physical characteristics. "essentially uniform" means that at least 90 % of the cells, such as at least 91 % of the cells, such as at least 92 % of the cells, such as at least 93 % of the cells, such as at least 94 % of the cells, such as at least 95 % of the cells, such as at least 96 % of the cells, such as at least 97 % of the cells, such as at least 98 % of the cells, and preferably at least 91 % of the cells, comply with one or more desired physical characteristic. It is particularly desirable that the cell volume, which is correlated to forward scattered light (FSC) during flow cytometry, as described above, is a physical characteristic, which is essentially uniform in the cell population as described herein.

Preferably the average volume of the cells comprised in the population as described herein is smaller than the average volume of PVWJ-derived MSCs. PVWJ-derived MSCs are obtainable e.g. as described in the Comparative Examples herein. Once the cell volume has been determined for an individual cell, the average cell volume can be arithmetically determined. Methods for determination of the cell volume are not particularly limited, although a FACS-based method, particularly as described above in detail for an individual cell, is preferred.

The cell population is preferably further characterized in that the volume and/or surface area of the cells in the cell population is similar, or, alternatively, in that a high percentage of the cells has a small volume and/or surface area.

Preferably, the cell population as described herein is characterized by an average FSC value of 60 or less, or preferred values within said range, as described above for an individual cell. For example, the cell population may be characterized by an average FSC value of 55 or less, such as 50 or less. In a specific description, at least 90 % of cells within the cell population are characterized by an FSC value of 45 +/- 10. In a more specific description, the FSC value of all cells of the population is 45 +/- 10. Preferably, the average FSC value of at least 90 % of cells within the cell population is about 45. More preferably, the average FSC value of all cells of the population is about 45. Preferably, the cell as described herein is characterized by a FSC value which is smaller than the FSC value of a PVWJ-derived cell.

Preferably the average surface area of the cells in the cell population is not more than 3000 $\mu$m². Preferably the cell has a surface area of not more than 2500 $\mu$m². Preferably the cell has a surface area of not more than 2100 $\mu$m². Sometimes, the cell has a surface area of not more than 2000 $\mu$m². In a population of multiple cells, the average surface area is preferably within the constraints given here. Sometimes, the average surface area of the cells in the cell population is preferably 1000 to 300 $\mu$m², preferably 1100 to 2700 $\mu$m², preferably 1200 to 2500 $\mu$m², preferably 1300 to 2200 $\mu$m², preferably 1400 to 2000 $\mu$m², preferably 1500 to 1900 $\mu$m², more preferably 1600 to 1800 $\mu$m², and most preferably ca. 1700 $\mu$m² .

Preferably the cell population is further characterized in that at least 70 % of the cells (by cell number), preferably at least 80 % (by cell number), preferably at least 90 % (by cell number), more preferably at least 95 %, such as at least 96 %, at least 97 %, at least 98 %, at least 99 % (each by cell number) are viable. "viable" designates a cell which is not apoptotic. Thus, in turn, the cell population comprises apoptotic cells in a ratio of 30 % or less of the total number of cells, preferably 20 % or less of the total number of cells, more preferably 10 % or less of the total number of cells, and most preferably 5 % or less of the total number of cells. Also described, the cell population does not comprise any apoptotic cells. A suitable marker for apoptotic cells is 7AAD (see e.g. Example 3). Also described, viability is determined immediately after isolation of the cells, i.e. prior to expansion. A high percentage of viability represents an advantage over previously described populations of MSCs.

Preferably the cell population is further characterized in that at least 60 % of the cells (by cell number), at least 70 % of the cells (by cell number), preferably at least 80 % (by cell number), preferably at least 90 % (by cell number), more preferably at least 95 %, such as at least 96 %, at least 97 %, at least 98 %, at least 99 % (each by cell number) are multipotent.

Also described, the cell population comprises mast cells in a ratio of 2 % or less of the total number of cells, preferably 1 % or less of the total number of cells. Most preferably, the cell population does not comprise mast cells.

Also described, the cell population comprises fibroblasts in a ratio of 2 % or less of the total number of cells, preferably 1 % or less of the total number of cells. Most preferably, the cell population does not comprise fibroblasts. Herein, the term "fibroblasts" is to be understood narrowly, to refer to those cells which have already differentiated into fibroblasts and to exclude those cells which are multipotent and have the mere capability to differentiate into fibroblasts.

Also described, the cell population comprises cells of the hematopoietic lineage in a ratio of 2 % or less of the total number of cells, preferably 1 % or less of the total number of cells. Most preferably, the cell population does not comprise cells of the hematopoietic lineage. In a preferred description, the cell does not comprise any blood cell, such as blood cell from the umbilical cord blood. Several markers are well established for blood cells, and therefore, compliance with this preferred description can readily be tested.

Also described, the cell population comprises perivascular cells in a ratio of 2 % or less of the total number of cells, preferably 1 % or less of the total number of cells. Most preferably, the cell population does not comprise any perivascular cell. A marker for perivascular cells is 3G5. Therefore, this can alternatively be described in that the cell population does not comprise any cell that expresses the marker 3G5 and/or any cell which displays 3G5 on its surface. Also described, the cell population is not contaminated with perivascular cells. Expression of 3G5 has also been described for bone-marrow-derived MSCs (Khan et al., J. Orthop. Res., 2010, vol. 28, p. 834-40). Therefore, non-expression of 3G5 may also be suitable to discriminate a cell population as described herein from bone-marrow derived MSC populations. Also described, the cell population is not contaminated with perivascular cells. Also described, the cell population does not comprise bone-marrow-derived MSCs.

Also described, the cell population comprises epithelial cells in a ratio of 2 % or less of the total number of cells, preferably 1 % or less of the total number of cells. Most preferably, the cell population does not comprise any epithelial cell. A marker for epithelial cells is Epithelial cell adhesion molecule (EpCAM). Therefore, this can alternatively be described in that the cell population does not comprise any cell which expresses the marker EpCAM and/or any cell which displays EpCAM on its surface. Also described, the cell population is not contaminated with epithelial cells.

Also described, the cell population comprises endothelial cells in a ratio of 2 % or less of the total number of cells, preferably 1 % or less of the total number of cells. Most preferably, the cell population does not comprise any endothelial cell. A marker for endothelial cells is CD31. Therefore, this can alternatively be described in that the cell population does not comprise any cell which expresses the marker CD31 and/or which displays CD31 on its surface. Also described, the cell population is not contaminated with endothelial cells.

Also described, the cell population comprises red blood cells in a ratio of 2 % or less of the total number of cells, preferably 1 % or less of the total number of cells. Most preferably, the cell population does not comprise any red blood cell. Markers for red blood cells are globin and BGP1. Therefore, this can alternatively be described in that the cell population does not comprise any cell which expresses the BGP1 and/or globin. Also described, the cell population is not contaminated with red blood cells.

Preferably, less than 10 % of cells comprised in the cell population show telomerase activity, more preferably less than 5 % of cells comprised in the cell population display telomerase activity, and most preferably less than 2.5 % of cells comprised in the cell population show telomerase activity. Determination of telomerase activity and aspects related thereto are described above and do also apply to this part of the description. Respective cell populations may be distinguished from other cell populations described previously (e.g. US 2004/0136967 A1).

Also described, the cell population is further characterized in that no cell contained therein, or, more specifically and preferably no MSC contained therein, displays MHC II on its surface, or expresses MHC II. Thus, preferably, no MSC of the cell population express no MHC II. Also described, MHC II expression may be suitable to distinguish the cell population as described herein from other MSC-containing cell populations (see e.g. Sarugaser et al., 2005, Stem Cells, vol. 23, p. 220-229).

Preferably, the cell population is obtainable by a method according to the first aspect of the invention, particularly when step (c), i.e. the expansion step, is comprised. Thus, the method according to the present invention, comprising step (c), can yield a population of mesenchymal stromal cells. Product-by-process features resulting from the method according to the first aspect of the present invention are generally combinable with product features according to the descriptions herein. Alternatively or additionally, the cell population as described herein can therefore be described as a cell population obtainable by the method according to the first aspect of the invention, comprising step (c).

Preferably the stromal cell population as described herein is essentially free of contaminating agents. A stromal cell population essentially free of contaminating agents is defined herein as a cell population essentially free of infecting agents such as viruses (encapsulated and non-encapsulated), parasites, bacteria and endotoxins, i.e. a cell population containing such infecting agents in a quantity that cannot be detected with culture-based, serological or molecular identification systems or with the LAL test (endotoxins).

Preferably, the cell population as described herein is essentially free of antibiotics. A cell population essentially free of antibiotics is obtainable by growing cells for at least one passage in the absence of antibiotics. For example, even if antibiotics are present in the first passage following isolation of the cells ("P0"), antibiotics are preferably discontinued for all passages after P0; in other words, for example the growth medium MSCBM CD with 5 % hPL may used without addition of antibiotics for P1 and all subsequent passages.

Also described, the ex *vivo* cell population is frozen. Frozen cells are usually storage-stable at temperatures of -180 °C or less, although any condition suitable for storing cells is suitable for this. Cells may be frozen, preferably snap-frozen in liquid nitrogen. A cryoprotectant may be added prior to freezing. Also described, the cell population is stored in liquid nitrogen. Preferably, the frozen cell population is a cell bank. This will now be described in further detail.

Cell Bank

As shown e.g. in Example 6, the cells as described herein are reliably characterized by a specific phenotype also for different isolations from individual umbilical cords, and maintain the specific phenotype along several cumulative population doublings. Thus, cells obtainable according to the invention or otherwise pertaining to the present invention reliably have a specific phenotype and maintain that phenotype during expansion over many passages. Therefore, the cells as described herein are particularly suitable for the creation of a cell bank.

Indeed, preferably, the cell population as described herein is a cell bank. Thus, the cell population as described herein may be in the form of a cell bank. The cell bank comprises SWJ-derived cells. Unless specified otherwise, the cell population comprised in the cell bank generally corresponds to the cell population described above. Preferably the cell bank comprises a multitude of cells, of which at least 70 %, preferably at least 80 %, more preferably at least 90 %, such as at least 95 %, at least 96 %, at least 97 %, at least 99 % or at least 99 %, most preferably 100 %, of the total number of cells are cells as defined herein, and optionally one or more its preferred description, alone or in combination.

The cell bank described herein is obtainable by a method comprising expanding SWJ-derived cells according to the invention. Alternatively, a cell bank of freshly isolated cells (non-expanded cells) may be maintained, from which expanded cells may optionally be gained at a later stage. Preferably, however, the cell bank comprises expanded cells, i.e. cell obtainable by the method of the invention including step (c).

The cell bank preferably comprises non-adherent cells. Such a cell bank is obtainable by a method comprising enzymatic detachment, e.g. trypsinization, of adherent cells prior to producing the cell bank. Enzymatic detachment may be performed by methods according to the state of the art.

Also described, a cell bank is generated after the cells have grown for 5 to 30 cumulative Population Doublings (not counting cumulative Population Doublings during P0), such as 8 to 25 cumulative Population Doublings (not counting cumulative Population Doublings during P0).

The cell bank is maintained, so that some or all of the cells can be withdrawn from the cell bank and/or used, e.g. for further cultivation, at a desired point in time, such as a future point on time.

When a cell population is "maintained", this can be done at any condition which does not interfere with the future culturing of the cell population; cell populations may be maintained in frozen or non-frozen form; for the cell bank as described herein, however, maintenance in frozen form is preferred.

Preferably, the cell bank as described herein is a frozen cell bank. Frozen cells are usually storage-stable at temperatures of -180 °C or less, although any condition suitable for storing cells is suitable for this. Cells may be frozen, preferably snap-frozen in liquid nitrogen. A cryoprotectant may be added prior to freezing. The cells can be stored at any suitable condition, although freezing at temperatures of -180 °C or less is most typical.

The cell bank typically contains cells contained in multiple separate aliquots, such as in multiple individual vials. Preferably, each vial comprises a suspension of cells. Any liquid which is suitable for suspending mesenchymal stromal cells, particularly growth medium, and preferably the growth medium suitable for the present invention, are suitable for resuspending the cells described herein. Preferably, the cell bank comprises a liquid comprising a basal medium for culturing mesenchymal stromal cells, such as e.g. MSCBM CD, or a basal medium substantially equivalent thereto. Preferably, the liquid in which the cells of the cell bank are suspended further comprises platelet lysate, e.g. 5 % (vol./vol.) platelet lysate (PL), and in the case of human cells, human platelet lysate (hPL).

Cell banks or aliquots thereof may be used at a future point in time, e.g. to inoculate a sub-culture, e.g. in a production bioreactor.

Typical descriptions of the cell bank as described herein include a Primary Cell Bank (PCB) and a Secondary Cell Bank (SCB). Thus, the cell bank as described herein is preferably selected from a Primary Cell Bank (PCB) and a Secondary Cell Bank (SCB).

In the broadest sense, the term "Primary Cell Bank" refers to the first cell bank, more preferably first frozen cell bank, that is being prepared following isolation of the cells from the umbilical cord.

Also described, a Primary Cell Bank is generated after the cells have grown for 5 to 14 cumulative Population Doublings (not counting cumulative Population Doublings during P0).

A Primary Cell Bank (PCB) is typically in the form of aliquots. Aliquots are prepared following the respective preceding passage (e.g. P2) and enzymatic detachment, such as e.g. trypsinization, centrifugation and resuspension. Preferably, the cells for the Primary Cell Bank are aliquoted in 100-300 vials, such as 150-200 vials, such as 160 to 180 vials, or more precisely 165 to 175 vials.

Preferably, each vial of the Primary Cell Bank comprises $1 \times 10^6$ cells to $1 \times 10^7$ cells, preferably $5 \times 10^6$

cells to 500 × 10^6 cells, preferably 10 × 10^6 cells to 100 × 10^6 cells, preferably 15 × 10^6 cells to 50 × 10^6 cells, preferably 18 × 10^6 cells to 30 × 10^6 cells, such as about 20 × 10^6 cells.

Preferably, the volume contained in each vial is between 1 and 100 ml, such as between 2 and 20 ml, preferably between 3 and 10 ml, and most preferably around 4 ml.

In one illustrative description, the Primary Cell Bank obtained after a Full Scale Run consists of 150 to 200 vials, each containing 20 × 10^6 cells, and each containing 4 ml of (frozen) liquid comprising the cells.

It is also possible to fill the suspension of cells destined for the Secondary Cell Bank in vials or containers of different size or content or with a different amount or concentration of cells. Nevertheless, the above amounts are indicative of what can typically be obtained in a full-scale run, and conversions to vials or containers of different size or content or a different amount or concentration of cells can be calculated.

In the broadest sense, the term "Secondary Cell Bank" refers to the second cell bank, more preferably second frozen cell bank, that is being prepared following isolation of the cells from the umbilical cord; in other words, the first cell bank, more preferably first frozen cell bank, that is being prepared from all or part of the cells comprised in the Primary Cell Bank.

Also described, a Secondary Cell Bank is generated after the cells have grown for 14 to 25 cumulative Population Doublings (not counting cumulative Population Doublings during P0).

Since the Primary Cell Bank comprises normally multiple aliquots of cells, which may be used at the same or different points in time for further culturing, it is possible to generate multiple Secondary Cell Banks, simultaneously or sequentially, from one Primary Cell Bank.

For example, it is possible to generate from each vial comprised in the Primary Cell Bank one Secondary Cell Bank. For example, it is possible to generate, starting from one Primary Cell Bank, 100 to 300 Secondary Cell Banks, such as 150 to 200 Secondary Cell Banks, such as 160 to 180 Secondary Cell Banks, or more precisely 165 to 175 Secondary Cell Banks.

A Secondary Cell Bank (SCB) is typically in the form of aliquots. Aliquots are prepared following the respective preceding passage (e.g. P4) and enzymatic treatment, such as e.g. trypsinization, centrifugation and resuspension. Preferably, the cells for the Secondary Cell Bank are aliquoted in 100 to 300 vials, such as 150 to 200 vials, such as 160 to 180 vials, or more precisely 165 to 175 vials.

Preferably, each vial of the Secondary Cell Bank comprises 1 × 10^6 cells to 1 × 10^7 cells, preferably 5 × 10^6 cells to 500 × 10^6 cells, preferably 10 × 10^6 cells to 100 × 10^6 cells, preferably 15 × 10^6 cells to 50 × 10^6 cells, preferably 18 × 10^6 cells to 30 × 10^6 cells, such as about 20 × 10^6 cells.

Preferably, the volume contained in each vial of the Secondary Cell Bank is between 1 and 100 ml, such as between 2 and 20 ml, preferably between 3 and 10 ml, and most preferably around 4 ml.

It is also possible to fill the cell suspension destined for the Secondary Cell Bank in vials or containers of different size or content or with a different amount or concentration of cells. Nevertheless, the above amounts are indicative of what can typically be obtained in a full-scale run, and conversions to vials or containers of different size or content or a different amount or concentration of cells can be calculated.

Industrial Applicability

Mammalian cells with proliferative properties, and respective cell populations, are valuable to non-commercial and commercial activities, such as research activities. The cells as described herein are particularly attractive for such purposes, *inter alia* in view of the good reproducibility of the method, the excellent proliferative properties and the high yield of the cells, among others. Cell populations as described herein may be suitable to manufacture cell products e.g. for research or commercial uses. For example, it is industrially feasible to prepare a Primary Cell bank or Secondary Cell Bank and to commercially offer such Cell Bank, or more typically individual aliquots therefrom, commercially for research purposes, such as e.g. *in vitro* cell-based assays.

In addition to that, it is possible to run all or part of the method of the invention in an automated manner, using machines, including robots, for execution of one or more process steps. Thus, also the method of the invention is susceptible to industrial application.

Sometimes, cells as described herein can be cultured and expanded *in vitro,* e.g. for research or production purposes. For example, the cell as described herein, or cells derived therefrom, may be used to screen for the efficacy and/or cytotoxicity and/or mechanism of action of pharmaceutical compounds and compositions and/or to study the mechanism of a certain disease and/or for *in vitro* gene transfer and/or to produce biomolecules, such as proteins including therapeutically active proteins.

The cells as described herein may be cultured as a pure culture, or co-cultured with other cell lines, primary or secondary.

Further, owing to their differentiation potential, the cells may be used to produce mature cells or cell lines. Differentiation of mesenchymal stromal cells to mature cells can be triggered by specific conditions, such as the addition of specific exogenous growth factors to the culture medium.

The following examples and figures are for illustrative purposes and are not intended to limit the invention, which is defined by the claims.

## Examples

Example 0: Materials and methods common to various aspects of the invention

In all experimental examples, unless indicated otherwise, MSCBM CD or a basal medium substantially equivalent thereto was used. Exemplary media are described in the following.

Basal medium MSCBM CD and growth media comprising the same

The basal medium MSCBM CD is commercially available from Lonza (Wakersville, MD, USA; Cat. No. 95062-688). According to the manufacturer, it is serum-free, optimized for multiple passage expansion of all types of human mesenchymal stem cells, supports multi-lineage differentiation and does not require an attachment matrix for plating of the cells. Basal media substantially equivalent to MSCBM CD are also suitable.

"MSCBM CD/GA" is prepared as follows:

- 500 ml MSCBM CD (Lonza)
- 0.5 ml GA1000 (Lonza; according to the manufacturer, GA1000 contains 30 mg/ml Gentamycin and 15 $\mu$g/ml Amphotericin)

"MSCBM CD/hPL" - unless diverging amounts or ratios are specifically indicated - contains 5 % (vol./vol.) human platelet lysate (hPL), and 2 U/ml heparin, which are both added to MSCBM CD (i.e. without the antibiotics Gentamycin and Amphotericin). The hPL is suitably prepared from a composition comprising between $3.8 \times 10^8$ platelets/ml and $1.2 \times 10^9$ platelets/ml, on average ca. $7.9 \times 10^8$ platelets/ml); such platelet lysate is obtainable e.g. from the Polyclinic Blood Bank in Modena, Italy or any other supplier.

"MSCBM CD/GA/hPL" - unless diverging amounts or ratios are specifically indicated - contains 5 % (vol./vol.) human platelet lysate (hPL), and 2 U/ml heparin, which are both added to MSCBM CD/GA (i.e. with the antibiotics Gentamycin and Amphotericin).

Growth factors (other than those eventually comprised in the hPL and/or in the basal medium) are not added, unless explicitly stated otherwise.

The above media are particularly suitable for expanding the cells as described herein.

Cell Counting

For determination of the cell number, unless indicated otherwise, cells are counted by automated Cell Counters, such as particularly the NucleoCounter®. If necessary, cells are enzymatically detached such as e.g. trypsinized, prior to cell counting. If counting of red blood cells is not desired, then the cell count is done after the cell population has been subjected to an ammonium chloride treatment; such treatment is known to lyse red blood cells. As a result, the total number of cells in a population, except red blood cells, can be determined. To that end, Ammonium Chloride Solution recommended for the lysis of red blood cells is commercially available from multiple providers (e.g. STEMCELL Technologies). Such solution can be added and used according to the manufacturer's instructions.

Example 1: Segmenting and sectioning of the umbilical cord, and obtaining minced stromal Wharton's Jelly

Obtain a human umbilical cord from a term birth. Accept the cord if the total weight is 40 g or more. Segment the cord by cutting perpendicular into ca. 2.5 cm long segments using a sharp, sterile scalpel. Rinse the cord segments with HEPES-Buffered Saline Solution (HBSS) + 300 $\mu$g/ml gentamycin and 0.15 $\mu$g/ml amphotericin B for a total of 10 min. Use mechanical shaking to wash blood off the tissue.

For each so-obtained segment, proceed to obtain the stromal Wharton's Jelly section as follows: incise the cord lengthwise and remove the vessels with the immediately surrounding matrix of typically 3 mm (vascular-perivascular zone, also referred to as vascular-perivascular Wharton's Jelly - PVWJ) and discard. The removal should be carried out with care, in the sense that opening of the vessels and/or loss of vascular or perivascular cells should be avoided. Avoid pulling out the cord matrix.

Before collecting the matrix tissue, optically re-examine each segment of tissue to ensure that PVWJ has been completely removed. No vessels or fragments of vessels should remain in the segment.

Once the PVWJ has been removed from the umbilical cord segments, snip out pieces of matrix tissue from the umbilical cord segments using sharp scissors. Scrape the inside surface of the cord segments with a sharp scalpel to obtain the remaining matrix tissue. The thin amniotic epithelial membrane remaining after scraping is very carefully removed and discarded. The sub-amniotic (or mesenchymal) layer of the cord lining is included in the WJ that is isolated. This is achieved by carefully scraping tissue off the amniotic epithelium; it is important to be careful not to include pieces of the amniotic epithelial membrane when collecting said tissue. The so-obtained tissue is the pure stromal Wharton's Jelly (SWJ).

Mince the so-obtained (snipped and scraped) SWJ finely with sharp scissors and/or scalpel. Add HEPES-Buffered Saline Solution (HBSS) to the minced tissue. Pool the minced tissue in a Petri dish.

To extract SWJ, care is taken during extraction to avoid extracting cells from UC-blood, epithelial or endothelial cells and cells from the vascular structure. For control purposes (optional but recommendable, particularly when re-working this example for the first time), a marker for endothelial cells is CD31; a marker for epithelial cells is Epithelial cell adhesion molecule (EpCAM), a marker for perivascular cells is 3G5.

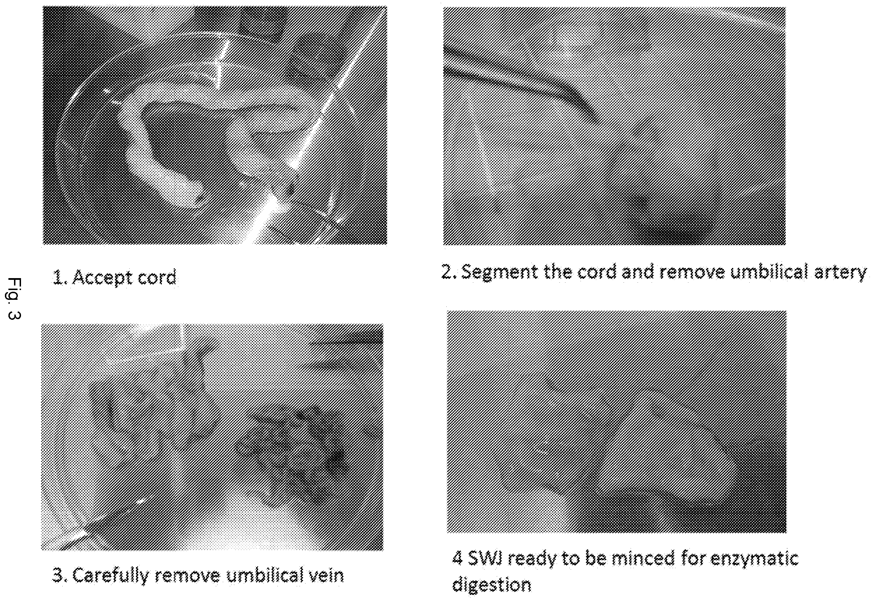

Steps of Example 1 are shown in Fig. 3.

The method of the present invention as exemplified in Example 1 yields pure Wharton's Jelly through the complete sectional removal and elimination of the non-WJ components of the cord (such as umbilical cord lining membrane/amniotic membrane, vessels and blood), avoids extracting cells of UC-blood, epithelial or endothelial cells and cells from the vascular structure. This allows to obtain pure Wharton's Jelly tissue (WJ tissue). Owing to its origin, this specific WJ tissue is stromal WJ tissue.

Example 2A: Enzymatic Treatment

Material and Methods: WJ tissue obtained as described in Example 1 was used to isolate WJ-derived primary cells therefrom, as follows: Minced tissue/HBSS mixture was suspended in Digestion Buffer (see Table) at a ratio of 100 mg of minced tissue/HBSS mixture per 1 mL of Digestion Buffer (see Table below) in a 250 ml conical tube. The digestion buffer comprises an enzyme suitable to release the MSCs from the isolated tissue.

TABLE: Formulation of 100 ml Digestion Buffer (all ingredients sterile)

- 1 ml hPL, e.g. from the Polyclinic Blood Bank, Modena, Italy (prepared from a composition comprising between $3.8 \times 10^8$ platelets/ml and $1.2 \times 10^9$ platelets/ml, on average ca. $7.9 \times 10^8$ platelets/ml)
- Heparin to a final concentration of 2 U/mL
- 1 ml sodium pyruvate (from a 100 mM stock)
- 36 $\mu$l 1M $CaCl_2$
- Collagenase preparation NB4 or NB6 (both SERVA) to a final activity of 0.18 Wünsch U/ml Digestion Buffer
- Basal medium (commercial growth medium for mammalian cells, such as DMEM or MSCBM CD) - ad 100 ml

Collagenase preparation from SERVA (NB4, NB6) was used, typically NB4. According to the manufacturer, collagenase preparation NB4/NB6 is a natural product from a collagenase-producing strain of *Clostridium histolyticum,* and is a collagenase preparation that is non-toxic, which contains collagenase and other proteases. According to the manufacturer, NB4 Standard Grade (17454.02 and 17454.01 (both SERVA)) is designed for dissociation of different tissues to isolate various cell types, e.g. chondrocytes, muscle cells, epithelial cells, skin fibroblasts, hepatocytes, adipocytes which are used for research purposes. According to the manufacturer, collagenase preparation NB4 can also be used as low cost alternative to collagenase preparation NB6 for procedure-establishing purposes. According to the manufacturer, collagenase preparation NB6 GMP Grade (NB6 GMP grade, 17454.02 (SERVA) is especially designed for the isolation of cells for some specific transplantation purposes. Throughout the experiments reported in the present disclosure, NB4 was used unless explicitly specified otherwise. According to SERVA, the manufacturer of the collagenase preparation used in this experiment, SERVA indicates collagenase activity in PZ units (PZ-U) according to Wünsch (synonymously termed Wünsch units herein).

It is known that heparin is a suitable additive for platelet lysate-containing liquid compositions to avoid clogging (e.g. Lohmann et al., 2012, PLoS ONE, vol. 7e37839).

Digestion Buffers with higher collagenase activity (final concentration higher than 0.18 Wünsch U/ml) were also tested, initially based on collagenase activity that has previously been described for enzymatic digestion of adipose tissue, but the digestion in presence of higher collagenase activity was not advantageous (data not shown).

The minced tissue (Example 1) was placed into sterile tubes. Digestion buffer (see above) was added. These tubes containing minced tissue and Digestion Buffer (digest) were incubated at 37±1°C for a digestion time interval of 3 hours with constant gentle agitation at 75 rpm on an orbital shaker for digestion, "digest" refers to the ensemble of tissue to be digested and digestion buffer.

At the end of the digestion time interval, the digest was diluted by adding 2 volumes of MSCBM CD, supplemented with 1 % (v/v) human platelet lysate (hPL) and passed through two layers of one sterile gauze pad to separate from undigested tissue/partially digested tissue. The filtrate was termed "digested mixture".

The digested mixture is centrifuged at 680 × g for 15 minutes using 250 ml centrifuge tubes. At the end of centrifugation, the supernatant is removed and the pellet (containing freshly-isolated mesenchymal stromal cells from the Wharton's Jelly of the umbilical cord) is re-suspended in 1-2 mL of MSCBM CD/GA/hPL (see Example 0).

Subsequently, the total cell number is determined. Most typically, the cells comprised in the digested mixture are not all MSCs:

Example 2B: First culturing (P0 and optionally P0+)

The re-suspended cells form Example 2A are plated in T-225 culture flasks (Falcon®, a brand of Corning, Corning, NY, USA) in MSCBM CD/GA with 5 % hPL (see Example 0; 50 mL medium per T-225 flask) to start the first *in vitro* culture of the cells originating from the stromal Wharton's Jelly. This cell culture is termed "P0". The day that P0 is initiated is termed "Day 0", and days are counted with ascending numbers thereafter. The cell density at the onset of P0 is 8.000 cells/cm$^2$ or more. The cm$^2$ refer to the surface of the flask which is at the bottom and covered by growth medium while the cells are being cultured in P0.

Feed cells every 3 days starting from Day 5 with fresh MSCBM CD/GA/hPL. Alternatively, it could also be possible to culture the cells during P0 in stacks, instead of flasks: in that case, 150 mL MSCBM CD/GA/hPL are used per 1×1 stack to start the first cell culture; however, unless explicitly mentioned otherwise, the P0 culture is performed in T-225 culture flasks.

The cells are cultured in P0 as described until the confluence of the cells is 80 to 90 %. This is usually the case after a period of 5-12 days. Then, the cells are trypsinized. Cells are counted following trypsinization at the end of the passage.

The following acceptance criteria are assessed: 1 yield, 2 viability, 3 confluence, 4 morphology. Determination of acceptance criteria 1 and 2 occurs after trypsinization of the cells. The details are as follows.

| Acceptance criterion | | After P0 and P0+ | Test |
|---|---|---|---|
| 1 | Yield | At least 8 million cells | Cell counting after trypsinization |
| 2 | Viability | More than 80 % of cells viable | Staining with 7-amino actinomycin D (7-AAD) |
| 3 | Confluence | At least 80 %, preferably 80 to 90 % confluent | Microscopic determination |
| 4 | Morphology | Majority of cells small, spindleshaped and in defined colonies | Microscopic determination |

In this experimental example, all four acceptance criteria must be fulfilled. In addition, in the morphological inspection, it is additionally visually inspected whether size and shape of the cells are relatively uniform. Preferably, this is the case.

In case any one or more of criteria 2, 3 or 4 are not fulfilled, the entire cell culture is discarded. It is the observation of the present inventors that criteria 2, 3 and 4 are normally fulfilled, and that discarding is hence not necessary. However, the possibility to discard in case of non-fulfillment guarantees that subsequent passages are only conducted if the acceptance criteria are fulfilled.

In case the total cell number after P0 is higher than 8 million cells (provided also acceptance criteria 2, 3 and 4 are fulfilled), proceed directly with Example 2B.

In case the total cell number after P0 is not higher than 8 million cells (provided however that acceptance criteria 2, 3 and 4 are fulfilled), an extra sub-cultivation step, termed "P0+" or "P0 plus" is performed. (For the avoidance of doubt, throughout this document, it is specifically indicated in case certain cells were sub-cultivated in a step "P0+"; in other words, where no such specific indication is given, cells were passed from P0 directly to P1 (Example 2B)). The conditions of P0 correspond exactly to the conditions of P0, described above, except that the resuspended cells being subjected to P0+ originate from P0, not directly from Example 2A.

The cells are cultured in P0+ until confluence of the cells is 80 to 90 %. Then, the cells are trypsinized. The above acceptance criteria are assessed: 1 yield, 2 viability, 3 confluence, 4 morphology. When all criteria are fulfilled, proceed with Example 2B. When one or more of the criteria are not fulfilled (including yield), discard the cells. In particular, the cells are discarded if the total cell number following P0+ is not at least 8 million cells.

Total cell numbers in this example are indicated based on an entire human umbilical cord as starting material (full scale run). If the amount of starting material is different (e.g. only half of a human umbilical cord), then the total amount of the cells for acceptance criterion 1 is adjusted arithmetically accordingly.

Depending *inter alia* on the yield of cells, which varies to some degree between different full-scale runs, also in view of the variation in the biological material from different individuals, the cumulative Population Doublings during P0 are subject to some variation.

Example 2C: Further Expansion (P1 and subsequent passages)

Trypsinized cells from P0 (or P0+, but only in case explicitly so described) in Example 2B, as described therein, were obtained and sub-cultured for one or more passages, as follows.

The first sub-culture is termed "P1". At the end of P1, the cells are again trypsinized and sub-cultured. This sub-culturing/trypsinization cycle is done preferably for 4 passages (P1 counting as the "first passage"), although up to 12 passages have been done in some cases. Each passage is consecutively numbered, i.e. P1 follows after P0, P2 follows after P1, and so on. Unless specified otherwise, the cells were grown and sub-cultured until passage 4 (P4).

For each passage from P1 onwards, all culture plates have the same defined size. In particular, for each passage, the culture vessel was composed of a stack of CS5, CS10 or CS20 culture plates (Corning, Corning, NY, USA). For each passage from P1 onwards, the cells are grown in MSCBM CD with 5 % hPL without antibiotics (see Example 0; 50 mL MSCBM CD/hPL per T-225 flask). Unless stated otherwise, antibiotics are discontinued in this example for all passages after P0; in other words, in this example MSCBM CD/hPL is used without addition of antibiotics for P1 and all subsequent passages.

The cell density at the onset of each passage is 2.500 cells/cm$^2$.

After each passage cells are trypsinized. Cells are counted at the end of each passage, after trypsinization

As a function of the cell density at the time of inoculation, the ground surface of the cell culture vessel (which together define the total cell count at the beginning of the passage) and the total cell count at the end of the passage, the cumulative Population Doublings can be arithmetically determined.

In the experience of the inventors, the cumulative Population Doublings during P1 and P2 (total of P1 plus P2, but not including P0), as a very general rule, are normally in the range of 9-13.

In the experience of the inventors, the cumulative Population Doublings during P3 and P4 (total of P3 plus P4, but not including P0, P1 and P2), as a very general rule, are normally in the range of 6-10. Consequently, the cumulative Population Doublings during P1, P2, P3 and P4 (total of P1 plus P2 plus P3 plus P4, but not including P0), as a very general rule, are normally in the range of 15-23.

Optionally - where specifically indicated herein - cells were frozen after a specific passage, e.g. P2. The cells can be frozen, e.g. snap-frozen in liquid nitrogen, according to standard procedures. It is well possible to de-frost such frozen cells and to e.g. subject them to further culture passages after de-frosting. The freezing can occur either as entire batch or in aliquots. Freezing in aliquots, preferably in vials, is much preferred and was typically done in this example, unless specified otherwise.

When cells are frozen and thawed after a specific passage, the numbering of the passage continues after thawing. For example, when the cells are frozen after P2, the next passage after the thawing is then P3. Preferably, and unless otherwise indicated, also the counting of the cumulative Population Doublings continues after the thawing.

Preferably, cells are aliquoted and frozen following P2. The so-obtained frozen aliquots are available for thawing individually at a desired point in time. The frozen cells, preferably in aliquots, can be referred to as Primary Cell Bank (PCB). In other words, it is not necessary - and typically not desired - to thaw all aliquots of the PCB at the same point of time. In contrast, the aliquoting and individual freezing of the aliquots provides the advantage that individual aliquots can be removed from freezing, thawed and subjected to further culturing (sub-culturing, further expansion) at individual, and individually desired points in time.

In a specific example of a full-scale run, 168 vials (each containing $20 \times 10^6$ cells, 4 ml content) were obtained after P2, beginning from one human umbilical cord as starting material. Each of the vials can be frozen, and individual vials can be thawed at individual time points for further sub-culturing, e.g. P3 and onwards. In one specific example of a full-scale run, one vial obtained and frozen after P2 is thawed and the cells contained therein are subjected to P3 and P4. In that specific example, after P4, 168 vials (each containing $20 \times 10^6$ cells, 4 ml content) were obtained.

The cells are cultured in each passage as described until the confluence of the cells is 80 to 90 %. This is usually the case after a period of ca. 3-4 days. Then, the following acceptance criteria are preferably assessed: 1 yield, 2 viability, 3 confluence, 4 morphology. Determination of acceptance criteria 1 and 2 occurs after trypsinization of the cells. The details are as follows.

| Acceptance criterion | | After P1 and all passages following P1 | Test |
|---|---|---|---|
| 1 | Yield (full scale run) | After P1: at least 225 x $10^6$ cells | Cell counting after trypsinization |
| | | After P2: at least 7 x $10^9$ cells (*) | |
| | | After P3: at least 250 x $10^6$ cells (*) | |
| | | After P4: at least 7 x $10^9$ cells (*) | |
| 2 | Viability | More than 80 % of cells viable | Staining with 7-amino actinomycin D (7-AAD) |
| 3 | Confluence | At least 80 %, preferably 80 to 90 % confluent | Microscopic determination |
| 4 | Morphology | Majority of cells small, spindleshaped and in defined colonies | Microscopic determination |
| (*) After P2 the cells can be frozen in aliquots to prepare a Primary Cell Bank (PCB). Cell numbers for P3 and P4 are indicated based on the scenario that P3 is initiated with one vial from the PCB, which contains 20 $\times$ $10^6$ cells. | | | |

It is important to note that the yields obtainable by expansion according to the present invention are very high compared to previously known methods for preparing and expanding mesenchymal stem cells.

In this example, all four acceptance criteria must be fulfilled: if one of the criteria is not fulfilled, the entire cell culture from the respective passage is discarded. It is the observation of the present inventors that all of criteria 1, 2, 3 and 4 are normally fulfilled, and that discarding is hence not necessary. However, the possibility to discard in case of non-fulfillment guarantees that subsequent passages are only conducted if all acceptance criteria are fulfilled.

In summary, Example 2 shows that the stromal Wharton's Jelly holds cells in an expansion-competent state. The stromal Wharton's Jelly cells were identified as a convenient source of potentially multi-potent stromal cells that can be maintained and expanded in culture.

Comparative Examples

For comparison purposes, cells were isolated as described by WO 2004/072273 A and Sarugaser et al., 2005, Stem Cells, vol. 23, p. 220-229, specifically as described in the following Comparative Examples 1, 2A, 2B and 2C.

Comparative Example 1

Vessels were isolated from human umbilical cord. The dissected vessels were sutured to create a loop, blocking the passage of fluid either into out of the vessel.

Comparative Example 2A

The loop (Comparative Example 1) was immediately placed into a 50 mL tube containing 1 mg/mL collagenase Sigma C-0130 solution in PBS (w/o $Mg^{2+}$ and $Ca^{2+}$) at 37 °C. According to the literature, the collagenase Sigma C-0130 has a specific activity of 0.25-1.0 FALGPA units/mg solid. For information, FALGPA units are not directly arithmetically convertible into Wünsch units because the respective units refer to different assays with different substrates; however, there is some general experience in the art on how Wünsch units relate to FLGPA units. Based on that general experience it can be concluded that the total activity of collagenase used in Comparative Example 2 is much higher than the total activity of collagenase used in Example 2.

After 18-20 hours of incubation with collagenase, the digestion solution was stopped by adding 2 volumes of PBS and 1 mL of Fetal Bovine Serum (FBS). Thus, the total duration of collagenase treatment in Comparative Example 2 is much longer than the total duration of collagenase treatment used in Example 2. The digested samples were filtered to remove undigested tissue and centrifuged at 1,800 rpm (Beckham Coulter Allegra™ 25R) for 15 minutes. The cells were re-suspend in 75 % α-MEM medium, 15 % defined FBS, 10 % antibiotics. To lyse the blood cells, a solution of ammonium chloride ($NH_4Cl$), potassium bicarbonate (KHCOs) and EDTA was used in a ratio of 1:3 v/v with the cellular suspension for 5-10 minutes on ice by gently agitation. After centrifugation at 500 $\times$ g for 10 minutes, the cells were re-suspend and counted by Trypan Blue (0.4 %) dye.

Comparative Example 2B

The perivascular-Wharton's Jelly-derived cells (PVWJ) obtained as described in Comparative Example 2A were seeded at density of 6,000-8,000 cells/cm$^2$ and incubated at 37 °C in $\alpha$-MEM (without nucleosides, Life Technologies, Waltham, MA, USA, #22561-021) supplemented with 15% defined FBS (HyClone, GE Healthcare, USA, #SH30070.03) and antibiotics in a 5 % CO$_2$, 95 % humidity incubator to start the culture "P0".

The media was exchanged for the first time after 5-6 days, than was refreshed every 2 days. After 6-10 days of culture the cells reached confluence. At end of P0, after a period of 6-10 days, the cells reached confluence. They were then trypsinized. The total cell number was determined with Trypan Blue dye (0.4 %).

Comparative Example 2C

Trypsinized cells obtained from Comparative Example 1B were obtained and sub-cultured, as follows. From culture passage 1 ("P1") onwards, the cells were seeded at density of 3,500-4,000 cells/cm$^2$ in $\alpha$-MEM with 15 % defined FBS. Antibiotics should be discontinued after P0; in other words, growth medium is used without addition of antibiotics. The cells are termed perivascular Wharton's Jelly-derived cells ("PVWJ"). Perivascular Wharton's Jelly (PVWJ)-derived mesenchymal stromal cells can also be reffered to as "perivascular zone-derived mesenchymal stromal cells". Unless specified otherwise, the cells were grown and sub-cultured until passage 4 (P4).

Optionally - but only where specifically indicated - cells were frozen after a specific passage, e.g. P2. It is well possible to thaw (de-frost) such frozen cells and to e.g. subject them to further culture passages after de-frosting.

Example 3: Analysis of cells after digestion

Cells obtained as described in Example 2A (i.e. after the digestion step, either with or without platelet lysate (PL)) are termed stromal-Wharton's Jelly derived cells or "SWJ". These are compared to PVWJ-derived control cells ("PVWJ", comparative Example 2A). SWJ and PVWJ were stained with 7-Amino-actinomycin-D (7AAD)-staining (Via Probe; BD Biosciences; used according to the manufacturer's instructions) to detect apoptosis by FACS analysis. Unstained sample was acquired to detect the sample auto-fluorescence and set the photomultiplying tubes (PMT; FACS sensors) for all the considered channels. Compensation settings were established by acquiring single color stained tubes. Collected data were analyzed by Diva software (BD Biosciences). Statistics by t-test. Results are shown in Fig. 4A.

The FACS analysis of freshly isolated samples revealed that, for both NB4-collagenase preparation-based digestion protocols (with and without platelet lysate (PL), see Fig. 4A), the inventors detected a viability greater than 97 % of total cell population. In particular after NB4-collagenase preparation-based digestion without PL (3 hour digestion) they observed an apoptosis rate of 1.5 %$\pm$1.3 %, and after NB4-collagenase preparation-based digestion with 1 % PL (3 hour digestion), the apoptotic cells were only 1.45 % $\pm$ 0.3 % of total isolated cells. However, 30.6 % $\pm$ 9.6 % of total isolated PVWJ according to Comparative Example 1 were positive for 7AAD (Fig. 4A, higher shaded bar on the right). Thus, it could be shown that samples treated with NB4-collagenase preparation (SERVA) for 3 hours were more viable than the cells isolated as described in Comparative Example 1. This suggests that the protocol according to Comparative Example 1 is more harmful for the cells than the protocol according to the present invention; i.e. while the protocol according to Comparative Example 1 leads to the isolation of more cells, a greater fraction of them is apoptotic. Apoptotic cells are not suitable for expansion.

Further, by comparing the use of platelet lysate (yes/no) and not observing a significant difference regarding the percentage of AAD7-positive cells (Fig. 4A), the inventors could show that platelet lysate does not negatively influence the collagenase activity, in releasing cells and in their viability. Surprisingly, the presence of platelet lysate (normally comprising plasma) had no negative effect on digestion, or more particularly on the yield of the cells, although platelet lysate had been previously reported to negatively affect the activity of certain enzymes, including proteases (Chesney et al., J. Clin. Invest., 1974, vol. 53, p. 1647-1654).

Example 4A: Analysis of physical properties of SWJ-derived cells isolated according to the present invention in comparison with PVWJ-derived cells

Next, the inventors aimed at evaluating the total cell number and the physical properties of SWJ-derived cells isolated according to the present invention (Example 1 and Example 2A) versus the PVWJ-derived cells from the protocol according to Comparative Example 1 and Comparative Example 2A). This comparative study was based on the evaluation of collagenase efficiency and physical parameters (by FACS) of respective freshly isolated cells.

First, regarding the total number of freshly isolated cells, the inventors obtained more PVWJ-derived cells by the method according to Comparative Example 1 than they obtained SWJ-derived cells by the method according to the present invention as described in Example 1.

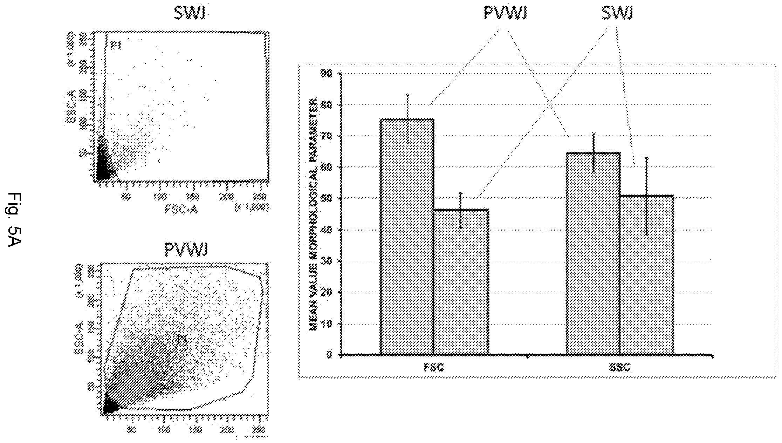

Second, the analysis of physical parameters by flow cytometry, namely forward-scattered light (FSC) and side-scattered light (SSC), revealed that the SWJ-derived cells have a lower FSC and SSC in comparison to the PVWJ-derived cells isolated by the method according to Comparative Example 1. Results are indicated in the following table and visualized in Fig. 5A.

**Table: SSC and FSC as experimentally determined**

| Cell | | FSC | SSC |
|---|---|---|---|
| **PVWJ-derived cells according to Comparative Example 2A** | Mean of six samples | 75.42166667 | 64.65666667 |
| | ST-DEV | 18.66791951 | 15.1647779 |
| | SEM | 7.75 | 6.19 |
| **SWJ-derived cells according to Comparative Example 2A** | Mean of four samples | 46.1925 | 50.795 |
| | ST-DEV | 9.660766986 | 21.28367273 |
| | SEM | 5.58 | 12.29 |
| | t-test | 0.025231608 | 0.296671217 |

On average, the SWJ-derived cells have a smaller cell volume (determined by FSC) with a lower internal complexity (determined by SSC) than the PVWJ-derived cells. The difference in FSC is statistically significant. In particular the SWJ-derived cells (according to the present invention, n=4) have a lower FSC and SSC in comparison to the PVWJ-derived cells isolated by the method according to Comparative Examples 1 and 2A (PVWJ, n=6). This flow cytometry readout indicates that the SWJ-derived cells isolated by the method according to the present invention are characterized by more homogeneous small cells with low cytoplasmic complexity, whereas the PVWJ-derived cells are characterized by a heterogeneous population with larger average volume and greater cytoplasmic complexity. Homogeneous in this context means that substantially all cells are much alike or similar. Smaller volume and lower internal complexity are both features that indicate stemness and performance (see e.g. Wagner et al., 2008, PLoS One, 2008, 3: e2213). In general, cells with a more complex internal structure appear more shiny/brighter in the side scatter analysis, owing to the internal structures.

Example 4B: Morphological phenotype of freshly isolated cells: PVWJ cells are different from the cells isolated according to the present invention

The morphology of SWJ cells and PVWJ cells was compared. To ensure a high degree of comparability, one single cord was divided in two parts: one part was prepared with the method described in Examples 1, 2A and 2B, the other part was prepared according to Comparative Examples 1, 2A and 2B, in both cases however, the cells were analyzed specifically two or three days post-seeding culture passage 1 (P1), (original magnification 100X).
The inventors found that cells derived from pure stromal Wharton's Jelly according to the present invention (SWJ) are homogeneous, small and spindle shaped (Fig. 5B, left), while cells derived from perivascular Wharton's Jelly (PVWJ) are large and have a prominent cytoskeleton (Fig. 5B, right). Perivascular Wharton's Jelly (PVWJ)-derived mesenchymal stromal cells can also be reffered to as "perivascular zone-derived mesenchymal stromal cells".

Example 4C: Colony formation of freshly isolated cells: PVWJ cells are different from the cells isolated according to the present invention

Next, the colony-formation potential of the cells as described herein was investigated. This was done as follows: SWJ were obtained as described in Examples 1 and 2A, PVWJ were obtained as described in Comparative Example 1. Then, using a sterile funnel, the cell-containing extract is filtered through two layers of one sterile gauze pad to remove undigested tissue. Centrifuge at 680 $\times$ g for 15 minutes using 250 ml centrifuge tubes. Discard the supernatant (pour off or aspirate using low vacuum). Re-suspend the pellet in 1-2 mL of MSCBM CD/GA/hPL (see Example 0). Plate the resuspended cells in 3 $\times$ T225 flasks or 1x 1-Stack in MSCBM CD/GA/hPL medium (see Example 0; 50 mL medium per T-225 flask or 150 mL per 1x1 stack) to start the P0 culture. Do not move the P0 culture for the first 5 days after seeding. Change medium after 5 days. Feed every 3 days thereafter with MSCBM CD/GA/hPL. Harvest P0 cells on day 12 with 3 U/mL trypsin and neutralize with MSCBM + 1 % hPL. Results are shown in Fig. 5 C. These findings demonstrate that the cell isolation protocol according to the present invention generates a more homogeneous cell population capable to generate more compact colonies after initial seeding (Passage 0).

Example 5: Comparison of SWJ-derived cells according to the present invention vs. bone marrow-derived cells (BM).

Bone marrow derived MSC (BM-MSC, obtained as described by Grisendi et al., Cytotherapy, 2010, vol., 12, p. 466-477, were seeded at density of 6,000 cell/cm$^2$. SWJ-derived cells as described herein (Examples 1, 2A, 2B and 2C, specifically cultured until passage 5 (P5), were seeded at density of 2,800 cell/cm$^2$. After 2-3 days of culture they were sub-confluent, and five representative images were acquired for both cell types. In particular, the plastic-adherent MSC were visualized with Axioskop-Observer-1 microscope (Zeiss) using a 10x objective, equipped with Axiocam camera. The images were acquired and processed by AxioVision 4.8.2 software to evaluate the cell area. (Moore et al., Exp. Eye Res. 2013, vol. 115, p. 178-188; Zeilbeck et al., PLoS One., 2014; vol. 9(4), e95546).
Results are shown in Fig. 6. It was found that cells SWJ-derived cells as described herein have a smaller average surface area than the MSCs extracted from BM.

Example 6: Cell surface markers of freshly isolated cells

Materials and Methods: Three umbilical cords were subjected to two different methods (state of the art according to Comparative Examples 1 and 2A, "PVWJ" vs. cells according to the present invention, see Examples 1 and 2A, "SWJ".
Immediately after isolation, the PVWJ/SWJ-derived cells were counted and resuspended in 1mL of PBS 1X in fluorescence-activated cell sorting (FACS) analysis polypropylene tubes (200,000 cells/tube). The cells were incubated in 100 $\mu$L blocking buffer containing Dulbecco's modified Eagle medium (DMEM), 10 % fetal bovine serum (FBS), 0.1 M sodium azide and 66.6 mg/mL human immunoglobulin G for 20 minutes on ice. Subsequently they were washed in 1 ml of 1 $\times$ PBS and centrifuged at 300 g for 5 minutes.
Samples were subsequently stained for 30 min on ice with primary antibodies in 90 $\mu$L PBS with 0.5 % bovine serum albumin (BSA; Sigma) and analyzed with FACSAria III flow cytometer (BD Biosciences) equipped by two air-cooled lasers at 488 and 633-nm wavelengths. For indirect staining (for anti-GD2 only), a secondary rat anti-mouse Allophycocyanin (APC)-conjugated antibody (BD Pharmingen) was used. 7-Amino-actinomycin-D (7AAD)-staining (ViaProbe; BD Biosciences; used according to the manufacturer's instructions) was evaluated by flow cytometry to detect apoptosis. Unstained sample was acquired to detect the sample auto-fluorescence and set the PMT for all the considered channels; fluorescent cross talk is controlled by compensation adjustment. Compensation settings were established by acquiring single color-stained tubes. Collected data were analyzed by Diva software (BD Biosciences).
Results: The immuno-phenotypic analysis of freshly isolated samples revealed that, for both isolation methods, the inventors identified CD105, CD73 and CD90, which are typical surface antigen expression of MSCs. Similarly, platelet-derived growth factor receptor $\beta$ (CD140b) was detected after both isolation methods. HLA-DR was not detected in both samples, however PVWJ-derived cells were found to express 3G5, while SWJ-derived cells did not, indicating that the method according to the present invention does not lead to isolation of perivascular cells, in accordance with the origin of the cells in the stromal Wharton's Jelly. Relative display of some surface markers is shown in Fig. 7 A, left panel. In Fig. 7A, right panel, some surface markers are indicated which are expressed/not expressed in the majority of cells.
The cell surface markers were also analyzed after prolonged culture of the cells. Cell cultures originating from two independent isolations and at different cumulative population doublings were analyzed. The following was found: There are no significant differences in the display of CD105, CD90 and CD73 (positive markers of MSC) among different isolations of cells. CD105, CD90 and CD73 are also uniformly high at different cumulative Population Doublings (cPD). To the contrary, CD45, CD14, CD34, CD19 and HLA-DR (known to be negative markers of MSC) are expressed at very low levels in cells from both isolations and at different cumulative Population Doublings (see following Table).

**Table: phenotypic characterization by flow cytometry (% positive cells). cPD = cumulative Population Doublings**

|  | CD45 | CD73 | HL-DR | CD14 | CD34 | CD90 | CD105 | CD19 |
|---|---|---|---|---|---|---|---|---|
| Batch 1 after 8 cPD | 3.8 | 98.9 | -0.6 | 0.0 | 1.1 | 99.2 | 99.1 | -0.5 |
| Batch 1 after 16 cPD | 4.1 | 99.1 | -0.7 | -0.2 | 1.7 | 99.2 | 99.1 | -0.8 |
| Batch 2 after 8 cPD | 1.4 | 99.2 | -0.3 | 0.1 | 2.6 | 99.2 | 99.1 | -0.2 |
| Batch 2 after 16 cPD | 0.8 | 99.1 | -0.2 | 0.7 | 4.2 | 99.1 | 99.10 | -0.1 |

These data show that the conditions of expanding stromal WJ-derived cells in the method according to the present invention are able to generate populations of pure stromal WJ-derived cells at high yield with a stable phenotype over several cumulative Population Doublings. Therefore, the cells as described herein are suitable for the creation of a Cell Bank.

Example 7: Cell surface markers on expanded cells

Materials and Methods: In order to characterize surface antigens on PVWJ-derived cells and SWJ-derived cells, a FACS array was performed from cells contained in a Cell Bank, prepared from the same umbilical cord according to different methods (Examples 1 and 2A, 2B and 2C vs. Comparative Examples 1 and 2A, 2B and 2C). The cells were de-frosted at P2 (Example 2C/Comparative Example 2C) and expanded until P4 (Example 2C/Comparative Example 2C) to perform FACS analyses. After detachment with a cell detachment solution of proteolytic and collagenolytic enzymes (Accutase, Thermo Fisher) the cells were centrifuged 500 $\times$ g for 10 minutes. The pellet was re-suspend in culture media (MSCBM CD/hPL for SWJ or $\alpha$-MEM with 15 % defined FBS for PVWJ) and counted by Trypan Blue 0.4 % dye. The cells were washed in 1X PBS and centrifuged at 300 $\times$ g for 5 minutes. The cells were resuspend in BD Pharmingen Stain Buffer + EDTA and 250,000 cells/well are plated in 96-well plates. The flow cytometry screening was conducted according to the Human Cell Surface Marker Panel datasheet (BD Lyoplate™, BD Biosciences) according to the Manufacturer's instructions. Results are shown in Fig. 7B.

It was specifically tested whether freshly isolated cells as described herein (Examples 1, 2A) express CD46, CD55, and CD59. As shown in Fig. 7B, both VPWL cells and SWJ cells express these surface complement regulators. This is in line with earlier descriptions, by e.g. Ignatius et al., J. Cell. Biochem., 2011, vol. 112, p. 2594-2605 and Schraufstatter et al., World J. Stem Cells, 2015, vol. 7, p. 1090-1108, who had shown that bone marrow-derived MSCs express the cell surface complement regulators, and MSC engineered to up-regulate CD46, CD55, and CD59 are believed to protect themselves from complement-mediated cell lysis *in vitro* and *in vivo.* Fig. 7B confirms that the cells described herein share this particular expression phenotype.

Example 8: Gene expression of cells described herein as determined by quantitative Reverse Transscriptase PCR (qRT-PCR).

Materials and methods: Cells obtained as described herein (Example 2C), in particular: after passage 4, were used for this analysis. Total cellular RNA was isolated using a single-step method with TRIzol (Life Technologies) according to the manufacturer's instructions. First-strand complementary cDNA was synthesized from 1 $\mu$g of total RNA using a revertAid H minus first-strand cDNA synthesis kit (Thermo Scientific) according to the manufacturer's instructions. The single strand cDNA was quantified by spectrophotometer (Beckman Coulter DU® 730) in order to use 10 ng of cDNA in each Real-Time PCR well.

The quantitative real-time PCR technique was performed by Applied Biosystems StepOne™ Real-Time PCR System and the Fast SYBR® Green Master Mix reagent. The quantification of gene expression for each target gene and reference gene was performed in separate tubes. Forward and reverse primers were designed using IDT PrimerQuest® (available online at http://eu.idtdna.com/PrimerQuest/Home/Index), ensuring they spanned an intron sequence to be specific for mRNA rather than genomic DNA. The relative expression level of the target gene was normalized to the expression level of the endogenous reference $\beta$-actin gene.

Results are shown in Fig. 8. Fig. 8 shows that e.g. APCDD1, PPARG and FLVCR2, are expressed in SWJ-derived cells. Expression is consistent between different SWJ-derived cell populations prepared according to the present invention. This shared expression pattern indicates the consistency of the cells of the present invention from the point of view of gene expression.

Remarkably however, the expression pattern of these genes differs remarkably from PVWJ-derived cells isolated according to the state of the art. In particular, APCDD1 is not expressed at all in PVWJ-derived cells. This is concluded from the finding that no amplification of the APCDD1 gene was observed at all in the sample from PVWJ-derived cells. The inventors conclude that by RealTime-PCR APCDD1 expression is detectable only in SWJ-derived cells decsribed herein, but not detectable in PVWJ-derived cells. The expression of APCDD1 thus clearly distinguishes the cells described herein from PVWJ-derived cells.

While earlier literature has been uncertain on whether or not nonperivascular MSCs are distinct from perivascular MSCs (e.g. Davies et al., Stem Cells Translational Medicine, 2017, vol. 6, p. 1620-1630), this example now provides proof that stromal Wharton's Jelly derived cells obtained according to the present invention are distinct from perivascular MSCs.

Example 9: Proliferative potential of the cells obtained according to the present invention vs. reference cells reference cells

Materials and Methods: Cell Culture: Stromal Wharton's Jelly-derived cells (SWJ) as described herein, obtained as described in Examples 1, 2A, 2B and 2C after P1, or perivascular Wharton's Jelly-derived cells (PVWJ), obtained according to Comparative Examples 1, 2A, 2B and 2C, both cell populations obtained from the same umbilical cord, were seeded MSCBM CD with 5 % hPL (see Example 0) without growth factors and placed in a 37 °C, 5 % $CO_2$ incubator with 95 % humidity. Three days after plating, and every 3 days thereafter, cultures were re-fed. On day 4 or 5 of each passage cells were trypsinized, and counted.

Cell Counting: the cells were counted using 0.4 % Trypan blue dye in a Burker chamber and the cumulative population doubling (CPD) was calculated as described above. Results are shown in Fig. 9. A greater number of Population Doublings (PD) per passage are observed in the SWJ-derived cells than in PVWJ-derived cells (Figure 9A). Thus, the PD in SWJ-derived cells as described herein is faster than PVWJ-derived cells. A statistically significant increase in population doubling was measured in SWJ-derived cells at passages 2, 3 and 4 compared with the PVWJ-derived cells (by t test).

SWJ-derived cells have a high proliferative potential, which is maintained for at least 9 passages in the culture conditions used (growth medium MSCBM CD with 5 % hPL). Under these culture conditions, as experimentally determined with two independent isolates of SWJ-derived cells, the SWJ-derived cells will reach senescence between passage 10 and passage 12, as shown in the following Table.

**Table: growth of SVWJ according to the invention**

| Passage | Isolation 1 | Isolation 2 | |
|---------|-------------|-------------|---|
| 1 | 5.89 | 4.99 | |
| 2 | 5.00 | 5.47 | |
| 3 | 5.04 | 4.76 | |
| 4 | 5-02 | 4.24 | |
| 5 | 4.58 | 5.03 | Population Doublings per passage |
| 6 | 4.95 | 5.30 | |
| 7 | 5.40 | 4.69 | |
| 8 | 5.22 | 4.63 | |
| 9 | 4.92 | 4.54 | |
| 10 | 4.83 | 1.81 | |
| 11 | 4.58 | n/a | |
| 12 | 3.27 | n/a | |
| **Total** | 58.70 | 45.44 | **Cumulative Population Doublings** |

This example adds additional evidence that the conditions of isolating and expanding stromal Wharton's Jelly-derived cells (SWJ-derived cells) according to the present invention allows to generate large cell populations of SWJ-derived cells. This Example also adds evidence that the SWJ-derived cells are different from PVWJ-derived and have a higher proliferative potential.

The high proliferative potential and population doubling (PD) per passage increases the yield of the cell bank and/or reduces the time for production of a cell bank, respectively, compared with reference MSCs, such as PVWJ-derived cells. This is advantageous from an industrial point of view.

Example 10: Telomerase Activity

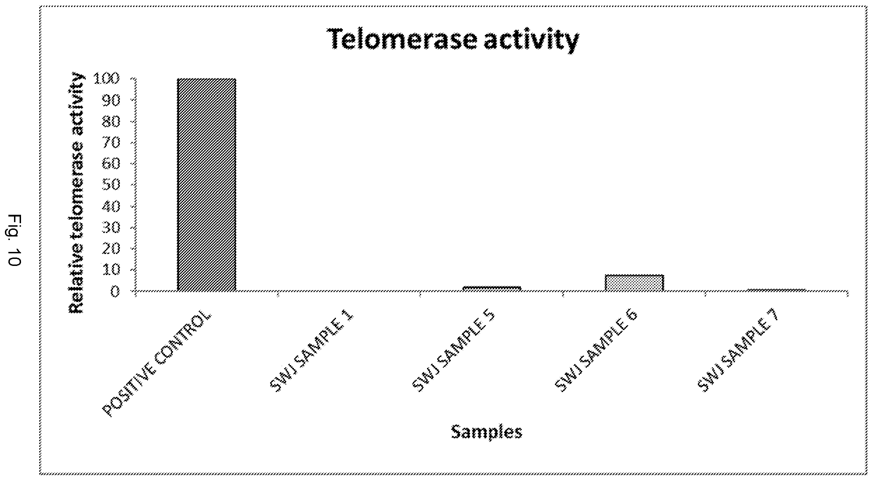

For this assay 200,000-500,000 Wharton's Jelly-derived stromal cells (SWJ) as described herein were aliquoted in a 1mL Eppendorf tube and then centrifuged at 1,200 rpm (Beckham Coulter Allegra ™ 25R) for 10 minutes at +4°C. The supernatant was removed and the cells were re-suspended in 1X PBS. After a second centrifuge step, the dry pellets were stored at -80°C. To perform the assay, the TeloTAGGG Telomerase PCR ELISA PLUS kit (Roche Diagnostics) was used according to the manufacturer's instructions. Results are shown in Fig. 10. The analyzed samples showed a mean of 2.4±1.7 % of telomerase activity respect to the positive control provided from the kit (considered as 100 %). This confirms that the higher proliferation ability of SWJ-derived cells is not correlated with an anomalously high telomerase activity.

Example 11: Senescence

Material and Methods: Wharton's Jelly-derived stromal cells (SWJ) as described herein after trypsinization (for cell origin see Examples 1 and 2A; for passages see Examples 2B and 2C) following passage 6 (P6), passage 8 (P8) or passage 11 (P11), passage 12 (P12), were seeded in six multi-well plates at a density of 3,000 cells/cm$^2$ in 3 ml of culture media (DMEM with 5 % hPL or MSCBM CD with 5 % hPL) and cultured for 4-5 days at 37 °C in 5 % $CO_2$, 95 % humidity incubator, in triplicates unless otherwise specified. Then, the cells were washed in 1X phosphate-buffered saline (PBS) and fixed for 10 minutes at room temperature in 2 % formaldehyde/0.2 % glutaraldehyde. The culture plate was rinsed two times in PBS 1Xand 1 mL of fresh $\beta$-Gal stain solution (1 mg of 5-bromo-4-chloro-3-indolyl-$\beta$D-galacto-side/100 $\mu$L of dimethylformamide, 40 mM citric acid/sodium phosphate, pH 6.0, 5 mM potassium ferrocyanide, 5 mM potassium ferricyanide, 150 mM NaCl, and 2 mM $MgCl_2$) was added (used according to the manufacturer's instructions). The cells were incubated over night at 37 °C and the color was developed with the light of an inverted microscope as described in the instructions provided by the manufacturer (Cell Signaling Technology). The positive stained cells appeared blue-green in the cytoplasm. Stained samples were washed in PBS 1X and visualized by 2.5X objective using an inverted microscope (Zeiss). The positive stained cells (per field) were quantified by one observer and at least 8 fields of view were examined for each donor (unless otherwise specified).

Results: At early culture passages (P6-P8) only a few cells were weakly positive for $\beta$-galactosidase staining (data not shown), indicating that, even at very low density of seeding, SWJ-derived cells as described herein have a tendency to be marginally senescent. Without wishing to be bound to any theory, it is possible that the platelet lysate comprises supplements which are advantageous for growth of the cells ex *vivo*, e.g. growth factors.

Example 12: Osteogenic differentiation potential

Description of the Method: SWJ at passage 5 (Example 2C) were induced to differentiate into the osteogenic lineage. Briefly, SWJ were seeded at density of 10,000 cell/cm$^2$ in MSCBM CD with 5 % hPL in 6-multiwell (6-MW) plates. These 6-MW plates were pre-coated with 0.1 % gelatine solution. After 3-4 days' equilibration, the maintenance medium was exchanged and replaced by osteogenic medium. The osteogenic medium was composed of MSCBM CD with 5 % hPL, supplemented with 10mM beta-glycerophosphate, 0.1 mM ascorbic acid-2-phosphate and 10 nM Dexamethasone, plus additionally of 100 ng/mL rhBMP-2 (bone morphogenic protein 2) from the 7th day of induction onwards. Confirmation of osteogenic differentiation was performed through von Kossa staining (so that bone matrix is labelled dark) combined with Real-Time qPCR analysis (described below).

After 2 weeks, the cultures under osteogenic medium (cf. above) and parallel-control cultures (grown in parallel in MSCBM CD plus 5 % hPL, i.e. a medium which does not induce osteogenesis) were fixed and stained to identify the organic calcium deposits. For von Kossa staining, samples were fixed in ice-cold methanol for 2 min, then rinsed twice in distilled water, and incubated under a ultraviolet (UV) lamp with silver nitrate in water for 30 min. After two further washes in distilled water, 10 high-power fields were observed using 10X magnification (Axioskop-Observer-1inverted microscope; Zeiss). Digitally recorded images were analysed by ImageJ software (National Institute of Health, USA) selectively quantifying dark positively stained areas of organic calcium deposits. The experiments were performed in triplicates.

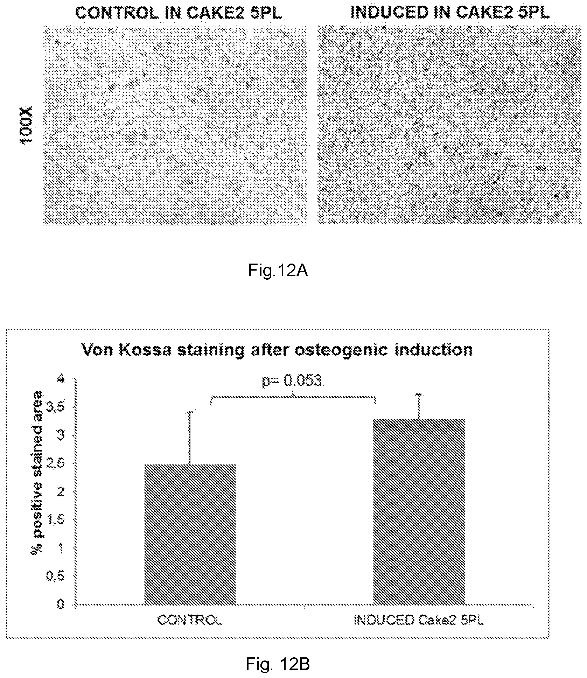

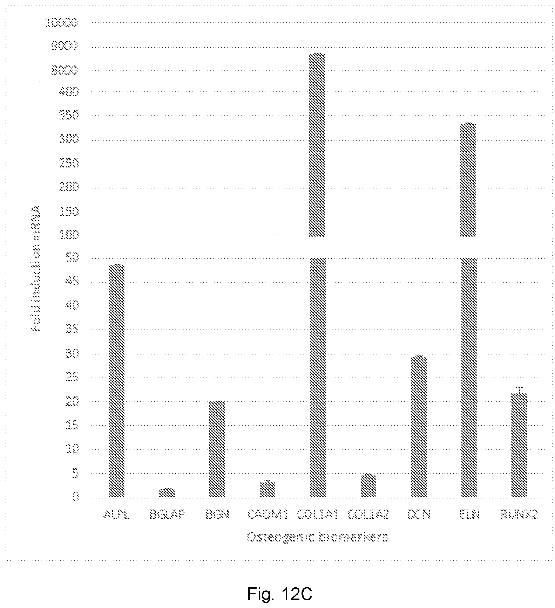

Results: Results are shown in Fig. 12. As shown, after 14 days of treatment with osteogenic medium, the morphology of control samples was different from the osteogenic medium-induced samples, and the von Kossa staining of calcium phosphate provided a black positive stain (Fig. 12A). The difference between the positive stained areas in control and induced samples were statistically significant (by T test) (Fig. 12B). To further confirm the osteogenic signature, molecular analyses were performed by RealTime-PCR (Fig. 12C). The relative expression level of osteogenic biomarker gene was normalized to that of the endogenous reference beta actin gene. The fold induction mRNA was calculated based on SWJ standard culture condition (MSCBM CD plus 5 % hPL), defined as non-induced and sub-confluent cells. All tested genes are significantly up-regulated respect to standard culture condition (p≤0.05, by T-test). The inventors observed that alkaline phosphatase (ALPL) and certain matrix proteins, such as biglycan (BGN), collagen (COL1A1), decorin (DCN) and elastin (ELN) were the most up-regulated biomarkers. A combination of these markers are characteristic for the osteogenic lineage (Murgia et al., PLoS One. 2016, vol. 11: e0163629). This expression profile confirms an initial osteogenic commitment of the cells.

Example 13: Adipogenic differentiation potential

Description of the Method: For adipogenic differentiation, SWJ cells (Examples 1, 2A, 2B, 2C) at culture passage 5 were seeded at 10,000 cell/cm$^2$ in MSCBM CD with 5 % hPL in 6 multiwell (6-MW) plates and, after 3 days, the medium was replaced by adipogenic induction medium. The adipogenic medium was composed of MSCBM CD with 5 % hPL, supplemented with 1 μM dexamethasone, 60 μM indomethacin, 10 μM insulin, 0.5 mM 3-isobutyl-1-methylxanthine (IBMX), 10% rabbit serum, 5% horse serum, 1 % L-glutamine and 1 % penicillin/streptomycin. The control cells were cultured with MSCBM CD with 5 % hPL. Cells were seeded in technical duplicates for each condition.

After 10 days the cells were stained with the stain Oil Red O (a commercially available stain for adipocytes) to confirm the differentiation, as follows: the cells were washed briefly with PBS 1x, fixed with vapors of 37 % formaldehyde for 10 minutes and then washed with water for 2 minutes. Staining was obtained by adding an Oil Red O solution (10mg/mL Oil Red O in ethanol 70 % and acetone) for 3 minutes into the wells. The cells were counterstained by Harris Hematoxylin (Bio-Optica, Milan, Italy) for 3 minutes. After treatment, the differentiated cells and controls were visualized by microscopical observations (Axio Observer A1 with Axiocam MRC5 color camera and Axiovision 4.82 software; all Zeiss).

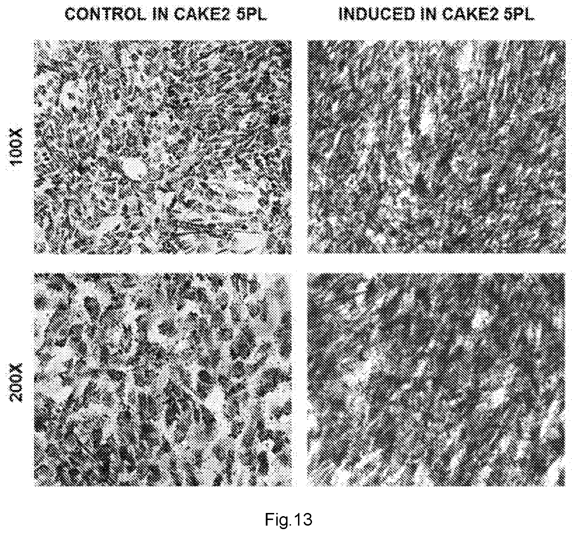

Results are shown in Fig. 13. The SWJ cells, after 10 days of treatment, were able to produce round lipid droplets inside the cells, confirming the adipose commitment. Lipid vacuoles were largely detected in induced samples, but not in the non-induced controls (Fig. 13).

Example 14: Chondrogenic differentiation potential

Description of the Method: For chondrogenic differentiation, 200,000-500,000 SWJ (Examples 1, 2A, 2B, 2C) at culture passage P5 were aliquoted in a 15 mL tube and centrifuged at 1,200 rpm (Beckham Coulter Allegra™ 25R) for 10 minutes. Cells were maintained in the pellet in growth medium at 37 °C with the plug opened. After 2 days of incubation the growth medium was substituted with the induction medium, composed of MSCBM CD with 5 % human platelet lysate (hPL), supplemented with: 500 ng/mL rhBMP-6, 10 ng/mL transforming growing factor-β, 50 mg/mL ITS+Premix (containing insulin 6.25 μg/mL, transferrin 6.25 μg/mL, selenic acid 6.25 ng/mL, BSA 1.25 mg/mL and linoleic acid 5.35 μg/mL), 100nM dexamethasone, 0.2 mM L-ascorbic acid-2-phosphate, 100 μg/mL sodium pyruvate, 40 μg/mL proline. The medium was exchanged every three days. Before and after each medium exchange, tubes were centrifuged at 1,200 rpm for 10 minutes as described above. During the incubation period, cells remain as pellet with the tube plugs opened. Induction lasted 21 days, and specimens were fixed for 1 hour in 10 % formaldehyde and then dehydrated by serial passages into ethanol at increasing concentrations, from 70 % (v/v) to 100 %. Samples were then included into paraffin blocks and cut in slices onto microscope slides for Alcian Blue staining. Slides were de-paraffinized with the Histo-C cleaning agent and rehydrated through passages into a decreasing concentration alcoholic ladder (from 100 % ethanol to 70 % ethanol) (v/v). Sample sections were then incubated with a 0.5 mg/mL hyaluronidase in buffer phosphate solution (8 g/L NaCl, 2 g/L NaH$_2$PO$_4$, and 0.3 g/L Na$_2$HPO$_4$) with 10 mg/mL BSA. Slides were washed in water for 5 minutes and then immersed in a 3 % (v/v) acetic acid solution for few seconds. The sections were stained with 10 mg/mL Alcian Blue solution (a commercial dye) in 3 % acetic acid (pH 2.5), remained there 30 minutes, and after a washing step in water, were counterstained for 5 minutes with nuclear Fast Red solution (a commercial dye).

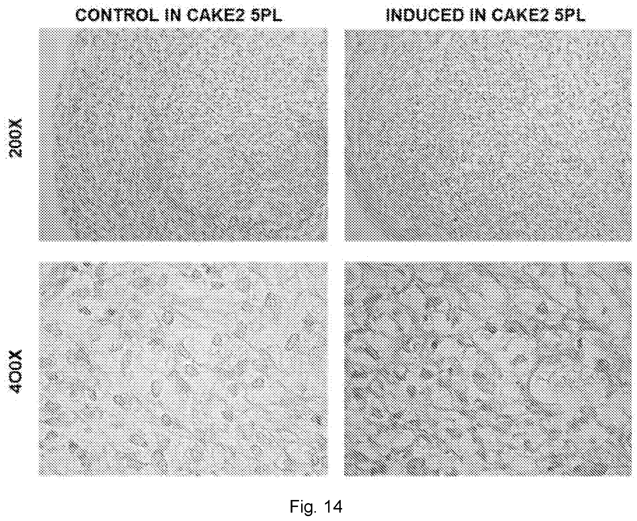

Results are shown in Fig. 14. The SWJ, after 21 days of treatment, had developed a multi-layered matrix-rich morphology. The induced SWJ showed a lower cellularity and the glycosaminoglycans within the extracellular matrix. The control SWJ showed a high cellularity and a poor matrix component.

**Brief description of the Figures**

**Fig. 1:**
Cross-section of the (human) umbilical cord and schematic view of sections (anatomical areas) of the (human) umbilical cord. the two grey-shaded elliptical shapes at the outside (1a and 1b) together form the umbilical cord lining membrane (comprising two layers: 1a (drawn as dark grey circle at the outside), the amniotic (or epithelial) layer, also termed umbilical cord lining membrane; and 1b (drawn as light grey circle at the inside): the sub-amniotic (or mesenchymal) layer; 2: umbilical vein; 3: umbilical arteries; 4: intervascular area; 5: perivascular area (shown in grey); 6: illustrative example of location of Wharton's Jelly; 7: umbilical cord blood (in the lumen of the vessel). Unless explicitly stated otherwise, the subamniotic stroma (1b) is comprised in the "stroma" referred to herein. Unless explicitly stated otherwise, the subamniotic Wharton's Jelly) is comprised in the Wharton's Jelly referred to as "stromal Wharton's Jelly" herein.

**Fig. 2:** Immunohistochemical characterization of different areas of the human umbilical cord. WJ: Wharton's Jelly; MW: multilayer wall; PV: perivascular; V: vessel; L: lumen. Stromal WJ: WJ at least 3 mm distant from the external wall of the cord vessels, and internal of the amniotic epithelium.

|↔| : Perivascular area of about 2.00 mm including WJ+pericyte+endothelial cells.

Materials and methods: A human umbilical cord specimen was formalin fixed and paraffin embedded. Sections were isolated and cut in 5 $\mu$m sections for further staining. The distinct staining in the different areas representing the stromal Wharton's Jelly and the perivascular area is apparent. The perivascular area has a more complex histomorphological pattern with vessels (V), perivascular cells (PV) bordering the WJ areas. The complexity is also related to the different cell type represented in the perivascular region. On the contrary, the stromal Wharton's Jelly appears histologically as a relatively more uniform area with more homogeneous cellular elements dispersed into the matrix of the stromal tissue. Isolated cells express markers that are commonly found in MSC populations ex *vivo*, such as CD10, CD140b, GD2, CD73 (not shown in this Figure). The immunohistochemical staining pattern for these markers also differs between the two regions. In Figure 2B, histological differences are more evident between the cord vessel area and the stromal Wharton's Jelly. In the vessels (V), the lumen (L) and the cells (arrows) that represents endothelial layer (CD31) and the sub-endothelial layer (CD146, CD271, GD2) are clearly evident. CD140b is also found to be expressed in the endoluminal part of the vessel. CD73-positive staining is found in the sub-endothelial layer, with an intensity that increases while going towards the matrix of the Wharton Jelly (Rasini et al., 2013, Cytotherapy, vol. 15, p. 292-306).

**Fig. 3:** Dissection (sectioning) and extraction of cells - for details see Example 1.

**Fig 4:** Results of Example 4, evidencing that the method of the invention is causative for significantly higher viability. For details see Example 4.

* PVWJ: Cells obtained according to a conventional method, see Comparative Examples. SWJ: cells isolated according to the present invention.

**A:** Apoptotic cells: effect of platelet lysate and comparison to a conventional method. Regarding the number of freshly isolated cells, the inventors obtained more cells (PVWJ) from the protocol according to Comparative Example 1, ($4.15 \pm 1.28 \times 10^6$) than from the protocol according to the present invention ($1.49 \pm 0.35 \times 10^6$). The graph represents the mean and the standard error of the mean (SEM) of apoptotic cells in three digestions (n=3). 30.6 % $\pm$ 9.6 % of total isolated PVWJ-derived cells according to Comparative Example 1 were positive for 7AAD (Fig. 4A, higher shaded bar on the right). In contrast, the percentage of SWJ-derived cells positive for 7AAD, both in presence and in absence hPL, is much lower. Thus, the inventors have found that cells obtained according to the present invention have a higher degree of viability than cells obtained by a method according to the state of the art.

**B:** FACS analysis of freshly isolated cell samples. The 4 FACS plots represent physical parameters (right) and the 7AAD positivity (i.e. apoptosis, on the left) of SWJ-derived cells (top) PVWJ-derived cells (bottom), respectively. In the graphs on the right, living cells are found left of the vertical line.

**C:** Graphical representation of total cell number, as determined by flow cytometry (Fig. 4B). The graph represents the mean and the standard error of the mean (SEM) of apoptotic cells in three digestions (n=3 for both cell populations).

**D:** Graphical representation of percentage of apoptotic cells, as determined by flow cytometry (Fig. 4B). The FACS analysis of freshly isolated samples revealed for SWJ-derived cells as described herein a viability greater than 79 % of total cell population. On the contrary 52.9 % $\pm$ 2.6 % of total cells isolated according to Comparative Example 1 (PVWJ-derived cells) were positive for 7AAD. **C:** cell isolation efficiency in SWJ (n=5) and PVWJ (n=9) samples **D:** The graph represents the mean and the standard error of the mean (SEM) of apoptotic cells in three digestions (n=3 for both cell populations).

**Fig 5:** Morphology and colony formation

**A:** Physical parameters determined by flow cytometry, i.e. Forward Scatter (FSC) and Side Scatter (SSC) of freshly isolated cells from an umbilical cord are represented (FACS plot on the left). The graph represents the mean and the SEM of physical parameters. The SWJ-derived cell population isolated (according to Examples 1 and 2A) is characterized by significantly smaller FSC values than the PVWJ-derived cell population isolated (according to Comparative Examples 1 and 2A; p=0.02 by T-Test). SSC: SWJ derived cells have lower cytoplasmic complexity than cells from PVWJ. For details see Example 4A.

**B**: Morphology studies at culture passage 1 (n=3). After expansion, the SWJ-derived cells (Example 2C) were characterized by small and spindle shape cell population, instead PVWJ-derived cells (according to Comparative 2C) were characterized by a large shape and prominent cytoskeleton. The two pictures represent the morphology of SWJ-derived cells (left) versus PVWJ-derived cells (right) two- or three days post-seeding in culture passage 1 (P1) in a representative sample (original magnification 100X).

**C:** Colony formation of the SWJ-derived cells and PVWJ-derived cells. For details see Example 4C. Cells were stained with crystal violet before taking the picture shown at the bottom. Cultures of SWJ -derived cell populations are characterized by consisting of cells with a small surface area; these cells tend to build colonies, while cultures of PVWJ-derived cell populations are characterized by consisting of cells with different surface areas, also including large cells. Thus, the PVWJ-derived cells appear more homogeneous; homogeneous in this context means that substantially all cells appear much alike or similar. The larger PVWJ-derived cells tend to grow relatively slower (data not shown).

**Fig. 6:** Quantitative determination of the cellular surface area: SWJ-derived cells vs. bone marrow-derived mesenchymal stromal cells (BM, or BM-MSC). Plastic-adherent MSC from bone marrow and from stromal Wharton's Jelly (BM and SWJ respectively) were visualized at culture passage 5 (P5). Five pictures for each sample were acquired, and 34 BM-MSC and 33 SWJ cells were optically identified. The cells were graphically encircled manually upon visual inspection. The BM-derived cells were characterized by a larger average cellular surface area ($4300.5 \pm 2486.5\ \mu m^2$) compared to the SWJ-derived cells ($1701.2 \pm 1137.3\ \mu m^2$) ($p = 5.15 \times 10^{-7}$, by ANOVA). It was thus concluded that the SWJ-derived MSCs as described herein are smaller than the BM-derived MSCs.

**Fig. 7:** Results of Example 7. The graph on the left represents the mean and the SEM of surface markers in PVWJ-derived cells and SWJ-derived cells, both freshly isolated from three umbilical cords by the two different isolation methods (SWJ: according to Examples 1 and 2A; PVWJ: according to Comparative Examples 1 and 2A). The immunophenotypic analysis of freshly isolated samples revealed for both methods display of CD105, CD73 and CD90, the typical surface antigen display of MSC. Similarly, display of platelet-derived growth factor receptor $\beta$ (CD140b) was detected after both methods. The HLA-DR was not expressed in both samples. However, display of 3G5 was detected only in the population of PVWJ-derived cells, indicating that the method of the present invention is specifically suitable for isolation of a population of SWJ-derived MSCs, which does not comprise perivascular cells.

**A:** The graph on the left represents the mean and the SEM of surface markers in PVWJ and SPPC freshly isolated from three umbilical cords treated by the two different digestion methods (Comparative Example 1, "PVWJ" vs. cells as described herein, SWJ). The panel on the right indicates some surface markers which are expressed/not expressed in the majority of cells.

**B:** The graph represents the mean percentage of cells displaying specific surface markers, in freshly isolated cells from umbilical cords by two different methods (Comparative Example 1, "PVWJ" vs. cells as described herein, SWJ).

**Fig. 8:** Quantitative gene expression of cells according to the present invention as determined by Real-Time-PCR (RT-PCR). Two independent cell populations isolated according to the present invention, were subjected to RT-PCR analysis; both are shown in Fig. 8 and compared to control PVWJ cells. The graph shows the difference of expression (Delta Ct) between 2 genes (gene of interest vs. beta-actin, a housekeeping gene).
APCDD1 expression was not detected in control cells.

**Fig. 9:** Proliferative potential: The graph represents the mean and the SEM (standard error of the mean) of cumulative population doublings of PVWJ-derived cells (n=9) and SWJ-derived cells (n=7) in the four culture passages analyzed (from P1 to P4). A greater number of Population Doublings (PD) per passage is observed in the SWJ-derived cells, compared to PVWJ-derived cells. For details see Example 9.

**Fig. 10:** Telomerase expression in different lots of SWJ-derived cells. For details see Example 10.

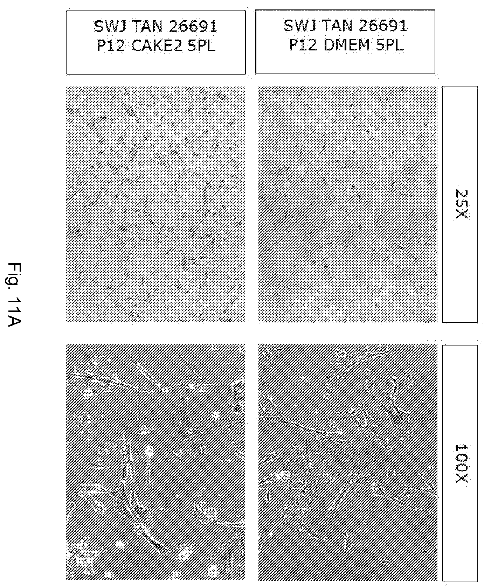

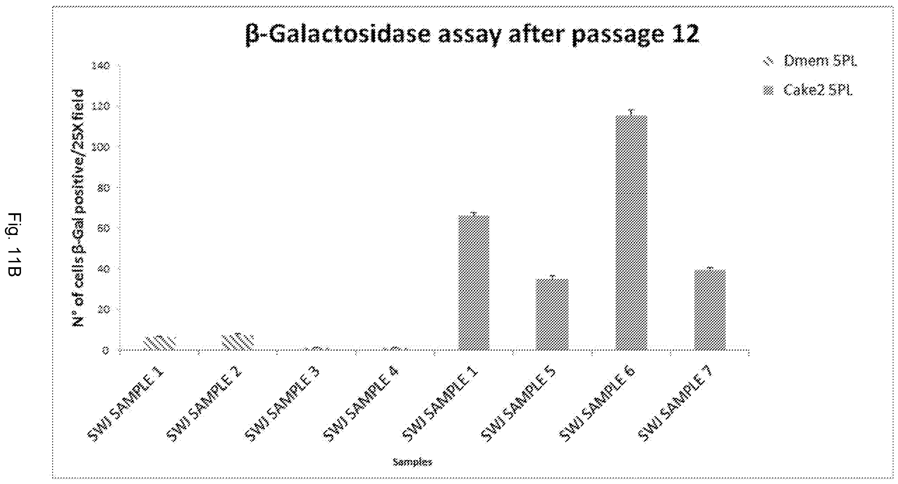

**Fig. 11:** Senescence in different lots of SWJ-derived cells. CAKE2 is an acronym for a basal medium substantially equivalent to MSCBM CD; 5PL is an acronym for 5 % (vol./vol.) human platelet lysate. For details see Example 11.

**Fig. 12:** Osteogenic potential of WJ-derived cell populations. The interruption of the bars indicates a change of scale. CAKE2 is an acronym for a basal medium substantially equivalent to MSCBM CD; 5PL is an acronym for 5 % (vol./vol.) human platelet lysate. For details see Example 12.

**Fig. 13:** Adipogenic potential of WJ-derived cell populations. For details see Example 13. The SWJ-derived cells produced round lipid droplets inside the cells (upper and lower panel on the right), confirming the adipose commitment. Lipid vacuoles were largely detected in induced samples, while absent in the non-induced controls. CAKE2 is an acronym for a basal medium substantially equivalent to MSCBM CD; 5PL is an acronym for 5 % (vol./vol.) human platelet lysate.

**Fig. 14:** Chondrogenic differentiation of SWJ-derived cell populations. For details see Example 14. After 21 days of treatment, the non-induced SWJ-derived cells (control) had developed a multi-layered matrix-rich morphology (two panels on the left). The induced SWJ-derived cells (two panels on the right) showed a lower cellularity, and the glycosaminoglycans within the extracellular matrix (dark-colored areas, corresponding to strong blue staining in the original colored image). CAKE2 is an acronym for a basal medium substantially equivalent to MSCBM CD; 5PL is an acronym for 5 % (vol./vol.) human platelet lysate.

## Claims

1. An ex vivo method for obtaining a mesenchymal stromal cell,
   the method comprising

   (a) isolating the stromal Wharton's Jelly or a fraction thereof from an umbilical cord, wherein the stromal Wharton's jelly is the Wharton's Jelly of the umbilical cord that excludes the perivascular area;
   (b) subjecting the stromal Wharton's Jelly or fraction thereof to enzymatic treatment comprising the enzyme collagenase in the presence of platelet lysate (PL), thereby releasing at least one cell from the stromal Wharton's Jelly or fraction thereof;

   wherein step (b) is followed by a step (c):
   (c) expanding said at least one cell in a growth medium comprising platelet lysate (PL).

2. The method according to claim 1, wherein (a) isolating the stromal Wharton's Jelly or a fraction thereof is **characterized by** a step of physically removing the vessels and the perivascular area from the umbilical cord.

3. The method according to claim 2, wherein the physical removing of the vessels and the perivascular area is **characterized by** stripping out the vessels and the perivascular area from the umbilical cord.

4. The method according to any one of the preceding claims, wherein (a) isolating the stromal Wharton's Jelly or a fraction thereof is additionally **characterized by** a step of physically separating the subamniotic stroma from the umbilical cord lining membrane, such as by separating the Wharton's Jelly from the umbilical cord lining membrane, particularly at least from the epithelial layer of the umbilical cord lining membrane.

5. The method according to claim 4, wherein physically separating Wharton's Jelly from the umbilical cord lining membrane is **characterized by** scraping out the Wharton's Jelly, preferably without mechanically dissecting the umbilical cord lining membrane.

6. The method according to any one of claims 2 to 5, wherein the umbilical cord is segmented prior to said step of physically removing the vessels and the perivascular area.

7. The method according to claim 6, wherein the segmenting is perpendicular to the cord, and wherein each perpendicular segment preferably has a length of about 1 to about 4 cm, preferably about 2 to about 3 cm, and more preferably about 2.5 cm.

8. The method according to claim 6 or claim 7, wherein segment(s) of vessels are removed from the segment(s) of the umbilical cord prior to step (b).

9. The method according to any one of the preceding claims, wherein the enzymatic treatment is performed by mixing stromal Wharton's Jelly or a fraction thereof with a Digestion Buffer comprising collagenase, preferably at a specific activity of not more than 0.5 Wünsch U/ml, more preferably not more than 0.25 Wünsch U/ml, and specifically 0.18 Wünsch U/ml.

10. The method according to any one of the preceding claims, wherein steps (a) and (b) together are conducted in a total time of less than 3 hours or the method according to any one of the preceding claims wherein in step (b) the platelet lysate is human platelet lysate (hPL).

11. The method according to any one of the preceding claims, wherein in step (c) the at least one cell is expanded in a growth medium comprising human platelet lysate (hPL).

12. The method according to any one of the preceding claims, wherein:

- the at least one cell is expanded in a growth medium that does not comprise any added serum, in particular no fetal bovine serum (FBS); or
- the at least one cell is expanded in a growth medium comprising basal medium MSCBM CD.

**Patentansprüche**

1. Ein Ex-vivo-Verfahren zur Gewinnung einer mesenchymalen Stromazelle,
das Verfahren umfasst

(a) die Isolation der stromalen Wharton-Sulze oder eines Anteils davon aus einer Nabelschnur, dabei handelt es sich um die stromale Wharton-Sulze aus der Nabelschnur unter Ausschluss des perivaskulären Bereichs;
(b) eine enzymatische Behandlung der stromalen Wharton-Sulze oder eines Anteils davon, die das Enzym Kollagenase in Gegenwart von Plättchenlysat (PL) umfasst, wodurch mindestens eine Zelle aus der stromalen Wharton-Sulze oder eines Anteils davon freigesetzt wird;

wobei sich an Schritt (b) ein Schritt (c) anschließt:
(c) die Expansion der mindestens einen Zelle in einem Wachstumsmedium, das Plättchenlysat (PL) enthält.

2. Verfahren nach Anspruch 1, wobei (a) die Isolierung der stromalen Wharton-Sulze oder eines Anteils davon durch einen Schritt der physischen Entfernung der Gefäße und des perivaskulären Bereichs aus der Nabelschnur gekennzeichnet ist.

3. Verfahren nach Anspruch 2, wobei die physische Entfernung der Gefäße und des perivaskulären Bereichs durch das Herausziehen der Gefäße und des perivaskulären Bereichs aus der Nabelschnur gekennzeichnet ist.

4. Verfahren nach einem der vorangehenden Ansprüche, wobei (a) die Isolierung der stromalen Wharton-Sulze oder eines Anteils davon zusätzlich durch einen Schritt der physischen Trennung des subamniotischen Stromas von der Nabelschnurschleimhaut gekennzeichnet ist, wie z. B. durch Trennung der Wharton-Sulze von der Nabelschnurschleimhaut, insbesondere zumindest von der Epithelschicht der Nabelschnurschleimhaut.

5. Verfahren nach Anspruch 4, wobei die physische Trennung der Wharton-Sulze von der Nabelschnurschleimhaut **dadurch gekennzeichnet ist, dass** die Wharton-Sulze herausgeschabt wird, vorzugsweise ohne die Nabelschnurschleimhaut mechanisch zu trennen.

6. Verfahren nach einem der Ansprüche 2 bis 5, wobei die Nabelschnur vor dem Schritt der physischen Entfernung der Gefäße und des perivaskulären Bereichs segmentiert wird.

7. Verfahren nach Anspruch 6, wobei die Segmentierung senkrecht zur Schnur erfolgt und wobei jedes senkrechte Segment vorzugsweise eine Länge von etwa 1 bis etwa 4 cm, vorzugsweise von etwa 2 bis etwa 3 cm und besonders bevorzugt von etwa 2,5 cm aufweist.

8. Verfahren nach Anspruch 6 oder 7, wobei das/die Gefäßsegment(e) vor Schritt (b) aus dem/den Segment(en) der Nabelschnur entfernt wird/werden.

9. Verfahren nach einem der vorhergehenden Ansprüche, wobei die enzymatische Behandlung durch Mischen von stromaler Wharton-Sulze oder eines Anteils davon mit einem Verdauungspuffer durchgeführt wird, der Kollagenase beinhaltet, bevorzugt mit einer spezifischen Aktivität von nicht mehr als 0,5 Wünsch-E/ml, besser nicht mehr als 0,25 Wünsch-E/ml und insbesondere 0,18 Wünsch-E/ml.

**10.** Verfahren nach einem der vorhergehenden Ansprüche, wobei Schritte (a) und (b) zusammen in einer Gesamtzeit von weniger als 3 Stunden durchgeführt werden, oder Verfahren nach einem der vorhergehenden Ansprüche, wobei es sich in Schritt (b) um humanes Plättchenlysat (hPL) handelt.

**11.** Verfahren nach einem der vorhergehenden Ansprüche, wobei in Schritt (c) die mindestens eine Zelle in einem Wachstumsmedium expandiert wird, das humanes Plättchenlysat (hPL) beinhaltet.

**12.** Verfahren nach einem der vorhergehenden Ansprüche, wobei:

- die mindestens eine Zelle in einem Wachstumsmedium expandiert wird, das kein zugesetztes Serum, insbesondere kein fetales Rinderserum (*Fetal Bovine Serum*, FBS) beinhaltet; oder
- die mindestens eine Zelle in einem Wachstumsmedium expandiert wird, das das Basalmedium MSCBM (*Mesenchymal Stem Cell Growth Medium*) CD beinhaltet.

## Revendications

**1.** Méthode ex vivo d'obtention d'une cellule stromale mésenchymateuse, ladite méthode comprenant :

(a) l'isolement de la gelée de Wharton stromale, ou d'une fraction de celle-ci, provenant d'un cordon ombilical, dans laquelle la gelée de Wharton est la gelée de Wharton du cordon ombilical à l'exclusion de la zone périvasculaire ;
(b) la soumission de la gelée de Wharton stromale, ou d'une fraction de celle-ci, à un traitement enzymatique comprenant l'enzyme collagénase en présence de lysat plaquettaire (PL), pour ainsi libérer au moins une cellule de la gelée de Wharton stromale, ou d'une fraction de celle-ci ;

dans laquelle l'étape (b) est suivie d'une étape (c) :
(c) le développement de ladite au moins une cellule dans un milieu de croissance comprenant du lysat plaquettaire (PL).

**2.** Méthode selon la revendication 1, dans laquelle : (a) l'isolement de la gelée de Wharton stromale, ou d'une fraction de celle-ci, est **caractérisé par** une étape d'ablation physique des vaisseaux et de la zone périvasculaire du cordon ombilical.

**3.** Méthode selon la revendication 2, dans laquelle l'ablation physique des vaisseaux et de la zone périvasculaire est **caractérisée par** le retrait des vaisseaux et de la zone périvasculaire du cordon ombilical.

**4.** Méthode selon l'une quelconque des revendications précédentes, dans laquelle :

(a) l'isolement de la gelée de Wharton stromale, ou d'une fraction de celle-ci, est également **caractérisé par** une étape de séparation physique du stroma sous-amniotique de la membrane de revêtement du cordon ombilical, par exemple par séparation de la gelée de Wharton de la membrane de revêtement du cordon ombilical, en particulier au moins de la couche épithéliale de la membrane de revêtement du cordon ombilical.

**5.** Méthode selon la revendication 4, dans laquelle la séparation physique de la gelée de Wharton de la membrane de revêtement du cordon ombilical est **caractérisée par** le raclage de la gelée de Wharton, de préférence sans dissection mécanique de la membrane de revêtement du cordon ombilical.

**6.** Méthode selon l'une quelconque des revendications 2 à 5, dans laquelle le cordon ombilical est segmenté avant ladite étape d'ablation physique des vaisseaux et de la zone périvasculaire.

**7.** Méthode selon la revendication 6, dans laquelle la segmentation est perpendiculaire au cordon, et dans laquelle chaque segment perpendiculaire a de préférence une longueur d'environ 1 à environ 4 cm, de préférence d'environ 2 à environ 3 cm, et plus préférablement d'environ 2,5 cm.

**8.** Méthode selon la revendication 6 ou la revendication 7, dans laquelle un ou plusieurs segments de vaisseaux sont enlevés des un ou plusieurs segments du cordon ombilical avant l'étape (b).

**9.** Méthode selon l'une quelconque des revendications précédentes, dans laquelle le traitement enzymatique est réalisé en mélangeant de la gelée de Wharton stromale, ou d'une fraction de celle-ci, à un tampon de digestion comprenant de la collagénase, de préférence avec une activité spécifique non supérieure à 0,5 U Wünsch/ml, si possible non supérieure à 0,25 U Wünsch/ml, et plus particulièrement de 0,18 U Wünsch/ml.

**10.** Méthode selon l'une quelconque des revendications précédentes, dans laquelle les étapes (a) et (b) ensemble sont conduites en un temps total inférieur à 3 heures, ou méthode selon l'une quelconque des revendications précédentes dans laquelle le lysat plaquettaire est un lysat plaquettaire humain (hPL) durant l'étape (b).

**11.** Méthode selon l'une quelconque des revendications précédentes, dans laquelle, à l'étape (c), au moins une cellule est développée dans un milieu de croissance comprenant du lysat plaquettaire humain (hPL).

**12.** Méthode selon l'une quelconque des revendications précédentes, dans laquelle :

- au moins une cellule est développée dans un milieu de croissance qui ne contient aucun sérum ajouté, en particulier aucun sérum de veau foetal (SVF) ; ou
- au moins une cellule est développée dans un milieu de croissance comprenant un milieu basal MSCBM CD.

Fig. 1

Fig. 2A

Fig. 2B

2. Segment the cord and remove umbilical artery

4 SWJ ready to be minced for enzymatic digestion

1. Accept cord

3. Carefully remove umbilical vein

Fig. 3

**Apoptosis by FACS after digestion**

*p=0.015
**p=0.016

% of 7AAD positive cells

45 40 35 30 25 20 15 10 5 0

SWJ DIGESTED WITH 1% PL

SWJ DIGESTED WITHOUT PL

PVWJ DIGESTED WITHOUT PL

Fig. 4A

Fig. 4B

Fig. 4C

Fig. 4D

Fig. 5A

SWJ                                          PVWJ

Fig. 5B

PVWJ                    SWJ

Fig. 5C

Fig. 6

Fig. 7A

Fig. 7B

Fig. 8

Fig. 9

Fig. 10

EP 3 728 566 B1

Fig. 11A

Fig. 11B

CONTROL IN CAKE2 5PL            INDUCED IN CAKE2 5PL

Fig.12A

Von Kossa staining after osteogenic induction

Fig. 12B

Fig. 12C

CONTROL IN CAKE2 5PL    INDUCED IN CAKE2 5PL

100X

200X

Fig.13

Fig. 14

## REFERENCES CITED IN THE DESCRIPTION

### Patent documents cited in the description

- WO 2008042174 A2
- WO 2004072273 A1
- US 5919702 A, Purchio
- US 20040136967 A1
- WO 2004072273 A
- WO 2017058003 A1
- WO 2013042095 A1
- US 20080181967 A1
- WO 2008146992 A1
- WO 2007080591 A2

### Non-patent literature cited in the description

- **PITTENGER et al.** *Science,* 1999, vol. 284, 143-147
- **IUDICONE et al.** *J. Transl. Med.,* 2014, vol. 12, 28
- **MAJORE.** *Cell Commun. Signal.,* 2009, vol. 20 (7), 6
- **BOLOMSKY et al.** *Experimental Hematology,* 2014, vol. 42, 516-525
- **HASS et al.** *Cell. Commun. Signal.,* 2011, vol. 9, 12
- **WEISS.** Histology, cell and tissue biology. Elsevier Biomedical, 1983, 997-998
- **WANG et al.** *Stem Cells,* 2004, vol. 22, 1330-1337
- **MITCHELL et al.** *Stem Cells,* 2003, vol. 21, 50-60
- **ROMANOV et al.** *Stem Cells,* 2003, vol. 21, 105-110
- **DAVIES et al.** *Stem Cells Translational Medicine,* 2017, vol. 6, 1620-1630
- **SUBRAMANIAN et al.** *PLOS ONE,* 2015, vol. 10 (6), 1-25
- **CONCONI et al.** Open Tissue Engineer. *Regen. Med. J.,* 2011, vol. 4, 6-20
- **RIBEIRO et al.** *International Review of Neurobiology,* 2013, vol. 108, 79-120
- **KITA et al.** *Stem Cells and Development,* 2010, vol. 19 (4), 491-501
- **LANGE-CONSIGLIO et al.** Open Tissue Engineer. *Regen. Med. J.,* 2011, vol. 4, 120-133
- **RAIO et al.** *Eur. J. Obstet. Gynecol. Reprod. Biol.,* 1999, vol. 83, 131-135
- **CONCONI et al.** Open Tissue Engineer. *Regen. Med. J.,* 2011, vol. 4, 6-20
- **SESHAREDDY et al.** *Meth. Cell Biol.,* 2008, vol. 86, 101-119
- **SARUGASER et al.** *Stem Cells,* 2005, vol. 23, 220-229
- **TAKECHI et al.** *Placenta,* 1993, vol. 14, 235-245
- **NAAIJKENS et al.** *Cell Tissue Res.,* 2012, vol. 348, 119-130
- **PURPURA et al.** *Stem Cells.,* 2004, vol. 22, 39-50
- **BATSALI et al.** *Blood,* 2013, vol. 122, 1212
- **COPLAND et al.** *Placenta,* 2002, vol. 23, 311-321
- **MIZOGUCHI et al.** *J. Dermatol. Sci.,* 2004, vol. 35, 199-206
- **PARRY.** *J. Anatomy,* 1970, vol. 107, 505-518
- **AKERMAN et al.** *Gynecol. Endocrinol.,* 2002, vol. 16, 299-306
- **KARAHUSEYINOGLU et al.** *Stem Cells,* 2007, vol. 25, 319-331
- **KARAHUSEYINOGLU et al.** *Stem Cells,* 2007, vol. 25
- **BAILEY et al.** *Tissue Eng.,* 2007, vol. 13, 2003-2010
- **LOHMANN et al.** *PLoS ONE,* 2012, vol. 7, e37839
- Online Medical Dictionary. Centre for Cancer Education, 2000
- **CAN et al.** *Stem Cells,* 2007, vol. 25, 2886-2895
- **WÜNSCH et al.** *Hoppe-Seyler's Z. Physiol. Chem.,* 1963, vol. 333, 149-51
- **CHESNEY et al.** *J. Clin. Invest.,* 1974, vol. 53, 1647-1654
- **LUDICONE et al.** *J. Transl. Med.,* 2014, vol. 12, 28
- **ASTORI et al.** *Stem Cell. Res. Ther.,* 2016, vol. 7, 93

- **DEL BUE.** *Veterin. Res. Comm,* 2007, vol. 31, 289-292
- **RUSSELL et al.** *Equine Vet. J.,* 2016, vol. 48, 261-264
- **RUSSELL et al.** *PLOS One,* 2015, vol. 10, e0136621
- **VIALE-BOURONCLE et al.** *Biochem. Biophys. Res. Commun.,* 2015, vol. 457, 314-317
- **YU.** *Cell Transplant.,* 2017, vol. 26, 365-377
- **DOMINICI et al.** *Cytotherapy,* 2006, vol. 8, 315-317
- **HORWITZ et al.** *Curr. Opin. Hematol.,* 2006, vol. 13, 419-425
- **XU et al.** *Curr. Stem Cell Res. Ther.,* 2016, vol. 11, 247-254
- **GOESSLING et al.** *Cell,* 2009, vol. 136, 1136-1147
- **ACHILLE et al.** *J. Cell. Biochem.,* 2011, vol. 112, 1817-1821
- **AGLEY et al.** *J. Histochem. Cytochem.,* 2012, vol. 60, 428-438
- **GE et al.** *Stem Cell Rev.,* 2014, vol. 10, 295-303
- **MELTON.** Essentials of Stem Cell. Elsevier, 2014
- **NEKANI et al.** *Stem Cell Res.,* 2010, vol. 3, 244-254
- **LEE et al.** *Aging Cell,* vol. 5, 187-195
- **KHAN et al.** *J. Orthop. Res.,* 2010, vol. 28, 834-40
- **WAGNER et al.** *PLoS One,* 2008, vol. 3, e2213
- **GRISENDI et al.** *Cytotherapy,* 2010, vol. 12, 466-477
- **MOORE et al.** *Exp. Eye Res,* 2013, vol. 115, 178-188
- **ZEILBECK et al.** *PLoS One.,* 2014, vol. 9 (4), e95546
- **IGNATIUS et al.** *J. Cell. Biochem,* 2011, vol. 112, 2594-2605
- **SCHRAUFSTATTER et al.** *World J. Stem Cells,* 2015, vol. 7, 1090-1108
- **MURGIA et al.** *PLoS One,* 2016, vol. 11, e0163629
- **RASINI et al.** *Cytotherapy,* 2013, vol. 15, 292-306